# EUROPEAN PATENT APPLICATION

(11) **EP 2 177 521 A1**
(43) Date of publication of application: **21.04.2010**
(21) Application number: 08382042.3
(22) Date of filing: 14.10.2008
(51) Int. Cl.: C07D 417/04, C07D 285/135, A61K 31/433, A61P 29/00

(54) **New 2-Amidothiadiazole Derivatives**

(71) Applicant: Almirall, S.A., 08022 Barcelona (ES)
(72) Inventor: Grima Poveda, Pedro Manuel, 08028, Barcelona (ES); Aguilar Izquierdo, Nuria, 08005, Barcelona (ES); Mir Cepeda, Marta, 08024, Barcelona (ES); Lopez Martinez, Manuel, 08013, Barcelona (ES)
(74) Representative: Elzaburu Marquez, Alberto

(57) **Abstract**

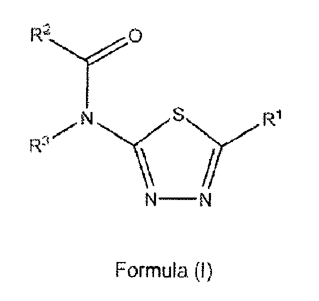

New 2-amidothiadiazole derivatives having the chemical structure of formula (I) or pharmaceutically acceptable salts or N-oxides thereof as agonists of S1P1 receptors.

## Description

The present invention relates to 2-amidothiadiazole derivatives, to processes for their preparation and to pharmaceutical compositions containing them. These compounds are potent agonists of S1P1 receptors and thus, they are useful in the treatment, prevention or suppression of diseases and disorders known to be susceptible to improvement by sphingosine-1-phosphate receptors agonists (S1P1), such as autoimmune diseases, chronic immune and inflammatory diseases, transplant rejection, malignant neoplastic diseases, angiogenic-related disorders, pain, neurological diseases,viral and infectious diseases.

Sphingosine -1 phosphate (S1 P) is a pleiotropic lipid mediator that exhibits a broad spectrum of biological activities, including cell proliferation, survival, lymphocyte trafficking, cytoskeletal organization, and morphogenesis. S1 P is generated from endogenous sphingosine through phosphorylation by specific kinases, named sphingosine kinases 1 and 2. The levels of S1 P in biological fluids and tissues are tightly regulated by the balance between its synthesis by sphingosine kinases and its degradation by S1 P lyase. This tight control is important since an excessive production of S1 P has been associated to various pathological conditions, such as angiogenesis and vascular permeability changes in cancer, inflammation, myocardial infarction or transplant rejection.

Gene deletion studies and reverse pharmacology have provided evidence that most of the effects of S1 P are mediated via five G-protein coupled receptor subtypes, named S1 P1 to S1 P5 (Brinkmann, Pharmacology & therapeutics 115:84-105, 2007). The interest on this family of receptors increased following the discovery that they were the pharmacological target of FTY720. This compound, a synthetic analog of a natural product derived from the fungus *Isaria sinclairii,* exhibited a peculiar immunomodulatory potential in vivo. When administered in vivo, it caused lymphopenia, due to the sequestration of lymphocytes from the blood into the lymph nodes and Peyer's patches. The close structural similarity of FTY720 to sphingosine, together with the discovery of the formation of phosphorylated FTY720 in vivo (FTY720P) prompted to speculate that FTY720-P could be acting as a mimetic of S1P. This proven to be the case and it was later on demonstrated that FTY-P binds 4 of the five known S1P receptors, namely S1P1, S1P3, S1P4 and S1P5.

Expression analysis identified S1P1 as the dominant S1P receptor expressed on lymphocytes. Moreover, the transfer of S1P1-deficient T cells to normal mice led to the cells being sequestered in lymph nodes, as occurred with animals treated with fingolimod. These two facts strongly pointed out at S1P1 as the main receptor involved in the lymphopenic effect of FTY-P in vivo (Baumruker et al, Exp. Opin. Invest. Drugs 2007; 16(3): 283-289). FTY720 is currently in phase III trials for the treatment of relapsing-remitting multiple sclerosis. The drug is presumed to act by causing the retention of pathogenic lymphocytes in lymph nodes, thus preventing them to infiltrate the central nervous system (CNS).

In view of the physiological effects, several S1P1 agonists have been recently disclosed for the treatment or prevention of autoimmune diseases, such as multiple sclerosis (WO2008000419, WO2008021532), rheumatoid arthritis or Crohn's disease (WO2007091501), chronic immune and inflammatory diseases such as asthma, transplant rejection (WO199400943) cancer (WO2003097028), lymphoid malignancies (WO2007143081), angiogenic-related disorders, pain (WO2004110421, WO2007089715) neurological diseases such as neurodegeneration (WO2005025553) or dementia (WO2005058295), cardiovascular diseases (WO2004010987),.

Autoimmune diseases include but are not limited to rheumatoid arthritis, multiple sclerosis, inflammatory bowel diseases such as Crohn's diseases and ulcerative colitis, psoriatic arthritis, thyroiditis such as Hashimoto's thyroiditis, type I diabetes; systemic lupus erythematosis and Sjögrn's syndrome.

Rejection transplanted organs such as kidney, liver, heart, lung, pancreas, cornea and skin; graft-versus-hist disease brought about by stem cell transplantation.

Immune and inflammatory diseases which may be prevented or treated include but are not limited to asthma, COPD, respiratory distress syndrome, acute or chronic pancreatitis and hepatitis; chronic sarcoidosis, contact dermatitis, atopic dermatitis, allergic rhinitis, allergic conjunctivitis, Behcet syndrome, inflammatory eye conditions such as conjunctivitis and uveitis.

Malignant neoplastic diseases that may be prevented or treated include but are not limited to solid cancer, tumor metastasis and lymphoid malignancies Angiogenesis-related disorders that may be prevented or treated include but are not limited to hemangiomas, ocular neovascularization, macular degeneration or diabetic retinopathy.

Pain including neuropathic pain, that may be prevented or treated include but are not limited to prophylaxis or treatment of chronic pain, wherein chronic pain is selected from chronic muscular diseases such as back pain, pain during menstruation, pain during osteoarthritis, pain during rheumatoid arthritis, pain during gastrointestinal inflammation, pain during inflammation of the heart muscle, pain during multiple sclerosis, pain during neuritis, pain during AIDS, pain during chemotherapy, tumor pain, neuropathic pain e. g. after amputation,trigeminal neuralgia, migraine or post herpetic neuralgia.

Cardiovascular diseases which may be prevented or treated include but are not limited to chronic heart failure, congestive heart failure, arrhythmia or tachyarrythmia, unstable angina, acute myocardial infarction or complications from cardiac surgery or for improving heart energy efficiency or cardiac output.

Neurological diseases including neurodegeneration, dementia or brain degeneration that may be prevented or treated include but are not limited to neurological disorders including Parkinson's desease, Parkinsonian disorders, Huntington's disease, Alzheimer's disease, amyotrophic lateral sclerosis, spinal ischemia, ischemic stroke, spinal cord injury, cancer-related brain injury, and cancer-related spinal cord injury, Shy-Drager syndrome, progressive supranuclear palsy, Lewy body disease, stroke, cerebral infarction, multi-infarct dementia, and geriatric dementia,

Viral diseases which may be prevented or treated include but are not limited to HIV infection, hepatitis C and cytomegalovirus infection.

Infectious diseases which may be prevented or treated include but are not limited to pathogenic fungal diseases.

It has now been found that certain 2-amidothiadiazoles are novel and potent agonists of S1 P1 and can therefore be used in the treatment or prevention of these diseases.

Thus the present invention is directed to new 2-amidothiadiazole derivatives of formula (I) or pharmaceutically acceptable salts or N-oxides thereof 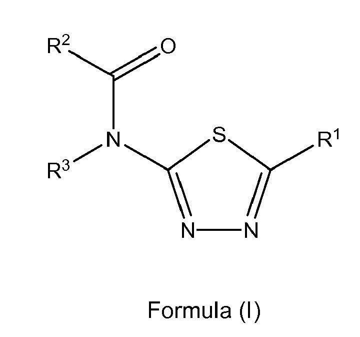 wherein
R¹ represents:
➢ an 8 to 10 membered bicyclic N-containing heteroaryl group optionally substituted by one or more substitutents selected from halogen atoms, hydroxycarbonyl groups, C₁₋₄ alkyl groups, C₁₋₄ haloaklyl groups, C₁₋₄ alkoxy groups and C₃₋₄ cycloalkyl groups;
➢ a pyridyl group substituted with one or more substituents selected from halogen atoms, hydroxy groups, hydroxycarbonyl groups, C₁₋₄ alkyl groups, C₁₋₄ haloaklyl groups, C₁₋₄ alkoxy groups, C₃₋₄ cycloalkyl groups and, -NR'R" groups, wherein R' represents a hydrogen atom or a C₁₋₄ alkyl group and R" represents a hydrogen atom or a C₁₋₄ alkyl group optionally substituted by a hydroxy group;
➢ a pyridinone group substituted with one or more substituents selected from halogen atoms, C₁₋₄ alkyl groups and C₁₋₄ haloaklyl groups; or
➢ a group of formula: 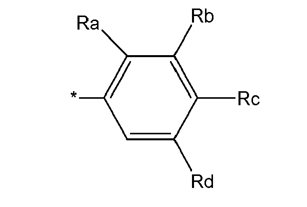
wherein:
- Ra represents a hydrogen atom or a C₁₋₄ alkyl group,
- Rb represents a hydrogen atom, halogen atom or C₁₋₄ alkyl group,
- Rd represents a hydrogen atom, a C₁₋₄ alkyl group or a C₃₋₄ cycloalkyl group,
- Rc represents a hydroxy group; a C₁₋₄ alkoxy group which is optionally substituted with one or more substituents selected from hydroxy groups, C₁₋₃ alkoxy groups, hydroxycarbonyl groups, C₁₋₄ alkoxycarbonyl groups and NHR⁴ groups, wherein R⁴ represents a hydrogen atom; or Rc is a C₂₋₄ acyl group or a C₁₋₄ alkyl group optionally substituted by a hydroxycarbonyl group, or Rc represents -(CH₂)₍₀₋₄₎-L-R⁵ wherein L represents -C(O)O-, -C(O)NH-, -S(O)₂NH-, -NH-, -CONHS(O)₂- or a group of formula: 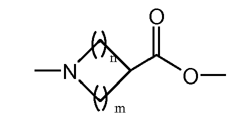
wherein n and m independently are integer from 1 to 2, and R⁵ represents a hydrogen atom or a C₁₋₄ alkyl group optionally substituted by a hydroxycarbonyl group;
R² represents:
- a monocyclic or bicyclic C₅₋₁₀ aryl group optionally substituted with one or more substituents selected from halogen atoms, C₁₋₄ alkyl groups, C₁₋₄ alkoxy groups and phenyl groups, wherein the alkyl group is optionally substituted by one or more halogen atoms,
- a monocyclic or bicyclic 5-10 membered heteroaryl group comprising one or more heteroatoms selected from N, S and O optionally substituted with one or more substituents selected from halogen atoms, C₁₋₄ alkyl and C₁₋₄ alkoxy groups, wherein the C₁₋₄ alkyl group is optionally substituted by one or more halogen atoms,
- a dihydrobenzodioxine group or a benzyl group which is optionally substituted with one or more substituents selected from halogen atoms,
   and
   R³ represents:

- a linear or branched C₁₋₆ alkyl, C₃₋₆ cycloalkyl-C₁₋₄ alkyl, C₁₋₄ alkoxy-C₁₋₄ alkyl, dialkylamino-C₁₋₄alkyl group, or phenyl-C₁₋₄alkyl group;
   with the proviso that when Rc represents a methoxy or ethoxy group, then one of Rb or Rd can not be a hydrogen atom;
with the additional proviso that the compound of formula (I) is not N-methyl-N-(5-(6-methylpyridin-3-yl)-1,3,4-thiadiazol-2-yl)nicotinamide, nor N-methyl-N-(5-(6-methylpyridin-3-yl)-1,3,4-thiadiazol-2-yl)isonicotinamide.
Further objectives of the present invention are to provide a method for preparing said compounds; pharmaceutical compositions comprising an effective amount of said compounds; the use of the compounds in the manufacture of a medicament for the treatment of pathological conditions or diseases susceptible to improvement by sphingosine-1-phosphate receptors agonists (S1P1), wherein the pathological condition or disease is selected from autoimmune diseases, chronic immune and inflammatory diseases, transplant rejection, malignant neoplastic diseases, angiogenic-related disorders, pain, neurological diseases,viral and infectious diseases, and methods of treatment of pathological conditions or diseases susceptible to amelioration by sphingosine-1-phosphate receptors agonists (S1P1), wherein the pathological condition or disease is selected from autoimmune diseases, chronic immune and inflammatory diseases, transplant rejection, malignant neoplastic diseases, angiogenic-related disorders, pain, neurological diseases,viral and infectious diseases comprising the administration of the compounds of the invention to a subject in need of treatment.

As used herein the term alkyl embraces optionally substituted, linear or branched hydrocarbon radicals having 1 to 6 carbon atoms, preferably from 1 to 4 carbon atoms. Preferred substituents on the alkyl groups are halogen atoms and hydroxy groups, and are more preferably halogen atoms.

Examples include methyl, ethyl, *n*-propyl, *i*-propyl, *n*-butyl, *n*-pentyl, *n*-hexyl, *i*-butyl, *sec*-butyl and *tert*-butyl radicals.

As used herein, a haloalkyl group is a said alkyl group, for example a C₁₋₄ or C₁₋₂ alkyl group, which is attached to 1, 2 or 3 halogen atoms.

Preferably, said haloakyl group is chosen from -CCl₃ and -CF₃.

As used herein the term alkoxy embraces optionally substituted, linear or branched oxy-containing radicals each having 1 to 4 carbon atoms. Preferred substituents on the alkoxy groups are halogen atoms and hydroxy groups, and are more preferably halogen atoms.

Examples include methoxy, ethoxy, *n*-propoxy, *i*-propoxy, *n*-butoxy, *sec*-butoxy and *tert*-butoxy radicals.

As used herein, the term cycloalkyl embraces saturated carbocyclic radicals and, unless otherwise specified, a cycloalkyl radical typically has from 3 to 6 carbon atoms, preferably from 3 to 4 carbon atoms.

Examples include cyclopropyl, cyclobutyl, cyclopentyl and cyclohexyl. When a cycloalkyl radical carries 2 or more substituents, the substituents may be the same or different. Preferred substituents on the cycloalkyl groups are halogen atoms and hydroxy groups, and are more preferably halogen atoms.

As used herein, the term dialkylamino embraces radicals containing a trivalent nitrogen atoms with two optionally substituted, linear or branched alkyl radicals of 1 to 4 carbon atoms in each alkyl radical.

A dialkylamino group typically contains two alkyl groups, each of which is unsubstituted or substituted with 1, 2 or 3 substituents which may be the same or different. The substituents are preferably selected from halogen atoms, preferably fluorine atoms, hydroxy groups and alkoxy groups having from 1 to 4 carbon atoms. Typically, the substituents on a dialkylamino group are themselves unsubstituted.

Preferred optionally substituted dialkylamino radicals include dimethylamino, diethylamino, methyl(ethyl)amino, di(n-propyl)amino, n-propyl(methyl)amino, n-propyl(ethyl)amino, di(i-propyl)amino, i-propyl(methyl)amino, i-propyl(ethyl)amino, di(n-butyl)amino, n-butyl(methyl)amino, n-butyl(ethyl)amino, n-butyl(i-propyl)amino, di(sec-butyl)amino, sec-butyl(methyl)amino, sec-butyl(ethyl)amino, sec-butyl(n-propyl)amino and sec-butyl(i-propyl)amino.

As used herein, the term alkoxycarbonyl embraces optionally substituted, linear or branched radicals each having alkyl portions of 1 to 4 carbon atoms.

An alkoxycarbonyl group is typically unsubstituted or substituted with 1, 2 or 3 substituents which may be the same or different. The substituents are preferably selected from halogen atoms, preferably fluorine atoms, hydroxy groups and alkoxy groups having from 1 to 4 carbon atoms. Typically, the substituents on an alkoxycarbonyl group are themselves unsubstituted.

Preferred optionally substituted alkoxycarbonyl radicals include methoxycarbonyl, ethoxycarbonyl, n-propoxycarbonyl, i-propoxycarbonyl, n-butoxycarbonyl, secbutoxycarbonyl, trifluoromethoxycarbonyl, difluoromethoxycarbonyl, hydroxymethoxycarbonyl, 2-hydroxyethoxycarbonyl and 2-hydroxypropoxycarbonyl.

As used herein, the term acyl embraces optionally substituted, linear or branched radicals having 2 to 4 carbon atoms.

An acyl group is typically unsubstituted or substituted with 1, 2 or 3 substituents which may be the same or different. The substituents are preferably selected from halogen atoms, preferably fluorine atoms, hydroxy groups and alkoxy groups having from 1 to 4 carbon atoms. Typically, the substituents on an acyl group are themselves unsubstituted.

Preferred optionally substituted acyl radicals include acetyl, propionyl and butiryl,

As used herein, the term aryl radical embraces typically a C₅-C₁₀ monocyclic or polycyclic aryl radical such as phenyl and naphthyl. C₆-C₁₀ aryl groups are preferred. Phenyl is more preferred.

A said optionally substituted aryl radical is typically unsubstituted or substituted with 1, 2 or 3 substituents which may be the same or different. The substituents are preferably selected from halogen atoms, preferably fluorine atoms, hydroxy groups, alkoxycarbonyl groups in which the alkyl moiety has from 1 to 4 carbon atoms, hydroxycarbonyl groups, carbamoyl groups, nitro groups, cyano groups, C₁-C₄ alkyl groups, C₁-C₄ alkoxy groups and C₁-C₄ hydroxyalkyl groups. When an aryl radical carries 2 or more substituents, the substituents may be the same or different. Unless otherwise specified, the substituents on an aryl group are typically themselves unsubstituted.

As used herein, the term heteroaryl radical embraces typically a 5- to 10- membered ring system comprising at least one heteroaromatic ring and containing at least one heteroatom selected from O, S and N. A heteroaryl radical may be a single ring or two or more fused rings wherein at least one ring contains a heteroatom.

A said optionally substituted heteroaryl radical is typically unsubstituted or substituted with 1, 2 or 3 substituents which may be the same or different. The substituents are preferably selected from halogen atoms, preferably fluorine, chlorine or bromine atoms, alkoxycarbonyl groups in which the alkyl moiety has from 1 to 4 carbon atoms, nitro groups, hydroxy groups, C₁-C₄ alkyl groups and C₁-C₄ alkoxy groups. When an heteroaryl radical carries 2 or more substituents, the substituents may be the same or different. Unless otherwise specified, the substituents on a heteroaryl radical are typically themselves unsubstituted.

Examples include pyridyl, pyrazinyl, pyrimidinyl, pyridazinyl, furyl, benzofuranyl, oxadiazolyl, oxazolyl, isoxazolyl, benzoxazolyl, imidazolyl, benzimidazolyl, thiazolyl, thiadiazolyl, thienyl, pyrrolyl, pyridinyl, benzothiazolyl, indolyl, indazolyl, purinyl, quinolyl, isoquinolyl, phthalazinyl, naphthyridinyl, quinoxalinyl, quinazolinyl, quinolizinyl, cinnolinyl, triazolyl, indolizinyl, indolinyl, isoindolinyl, isoindolyl, imidazolidinyl, pteridinyl, thianthrenyl, pyrazolyl, 2H-pyrazolo[3,4-d]pyrimidinyl, 1 H-pyrazolo[3,4-d]pyrimidinyl, thieno[2,3-d] pyrimidnyl and the various pyrrolopyridyl radicals.

Oxadiazolyl, oxazolyl, pyridyl, pyrrolyl, imidazolyl, thiazolyl, thiadiazolyl, thienyl, furanyl, quinolinyl, isoquinolinyl, indolyl, benzoxazolyl, naphthyridinyl, benzofuranyl, pyrazinyl, pyrimidinyl and the various pyrrolopyridyl radicals are preferred.

As used herein, some of the atoms, radicals, moieties, chains or cycles present in the general structures of the invention are "optionally substituted". This means that these atoms, radicals, moieties, chains or cycles can be either unsubstituted or substituted in any posiition by one or more, for example 1, 2, 3 or 4, substituents, whereby the hydrogen atoms bound to the unsubstituted atoms, radicals, moieties, chains or cycles are replaced by chemically acceptable atoms, radicals, moieties, chains or cycles. When two or more substituents are present, each substituent may be the same or different.

As used herein, the term halogen atom embraces chlorine, fluorine, bromine or iodine atoms typically a fluorine, chlorine or bromine atom, most preferably bromine or fluorine. The term halo when used as a prefix has the same meaning.

As used herein, the term pharmaceutically acceptable salt embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic or p-toluenesulphonic acid. Pharmaceutically acceptable bases include alkali metal (e.g. sodium or potassium) and alkali earth metal (e.g. calcium or magnesium) hydroxides and organic bases, for example alkyl amines, arylalkyl amines and heterocyclic amines.

Other preferred salts according to the invention are quaternary ammonium compounds wherein an equivalent of an anion (X-) is associated with the positive charge of the N atom. X- may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and p-toluenesulphonate. X- is preferably an anion selected from chloride, bromide, iodide, sulphate, nitrate, acetate, maleate, oxalate, succinate or trifluoroacetate. More preferably X- is chloride, bromide, trifluoroacetate or methanesulphonate.

Typically, in the definition of R¹, C₁₋₄ alkyl group, C₁₋₄ haloaklyl group, C₁₋₄ alkoxy group and C₃₋₄ cycloalkyl group substituents on 8 to 10 membered bicyclic N-containing heteroaryl groups or pyridyl groups are themselves unsubstituted.

Typically, in the definition of R¹, C₁₋₄ alkyl group and C₁₋₄ haloaklyl group substituents on pyridinone groups are themselves unsubstituted.

Typically, in the definition of R¹, when Rc represents a C₁₋₄ alkoxy group, C₁₋₃ alkoxy group, and C₁₋₄ alkoxycarbonyl group substituents on said C₁₋₄ alkoxy group are themselves unsubstituted.

Typically, in the definition of R², C₁₋₄ alkoxy group and phenyl group substituents on monocyclic or bicyclic C₅₋₁₀ aryl groups or monocyclic or bicyclic 5-10 membered heteroaryl group comprising one or more heteroatoms selected from N, S and O are themselves unsubstituted.

As used herein, an N-oxide is formed from the tertiary basic amines or imines present in the molecule, using a convenient oxidising agent.

Typically, R¹ represents an imidazo[1,2-a]pyridyl group; a pyridyl group which is substituted with one or more substituents selected from halogen atoms, C₁₋₄ alkyl groups and C₁₋₄ alkoxy groups; a pyridinone group substituted with a chlorine atom; or a group of formula: 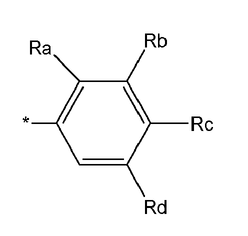 wherein:
- Ra represents a hydrogen atom or a methyl group,
- Rb represents a hydrogen atom, halogen atom or C₁₋₄ alkyl group,
- Rd represents a hydrogen atom or a C₁₋₄ alkyl group,
- Rc represents a hydroxy group, a C₁₋₄ alkoxy group which is optionally substituted with one or more substituents selected from hydroxy groups or C₁₋₃ alkoxy groups, or Rc represents -(CH₂)₍₀₋₂₎-L-R⁵, wherein L represents -C(O)NH-, -NH-, or a group of formula: 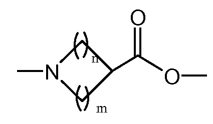
wherein n and m independently are integers from 1 to 2, and R⁵ represents a hydrogen atom or a C₁₋₄ alkyl group optionally substituted by a hydroxycarbonyl group.

Preferably, R¹ represents a pyridyl group substituted with one or two substituents selected from halogen atoms, C₁₋₄ alkyl groups and C₁₋₄ alkoxy groups; or a group of formula: 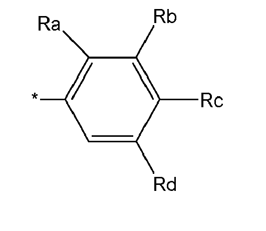 wherein:
- Ra represents a hydrogen atom,
- Rb represents a hydrogen atom or a C₁₋₄ alkyl group,
- Rd represents a hydrogen atom or C₁₋₄ alkyl group,
- Rc represents a hydroxy group, a C₁₋₄ alkoxy group which is optionally substituted by one or more substituents selected from hydroxy groups, or Rc represents -(CH₂)₍₀₋₂₎-L-R⁵, wherein L represents -C(O)NH-, -NH-, or a group of formula: 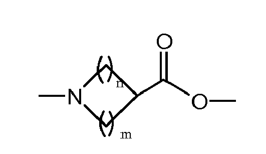
wherein n and m independently are integers from 1 to 2, and R⁵ represents a hydrogen atom or a C₁₋₄ alkyl group optionally substituted by a hydroxycarbonyl group.

More preferably, R¹ represents represents a pyridyl group substituted with one or two substituents selected from chlorine atoms, methyl and methoxy groups; or a group of formula: 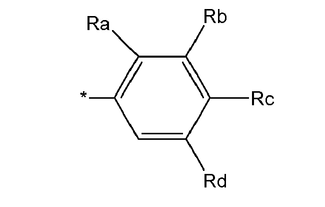 wherein:
- Ra represents a hydrogen atom,
- Rd represents a hydrogen atom or methyl group,
- Rb represents a hydrogen atom or methyl group,
- Rc represents a hydroxy group, a C₁₋₄ alkoxy group which is optionally substituted by one or more substituents selected from hydroxy groups; or Rc represents -(CH₂)₍₀₋₁₎-L-R⁵, wherein L represents -C(O)NH-, -NH-, or a group of formula: 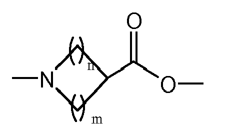
wherein both n and m have a value of 1, and R⁵ represents a hydrogen atom or a C₁₋₂ alkyl group optionally substituted by a hydroxycarbonyl group.

Typically, R² represents a phenyl group substituted by one substituent selected from a halogen atom, a C₁₋₄ alkyl group and a C₁₋₄ alkoxy group, wherein the alkyl group is optionally substituted by one or more halogen atoms; or R² represents a monocyclic N-containing 5-10 membered heteroaryl group which is substituted by a halogen atom.

Preferably, R² represents represents a phenyl group which is substituted with one substituent selected from a fluorine atom, a chlorine atom, a methyl, a trifluoromethyl or a methoxy group; or R² represents a pyridyl group which is substituted with a fluorine atom.

More preferably, R² represents a phenyl group substituted with one substituent selected from a fluorine atom, a chlorine atom or a methoxy group.

Typically, R³ represents a linear C₃₋₆ alkyl group or a C₃₋₄ cycloalkyl-C₁₋₂ alkyl group.

Preferably, R³ represents a propyl, butyl or cyclopropylmethyl group.

More preferably, R³ represents a butyl or a cyclopropylmethyl group.

Most preferably, R¹ represents a pyridyl group substituted with one or two substituents selected from chlorine atoms, methyl and methoxy groups; or a group of formula: 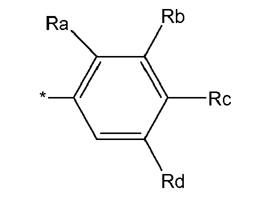 wherein:
- Ra represents a hydrogen atom,
- Rb represents a hydrogen atom or methyl group,
- Rd represents a hydrogen atom or methyl group,
- Rc represents a hydroxy group, a C₁₋₄ alkoxy group which is optionally substituted with one or more substituents selected from hydroxy groups; or Rc represents -(CH₂)₍₀₋₁₎-L-R⁵, wherein L represents -C(O)NH-, -NH-, or a group of formula: 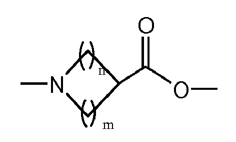
wherein both n and m have a value of 1, and R⁵ represents a hydrogen atom or a C₁₋₄ alkyl group optionally substituted by a hydroxycarbonyl group;
R² represents a phenyl group substituted with one substituent selected from a fluorine atom, a chlorine atom and a methoxy group;
and R³ represents a butyl or a cyclopropylmethyl group.

In a preferred embodiment, R¹ represents an imidazo [1, 2-a] pyridyl group; a pyridyl group substituted with one or two substituents selected from chlorine atoms, methyl groups and methoxy groups; pyridone group substituted with a chlorine atom; or a group of formula: 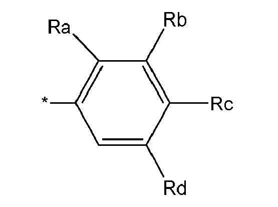 wherein
- Ra represents a hydrogen atom or a methyl group,
- Rb represents a hydrogen atom, a chlorine atom or a methyl group,
- Rd represents a hydrogen atom or a methyl group,
- Rc represents a hydroxy group; a C₁₋₄ alkoxy group which is optionally substituted with one or more substituents selected from hydroxy groups, methoxy groups, hydroxycarbonyl groups, C₁₋₄ alkoxycarbonyl groups; or Rc represents -(CH₂)₍₀₋₂₎-L-R⁵, wherein L represents -CO(O)-, -C(O)NH-, - S(O)₂NH- or a group of formula: 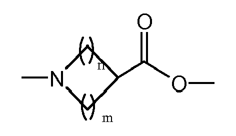
wherein n and m are both 1, and R⁵ represents a hydrogen atom or a C₁₋₂ alkyl group optionally substituted by a hydroxycarbonyl group;
R² represents:
- a phenyl or naphthyl group optionally substituted with one or two substituents selected from chlorine atoms, fluorine atoms, methyl groups, trifluoromethyl groups, C₁₋₄ alkoxy groups and phenyl groups;
- a pyridine group optionally substituted with a fluorine atom;
- a dihydrobenzodioxine group or a benzyl group; and

R³ represents a linear or branched C₁₋₅ alkyl, cyclopropylmethyl, methoxypropyl, diethylaminopropyl or phenylethyl group.

Particular individual compounds of the invention include:
N-{5-[4-(aminosulfonyl)phenyl]-1,3,4-thiadiazol-2-yl}-N-butyl-3-methoxy-benzamide
N-{5-[4-(aminosulfonyl)phenyl]-1,3,4-thiadiazol-2-yl}-N-butyl-2-naphthamide
N-{5-[4-(aminosulfonyl)phenyl]-1,3,4-thiadiazol-2-yl}-N-butylbiphenyl-4-carboxamide
N-{5-[4-(aminosulfonyl)phenyl]-1,3,4-thiadiazol-2-yl}-4-butoxy-N-butylbenzamide
N-{5-[4-(aminosulfonyl)phenyl]-1,3,4-thiadiazol-2-yl}-N-butylbenzamide
3-(4-(5-(N-butyl-3-methoxybenzamido)-1,3,4-thiadiazol-2-yl)phenyl)propanoic acid
N-butyl-2-chloro-N-[5-(4-methoxy-3,5-dimethylphenyl)-1,3,4-thiadiazol-2-yl] benzamide
N-butyl-2-chloro-N-[5-(4-hydroxy-3,5-dimethylphenyl)-1,3,4-thiadiazol-2-yl]benzamide
N-butyl-2-fluoro-N-[5-(4-methoxy-3,5-dimethylphenyl)-1,3,4-thiadiazol-2-yl]benzamide
N-Butyl-2-fluoro-N-[5-(4-hydroxy-3,5-dimethylphenyl)-1,3,4-thiadiazol-2-yl]benzamide
N-butyl-N-[5-(4-methoxy-3,5-dimethylphenyl)-1,3,4-thiadiazol-2-yl]benzamide
N-butyl-3-methoxy-N-[5-(4-methoxy-3,5-dimethylphenyl)-1,3,4-thiadiazol-2-yl]benzamide
N-butyl-N-[5-(4-methoxy-3,5-dimethylphenyl)-1,3,4-thiadiazol-2-yl]-1-naphthamide
N-butyl-2,6-dichloro-N-(5-(4-methoxy-3,5-dimethylphenyl)-1,3,4-thiadiazol-2-yl)benzamide
N-butyl-N-[5-(4-methoxy-3,5-dimethylphenyl)-1,3,4-thiadiazol-2-yl]-2-(trifluoromethyl)benzamide
N-butyl-N-[5-(4-methoxy-3,5-dimethylphenyl)-1,3,4-thiadiazol-2-yl]-2-phenylacetamide
N-butyl-N-[5-(4-methoxy-3,5-dimethylphenyl)-1,3,4-thiadiazol-2-yl]-2-naphthamide
N-butyl-N-[5-(4-methoxy-3,5-dimethylphenyl)-1,3,4-thiadiazol-2-yl]-2,3-dihydro-1,4-benzodioxine-6-carboxamide
N-butyl-2-methoxy-N-[5-(4-methoxy-3,5-dimethylphenyl)-1,3,4-thiadiazol-2-yl]benzamide
N-Butyl-3-fluoro-N-[5-(4-methoxy-3,5-dimethylphenyl)-1,3,4-thiadiazol-2-yl]benzamide
N-Butyl-N-[5-(4-methoxy-3,5-dimethylphenyl)-1,3,4-thiadiazol-2-yl]nicotinamide
N-Butyl-N-[5-(4-methoxy-3,5-dimethylphenyl)-1,3,4-thiadiazol-2-yl]pyridine-2-carboxamide
N-Butyl-6-fluoro-N-[5-(4-methoxy-3,5-dimethylphenyl)-1,3,4-thiadiazol-2-yl]pyridine-2-carboxamide
N-butyl-N-[5-(4-methoxy-3,5-dimethylphenyl)-1,3,4-thiadiazol-2-yl]-2-methylbenzamide
2-chloro-N-[5-(4-methoxy-3,5-dimethylphenyl)-1,3,4-thiadiazol-2-yl]-Nmethylbenzamide
N-butyl-2-chloro-N-[5-(4-methoxy-3-methylphenyl)-1,3,4-thiadiazol-2-yl]benzamide
N-butyl-2-chloro-N-[5-(3-chloro-4-methoxyphenyl)-1,3,4-thiadiazol-2-yl]benzamide
Methyl 4-{5-[butyl(2-chlorobenzoyl)amino]-1,3,4-thiadiazol-2-yl}benzoate
4-{5-[Butyl(2-chlorobenzoyl)amino]-1,3,4-thiadiazol-2-yl}benzoic acid
N-Butyl-2-chloro-N-{5-[4-(2,3-dihydroxypropoxy)-3,5-dimethylphenyl]-1,3,4-thiadiazol-2-yl}benzamide
N-Butyl-2-chloro-N-[5-(2-chloro-6-methoxypyridin-4-yl)-1,3,4-thiadiazol-2-yl]benzamide
N-butyl-2-chloro-N-{5-[4-(2-methoxyethoxy)-3,5-dimethylphenyl]-1,3,4-thiadiazol-2-yl}benzamide
2-Chloro-N-[5-(4-methoxy-3,5-dimethylphenyl)-1,3,4-thiadiazol-2-yl]-N-(2-methoxyethyl)benzamide
2-Chloro-N-ethyl-N-[5-(4-methoxy-3,5-dimethylphenyl)-1,3,4-thiadiazol-2-yl]benzamide
*tert*-Butyl (4-{5-[butyl(2-chlorobenzoyl)amino]-1,3,4-thiadiazol-2-yl}-2,6-dimethyl-phenoxy)acetate
(4-{5-[Butyl(2-chlorobenzoyl)amino]-1,3,4-thiadiazol-2-yl}-2,6-dimethylphenoxy)acetic acid
2-Chloro-N-[5-(4-methoxy-3,5-dimethylphenyl)-1,3,4-thiadiazol-2-yl]-N-(3-methylbutyl)benzamide
2-Chloro-N-[3-(diethylamino)propyl]-N-[5-(4-methoxy-3,5-dimethylphenyl)-1,3,4-thiadiazol-2-yl]benzamide
N-Butyl-2-chloro-N-[5-(2-chloro-6-methylpyridin-4-yl)-1,3,4-thiadiazol-2-yl]benzamide
N-Butyl-2-chloro-N-[5-(6-chloro-2-oxo-1,2-dihydropyridin-4-yl)-1,3,4-thiadiazol-2-yl]benzamide
N-Butyl-N-[5-(2-chloro-6-methoxypyridin-4-yl)-1,3,4-thiadiazol-2-yl]-2-fluorobenzamide
N-Butyl-N-[5-(2-chloro-6-methylpyridin-4-yl)-1,3,4-thiadiazol-2-yl]-2-fluorobenzamide
2-fluoro-N-[5-(4-methoxy-3,5-dimethylphenyl)-1,3,4-thiadiazol-2-yl]-N-propylbenzamide
N-Butyl-2-fluoro-N-[5-(2-methylpyridin-4-yl)-1,3,4-thiadiazol-2-yl] benzamide
N-butyl-2-fluoro-N-[5-(2-methoxypyridin-4-yl)-1,3,4-thiadiazol-2-yl] benzamide
N-(cyclopropylmethyl)-2-fluoro-N-[5-(4-methoxy-3,5-dimethylphenyl)-1,3,4-thiadiazol-2-yl]benzamide
3-(4-{5-[butyl(2-fluorobenzoyl)amino]-1,3,4-thiadiazol-2-yl}-2,6-dimethylphenyl)propanoic acid
N-butyl-2-fluoro-N-(5-(6-methoxypyridin-3-yl)-1,3,4-thiadiazol-2-yl)benzamide
N-butyl-2-fluoro-N-(5-(imidazo[1,2-a]pyridin-6-yl)-1,3,4-thiadiazol-2-yl)benzamide
N-butyl-2-fluoro-N-(5-(imidazo[1,2-a]pyridin-7-yl)-1,3,4-thiadiazol-2-yl)benzamide
3-(4-(5-(N-butyl-2-chlorobenzamido)-1,3,4-thiadiazol-2-yl)benzamido) propanoic acid
Ethyl 3-(4-(5-(N-butyl-2-chlorobenzamido)-1,3,4-thiadiazol-2-yl)benzamido) propanoate
N-butyl-N-(5-(4-carbamoylphenyl)-1,3,4-thiadiazol-2-yl)-2-chlorobenzamide
1-(4-(5-(N-butyl-2-fluorobenzamido)-1,3,4-thiadiazol-2-yl)benzyl)azetidine-3-carboxylic acid
(R)-N-butyl-N-(5-(4-(2,3-dihydroxypropoxy)-3,5-dimethylphenyl)-1,3,4-thiadiazol-2-yl)-2-fluorobenzamide
2-fluoro-N-(5-(4-methoxy-3,5-dimethylphenyl)-1,3,4-thiadiazol-2-yl)-N-phenethylbenzamide
2-(4-(5-(N-butyl-2-fluorobenzamido)-1,3,4-thiadiazol-2-yl)benzamido)acetic acid
Of outstanding interest are:
N-butyl-2-chloro-N-[5-(4-hydroxy-3,5-dimethylphenyl)-1,3,4-thiadiazol-2-yl]benzamide
N-butyl-2-fluoro-N-[5-(4-methoxy-3,5-dimethylphenyl)-1,3,4-thiadiazol-2-yl]benzamide
N-Butyl-2-fluoro-N-[5-(4-hydroxy-3,5-dimethylphenyl)-1,3,4-thiadiazol-2-yl]benzamide
N-butyl-2-methoxy-N-[5-(4-methoxy-3,5-dimethylphenyl)-1,3,4-thiadiazol-2-yl]benzamide
N-butyl-2-chloro-N-{5-[4-(2-methoxyethoxy)-3,5-dimethylphenyl]-l,3,4-thiadiazol-2-yl}benzamide
2-Chloro-N-[5-(4-methoxy-3,5-dimethylphenyl)-1,3,4-thiadiazol-2-yl]-N-(2-methoxyethyl)benzamide
N-Butyl-N-[5-(2-chloro-6-methoxypyridin-4-yl)-1,3,4-thiadiazol-2-yl]-2-fluorobenzamide
2-fluoro-N-[5-(4-methoxy-3,5-dimethylphenyl)-1,3,4-thiadiazol-2-yl]-N-propylbenzamide
N-Butyl-2-fluoro-N-[5-(2-methylpyridin-4-yl)-1,3,4-thiadiazol-2-yl] benzamide N-(cyclopropylmethyl)-2-fluoro-N-[5-(4-methoxy-3,5-dimethylphenyl)-1,3,4-thiadiazol-2-yl]benzamide
3-(4-{5-[butyl(2-fluorobenzoyl)amino]-1,3,4-thiadiazol-2-yl}-2,6-dimethylphenyl)propanoic acid
N-Butyl-2-chloro-N-[5-(2-chloro-6-methylpyridin-4-yl)-1,3,4-thiadiazol-2-yl]benzamide
3-(4-(5-(N-butyl-2-chlorobenzamido)-1,3,4-thiadiazol-2-yl)benzamido) propanoic acid
2-fluoro-N-(5-(4-methoxy-3,5-dimethylphenyl)-1,3,4-thiadiazol-2-yl)-N-phenethylbenzamide
2-(4-(5-(N-butyl-2-fluorobenzamido)-1,3,4-thiadiazol-2-yl)benzamido)acetic acid

Compounds of general formula (I) may be prepared following the synthetic scheme depicted in figure 1. 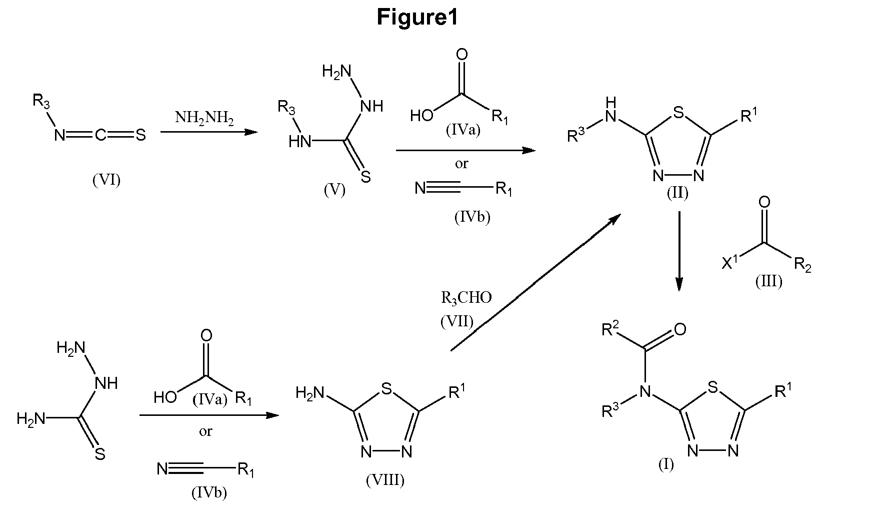 Compounds of general formula (I) may be prepared by the reaction of 1,3,4-thiadiazol-2-amine derivatives (II), wherein R¹ and R³ are as described above, with the corresponding acylating agent (III), wherein R2 is as described above and X¹ represents a hydroxy group or a chlorine atom. When X¹ is a chlorine atom, the reaction takes place in basic media such as triethylamine, pyridine or diisopropylethylamine with a solvent such as dichloromethane, DMF, THF or pyridine at a temperature between 20 and 150°C and in a standard reactor or in a microwave apparatus. These reactions may be catalyzed by 4-dimethylaminopyridine. When X¹ is a hydroxy group, a coupling agent is used such as 2-(1H-7-Azabenzotriazol-1-yl)-1,1,3,3-tetramethyl uronium hexafluorophosphate (HATU) or O-Benzotriazole-N,N,N',N'-tetramethyl-uronium-hexafluoro-phosphate HBTU, with a base such as triethylamine, pyridine or diisopropylethylamine, in the presence of a solvent such as dichloromethane, DMF, THF or pyridine, at a temperature between 20 and 150°C and in a standard reactor or in a microwave apparatus. Other coupling agents such as 1-[3-(dimethylamino)propyl]-3-ethylcarbodiimide hydrochloride EDC or dicyclohexylcarbodiimide DCC, may be used in the presence of N-hydroxybenzotriazole HOBt as a catalyst in a solvent such as dichloromethane, DMF or THF at a temperature between 20 and 150°C and in a standard reactor or in a microwave apparatus.

Compounds of general formula (II) may be prepared by condensation of the compounds of formula (V) with the corresponding acid derivative (IVa) using POCl₃. Alternatively they may be prepared by condensation of compounds of formula (V) with the corresponding nitrile (IVb) in the presence of trifluoroacetic acid. Both reactions may be performed without a solvent or in a solvent such as dioxane or THF or dichloromethane at a temperature from 20 to 150°C.

Intermediates of formula (V) may be obtained by the reaction of hydrazine hydrate with the corresponding isothiocyanate (VI) in a solvent such as THF, methanol or ethanol and at a temperature from 0 to 40°C.

Alternatively, Intermediates of general formula (II) may be prepared by reductive amination of compounds of general formula (VIII) with the corresponding aldehyde (VII) in acid media such as acetic acid and in a protic solvent such as methanol or ethanol and with a reductive agent such as sodium borohydride or sodium cyanoborohydride at a temperature from 0°C to the boiling point of the solvent.

Intermediates of general formula (VIII) may be prepared by condensation of hydrazinecarbothioamide with the corresponding acid derivative (IVa) using POCl₃ or with the corresponding nitrile derivative (IVb) in the presence of TFA. Both reactions may be performed without a solvent in a solvent such as dioxane or THF or dichloromethane at a temperature from 20 to 100°C.
In the particular case where R¹ represents a group of formula 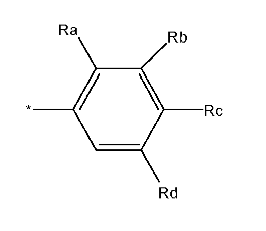 and Rc is a C₁₋₄ alkoxy group of formula -OG, wherein G is the alkyl radical of the alkoxy group which is substituted with one or more substituents, the compounds of formula (Ia) may be obtained following the synthetic path shown in Figure 2. 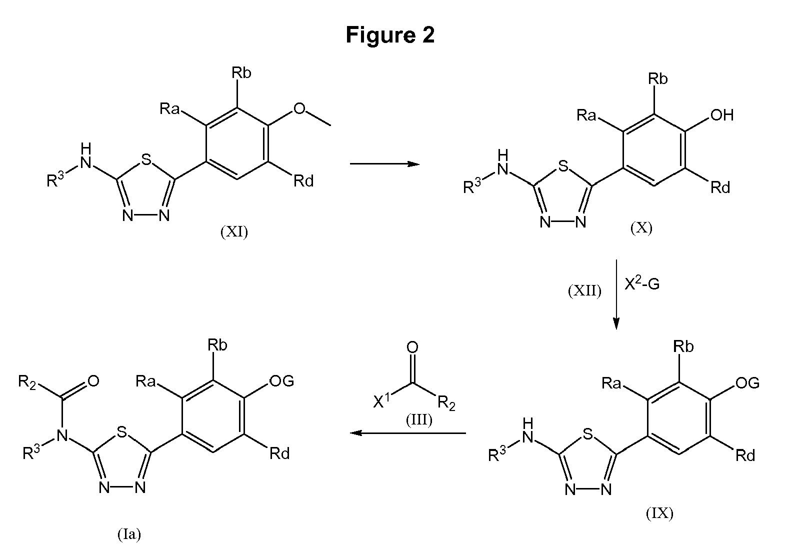 The compounds of formula (Ia) may be obtained by the reaction of 1,3,4-thiadiazol-2-amine derivatives (IX), wherein Ra, Rb, Rd, R³ and G are as described above, with the corresponding acylating agent (III) wherein R² and X¹ are as decribed above, following the same procedure used for the preparation of compounds of formula (I).

1,3,4-Thiadiazol-2-amine derivatives of formula (IX) may be obtained by the reaction of the phenol derivatives of formula (X) with the corresponding alkylating agent (XII), wherein X² is an halogen atom such as chlorine, bromine or iodide in basic media such as sodium hydride in a solvent such as THF or DMF at a temperature from 0 to 150°C.

Alternatively, the phenolic functionality of (X) may be coupled to suitable alcohol derivatives HO-G, wherein G is as defined above, using a Mitsunobu coupling procedure (Mitsunobu, O., Synthesis 1 (1981)). Preferred coupling conditions include the use of a trialkylphosphine or triarylphosphine, such as tri-n-butylphosphine or triphenylphosphine, in a suitable solvent, such as tetrahydrofuran or dichloromethane, and an azodicarbonyl reagent, such as diethyl azodicarboxylate or 1,1'-(azodicarbonyl)dipiperidine.

The compounds of general formula (X) may be prepared by demethylation of the corresponding compound of general formula (XI) using BBr₃ or AlBr₃ or BF₃ as demethylating agent in a solvent such as dichloromethane or 1,2-dichloroethane, chloroform at a temperature between 0 and the 60°C. Alternatively compounds of general formula (X) may be prepared by demethylation using HBr in acetic acid as a solvent.

Finally, compounds of formula (XI) may be obtained from the condensation of the compounds of general formula (V) with the corresponding acid derivative (IVa) or nitrile derivative (IVb) or by the reductive amination of compounds of general formula (VIII) with the corresponding aldehyde of general formula (VII), as described for the compounds of formula (II) depicted in figure 1.

In the particular case where R¹ represents a group of formula, 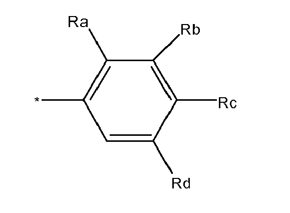 Rc is -(CH₂)₍₀₋₄₎-L-R⁵ and L is a group of formula 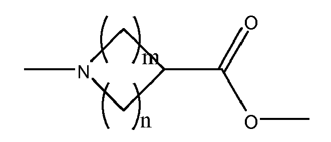 wherein n and m are as described above, the compound of formula (Ib) may be obtained following the synthetic path shown in Figure 3.

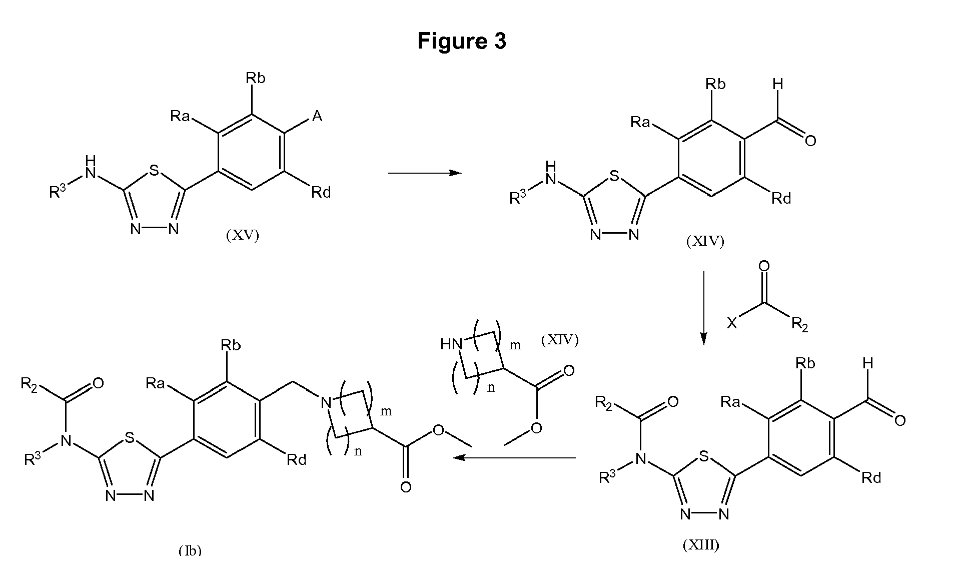

Compounds of general formula (Ib), wherein Ra, Rb, Rd, R², R³, n and m are as decribed above may be prepared by the reductive amination of the aldehyde derivatives of general formula (XIII) with the corresponding aminoacid of formula (XIV) in acid media such as acetic acid and in a protic solvent such as methanol or ethanol and with a reductive agent such as sodium borohydride or sodium cyanoborohydride at a temperature from 0°C to the boiling point of the solvent.

Compounds of formula (XIII) may be obtained by acylation of compounds of general formula (XIV) with the corresponding acylating agent of formula (III) by standard methods as described before.

Finally, aldehyde derivatives of general formula (XIV) may be obtained by reduction of the corresponding nitrile of general formula (XV), wherein A represents CN, by using a reductive agent such as DIBAL-H in a solvent such as dichloromethane, THF or dioxane at a low temperature from -78° to 0°C.

Alternatively, compounds of general formula (XIV) may also be obtained by reduction of the corresponding ester (XV), wherein A represents a -COOR radical, to the alcohol using LiAlH₄ as reductive agent in a solvent such as THF and the following oxidation to the corresponding aldehyde of formula (XIV) by a standard Swern oxidation or other oxidazing agents such as Dess-Martin reagent.

In the particular case where R¹ represents a group of formula 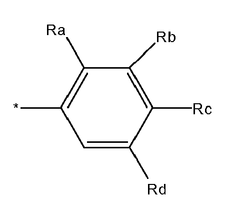 and Rc is CH₂-NH-(CH₂)₍₀₋₄₎-COOH, the compounds of formula (Ic) may also be obtained by the reductive amination of the aldehyde derivatives of general formula (XIII) with the corresponding aminoacid of formula (XVI) in acid media such as acetic acid and in a protic solvent such as methanol or ethanol and with a reductive agent such as sodium borohydride or sodium cyanoborohydride at a temperature from 0°C to the boiling point of the solvent as shown in figure 4. 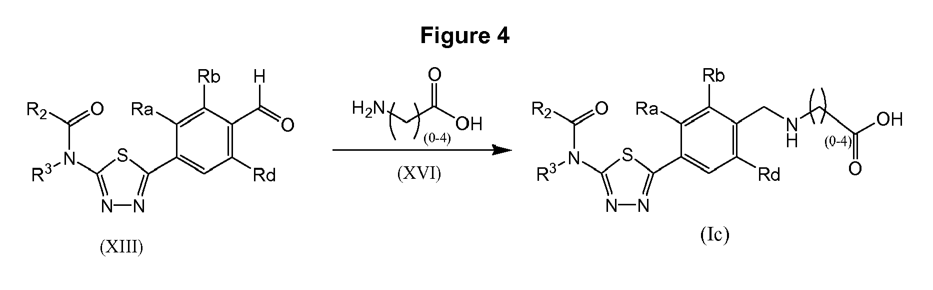 The compounds of general formula (Ib) may be prepared by the reductive amination of the aldehyde derivatives of general formula (XIII) with the corresponding aminoacid of formula (XVI) in acid media such as acetic acid and in a protic solvent such as methanol or ethanol and with a reductive agent such as sodium borohydride or sodium cyanoborohydride at a temperature from 0°C to the boiling point of the solvent.
In the particular case where R¹ represents a group of formula 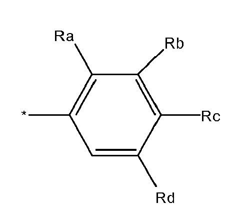 and Rc is -C(O)NH-R⁵ the compounds of formula (Id) may be obtained following the synthetic path shown in Figure 5.

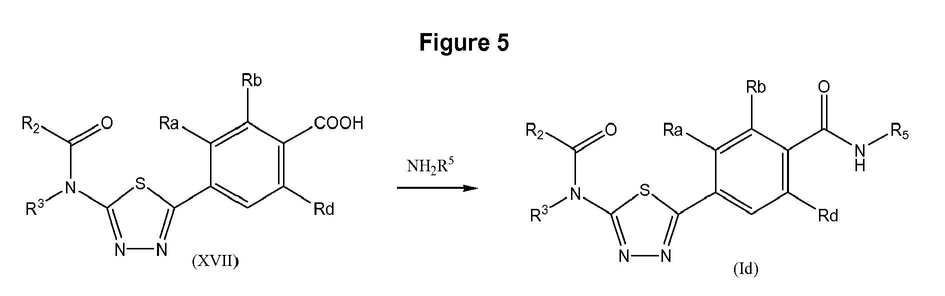

Reaction of the acid derivatives of general formula (XVII) with the corresponding amine using as a coupling agent HATU or HBTU with a base such as triethylamine, pyridine or diisopropylethylamine, with a solvent such as dichloromethane, DMF, THF or pyridine, or using as a coupling agent EDC or DCC, with HOBt and in a solvent such as dichloromethane, DMFor THF, yield the final compounds of formula (Id).

When the defined groups R¹ to R⁵ and Ra to Rd are susceptible to chemical reaction under the conditions of the hereinbefore described processes or are incompatible with said processes, conventional protecting groups may be used in accordance with standard practice, for example see T.W. Greene and P.G.M. Wuts in "protective Groups in Organic Chemistry", 3rd Edition, John Wiley&Sons (1999). It may be that deprotection will form the last step in the synthesis of compounds of formula (I).

The syntheses of the compounds of the invention and their intermediates are illustrated by the following Examples (1 to 57) including Preparation Examples (1 to 63) which do not limit the scope of the invention in any way.

Starting compounds are commercially available or may be obtained following the conventional synthetic method already known in the art.

¹H Nuclear Magnetic Resonance Spectra were recorded on a Varian Mercury 200 spectrometer. Low Resolution Mass Spectra (m/z) were recorded on a Micromass ZMD mass spectrometer using ESI ionization. The chromatographic separations were obtained using a Waters 2690 system equipped with a Symmetry C18 (2.1 x 50 mm, 3.5 µM) column for method A and B and a Symmetry C18 (2.1 x 100 mm, 3.5 µM) for method C. The mobile phase was formic acid (0.4 mL), ammonia (0.1 mL), methanol (500 mL) and acetonitrile (500 mL) (B) and formic acid (0.46 mL), ammonia (0.115 mL) and water (1000 mL) (A), the gradients are specified in the following table for each methode used. The reequilibration time between two injections was 1 min. The flow rate was 0.8 mL/min for method A and 0.4 mL/min for method B and C. The injection volume was 5 microliter for method A and B and 3 microliter for method C. Diode array chromatograms were collected at 210 nM.

| Method | 0% B | 0 to 95% B | 95% B |
|---|---|---|---|
| A | 0.2 min | 3min | 0.8min |
| B | 0.5min | 6.5min | 1min |
| C | 0min | 20min | 4min |

### Purification method A:

The solid was dissolved in DMSO/MeOH, injected into a Biotage C18 silica column (40M, 25M or 25S according to the crude amount) and eluted on the SP1® automated purification system from Biotage. The gradient used was H2O/Acetonitrile/MeOH (1:1) (0.1% v/v HCOONH4 both phases) from 0% to 100% acetonitrile/MeOH (1:1) in 80 column volumes. The appropriate fractions were collected and the organic solvent evaporated under reduced pressure or liofilized.

### PREPARATION EXAMPLES

### PREPARATION 1

### N-Butylhydrazinecarbothioamide

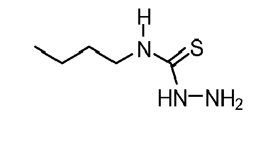

To a 10°C stirred solution of hydrazine hydrate (32 ml, 0.66 mol) in methanol (500 ml), butylisothiocyanate (25 g, 0.22 mol) was added dropwise keeping the temperature between 10-15°C. The final mixture was stirred for 1 h at 10°C and the solvent was removed to yield an oil that was lyophilized. A white solid was obtained. It was washed with diethyl ether. 28g (87% yield).
LRMS: m/z 148 (M+1)⁺
Retention time: 4.17min (method B)
¹H NMR (200 MHz, CDCl₃) δ ppm 1.0 (t, *J*=7.0 Hz, 3 H) 1.4 (m, 2 H) 1.6 (m, 2
H) 3.6 (m, 2 H) 3.8 (s, 2 H) 7.4 (s, 1 H) 7.8 (s, 1 H)

### PREPARATION 2

### 4-(5-(Butylamino)-1,3,4-thiadiazol-2-yl)benzenesulfonamide

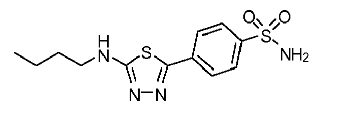

To a stirred suspension of 4-sulfamoylbenzoic acid (1.36 g, 6.8 mmol) and N-butylhydrazinecarbothioamide (Preparation1) (1 g, 6.8 mmol) in dioxane (8 ml), POCl3 (1.46 ml, 15.7 mmol) was added dropwise and the final mixture was stirred at 70°C for 3 h. The mixture was let to cool down and was carefully poured onto ice water. The solid thus obtained was filtered and suspended in water. It was basified to pH 12 and the solution thus formed was neutralized. The solid thus formed was filtered and thoroughly washed with water and hexanes to yield 1.26 g (63%) of the title compound.
LRMS: m/z 313 (M+1)⁺
Retention time: 10.31min (method C)
¹H NMR (200 MHz, DMSO-*d*₆) δ ppm 0.92 (t, *J*=7.22 Hz, 3 H) 1.11 - 1.79 (m, 4
H) 3.40 (m, 2H) 7.47 (s, 2 H) 7.68 - 8.03 (m, 4 H) 8.12 (t, *J*=5.27 Hz, 1 H)

### PREPARATION 3

### Tert-butyl ({4-[5-(butylamino)-1,3,4-thiadiazol-2-yl]phenyl}sulfonyl)carbamate

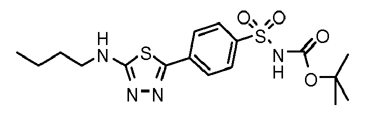

To a stirred suspension of the title compound of Preparation 2 (1 g, 3.20 mmol) in dichloromethane (30 ml), ethyldiisoproplylamine (0.64 ml, 3.69 mmol) and 4-DMAP (391 mg, 3.2 mmol) were added and the mixture was stirred for a while. Then Boc₂O (803 mg, 3.69 mmol) was added and the final mixture was stirred at rt overnight. The reaction crude was diluted with dichloromethane and washed with water and HCl 2 M. The organic layer was dried and solvent was removed to yield 1.32 g of a 1:1 mixture of the title compound and 4-DMAP which was used without further purification. Yield 75%
LRMS: m/z 413 (M+1)⁺
Retention time: 3.21 min (method A)
1 H NMR (200 MHz, DMSO-*d*₆) δ ppm 0.91 (t, *J*=7.22 Hz, 3 H) 1.15 (s, 9H) 1.35
(m, 2 H) 1.60 (m, 2 H) 3.52 (q, 2H) 7.75 (s, 4H) 8.10 (t, 1 H)

### PREPARATION 4

### tert-Butyl 4-(5-(N-butyl-3-methoxybenzamido)-1,3,4-thiadiazol-2-yl)phenylsulfonylcarbamate

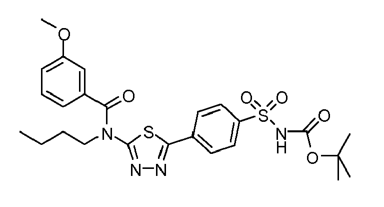

To a stirred solution of the title compound of Preparation 3 (88 mg, 0.52 mmol) in dichloromethane (5 ml), ethyldiisopropylamine (0.360 ml, 2.07 mmol) and 3-methoxybenzoyl chloride were added and the mixture was stirred at rt overnight. Solvent was removed and the solid thus obtained was purified according to purification method A. 120 mg (44% yield) were obtained.
LRMS: m/z 547 (M+1)⁺
Retention time: 17.11 min (method C)
¹H NMR (200 MHz, DMSO-*d*₆) δ ppm 0.91 (t, 3 H) 1.18 (q, 2H) 1.35 (s, 9 H)
1.60 (m, 2 H) 3.81 (s, 3H) 4.15 (t, 2H) 7.25 (m, 3H) 7.45 (m, 1 H) 8.02 (d, 2H)
8.25 (d, 2H)

### PREPARATION 5

### tert-Butyl 4-(5-(N-butyl-2-naphthamido)-1,3,4-thiadiazol-2-yl)phenylsulfonylcarbamate

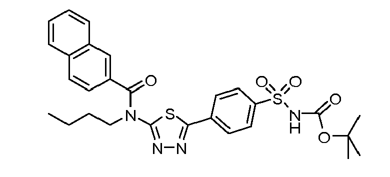

Obtained (60% yield) from the title compound of Preparation 3 and 2-naphtoyl chloride following the experimental procedure of Preparation 4.
LRMS: m/z 567 (M+1)⁺
Retention time: 3.85min (method A)
¹H NMR (200 MHz, DMSO-D6) δ ppm 0.7 (t, *J*=7.2 Hz, 3 H) 1.1 (m, 2 H) 1.3 (s,
9H) 1.7 (m, 2 H) 4.2 (m, 2 H) 7.7 (m, 3 H) 8.1 (m, 6 H) 8.3 (m, 3 H)

### PREPARATION 6

### tert-Butyl 4-(5-(N-butylbiphenyl-4-ylcarboxamido)-1,3,4-thiadiazol-2-yl)phenylsulfonylcarbamate

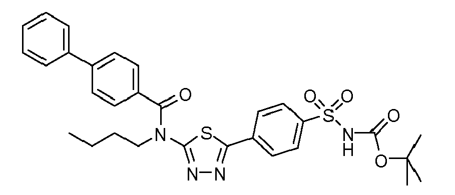

Obtained (49% yield) from the title compound of Preparation 3 and biphenyl-4-carbonyl chloride following the experimental procedure of Preparation 4.
LRMS: m/z 593(M+1)⁺
Retention time: 3.89min (method A)
¹H NMR (200 MHz, DMSO-D6) δ ppm 0.7 (t, *J*=7.4 Hz, 3 H) 1.2 (m, 2 H) 1.3 (s,
9 H) 1.7 (m, 2 H) 4.2 (m, 2 H) 7.5 (m, 3 H) 7.8 (d, *J*=6.6 Hz, 4 H) 7.9 (m, 4 H)
8.1 (d, *J*=9.8 Hz, 2 H) 8.1 (s, 1 H)

### PREPARATION 7

### tert-Butyl 4-(5-(4-butoxy-N-butylbenzamido)-1,3,4-thiadiazol-2-yl)phenylsulfonylcarbamate

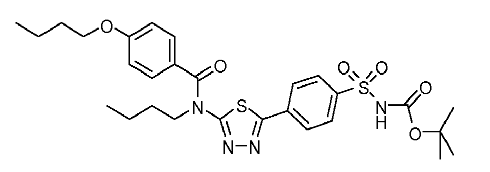

Obtained (49% yield) from the title compound of Preparation 3 and 4-butoxybenzoyl chloride following the experimental procedure of Preparation 4.
LRMS: m/z 589(M+1)⁺
Retention time: 3.95min (method A)
¹H NMR (200 MHz, DMSO-D6) δ ppm 0.7 (t, *J*=7.2 Hz, 3 H) 0.9 (t, *J*=7.2 Hz, 3
H) 1.1 (m, 2 H) 1.3 (s, 9 H) 1.5 (m, 2 H) 1.7 (m, 4 H) 4.1 (t, *J*=6.4 Hz, 2 H) 4.2
(m, 2 H) 7.1 (d, *J*=9.0 Hz, 2 H) 7.6 (d, *J*=8.6 Hz, 2 H) 7.9 (d, *J*=7.4 Hz, 2 H) 8.1
(m, 3 H)

### PREPARATION 8

### tert-Butyl 4-(5-(N-butylbenzamido)-1,3,4-thiadiazol-2-yl)phenylsulfonylcarbamate

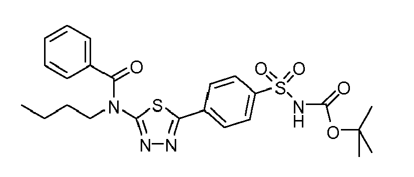

Obtained (20% yield) from the title compound of Preparation 3 and benzoyl chloride following the experimental procedure of Preparation 4.
LRMS: m/z 517(M+1)⁺
Retention time: 3.71 min (method A)

### PREPARATION 9

### N-Butyl-5-(4-methoxy-3,5-dimethylphenyl)-1,3,4-thiadiazol-2-amine

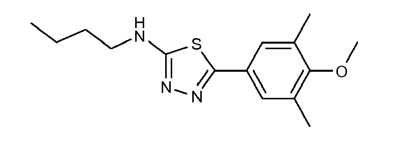

Obtained (99% yield) from the title compound of Preparation 1 and 4-methoxy-3,5-dimethylbenzoic acid following the experimental procedure of Preparation 2. The final product was recrystallized from isopropanol.
LRMS: m/z 292(M+1)⁺
Retention time: 6.67min (method B)
¹H NMR (200 MHz, DMSO-*d*₆) δ ppm 0.92 (t, *J*=7.22 Hz, 3 H) 1.39 (dq, *J*=14.54,
7.13 Hz, 2 H) 2.28 (m, 2 H) 3.38 (t, *J*=6.64 Hz, 2 H) 3.70 (s, 3 H) 4.58 - 5.60 (m,
6 H) 7.48 (s, 2 H).

### PREPARATION 10

### 5-(4-Bromophenyl)-N-butyl-1,3,4-thiadiazol-2-amine

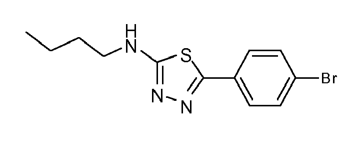

To a stirred solution of 5-(4-bromophenyl)-1,3,4-thiadiazol-2-amine (2.0 g, 7.81 mmol) in methanol (60ml), butyraldehyde (2.13 g, 29 mmol) and AcOH (two drops) were added and the mixture was stirred at 60°C for 48h. Then it was cooled to 0°C and NaBH4 (1.12 g, 29 mmol) was added portionwise and the final mixture was stirred at rt for 2h. Solvent was removed and the solid thus obtained was partitioned between dichloromethane and water. The organic layer was washed with water and brine. It was dried and solvent removed to yield a crude product that was purified on a Biotage 40S chromatography column of silica gel using mixtures of hexane/ethyl acetate as eluent. 2.44 g (25% yield).
LRMS: m/z 312(M+1)⁺
Retention time: 6.77min (method B)
¹H NMR (200 MHz, DMSO-*d*₆) δ ppm 0.91 (t, *J*=7.03 Hz, 3 H) 1.09 - 1.81 (m, 4
H) 7.83 (d, 2 H) 8.01 (m, 3 H)

### PREPARATION 11

### N-(5-(4-bromophenyl)-1,3,4-thiadiazol-2-yl)-N-butyl-3-methoxybenzamide

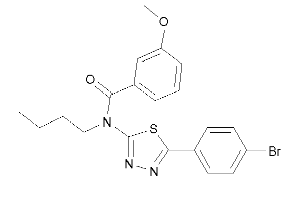

To a stirred suspension of the title compound of Preparation 10 (1.0 g, 3.20 mmol) and diisopropylethylamine (2.23 ml, 12.8 mmol) in dioxane (20 ml), 3-methoxybenzoyl chloride (1.10 g, 6.45 mmol) was added and the mixture was stirred at 80°C for 4 h and then at rt overnight. Solvent was removed and the mixture was partitioned between water and ethyl acetate. The organic layer was washed with HCl 2N, with potassium carbonate and brine. Solvent was removed and the crude thus obtained was purified by column cromathography using a 40S Biotage column and a gradient from 100% hexane to hexane/ethyl ether 80:20. 450 mg (31 % yield) of the title product were obtained.
LRMS: m/z 446(M+1)⁺
Retention time: 7.66min (method B)
¹H NMR (200 MHz, DMSO-*d*₆) δ ppm 0.73 (t, *J*=7.22 Hz, 3 H) 0.95 - 1.31 (m, 2
H) 1.70 (m, 2 H) 3.82 (s, 3 H) 4.10 (t, *J*=7.22 Hz, 2 H) 7.20 (d, *J*=8.59 Hz, 2 H)
7.49 (d, *J*=7.61 Hz, 2 H) 7.64 - 7.85 (m, 2 H) 7.95 (d, *J*=8.20 Hz, 2 H)

### PREPARATION 12

### Tert-butyl (2E)-3-{4-[5-(butylamino)-1,3,4-thiadiazol-2-yl]phenyl}acrylate

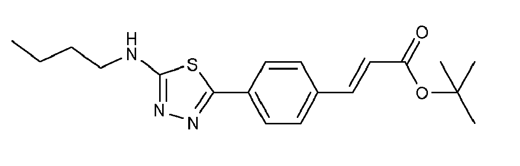

Under nitrogen atmosphere, a mixture of the title compound of Preparation 11 (300 mg, 0.67 mmol), tert-butyl acrylate (129 mg, 1.06 mmol), potassium carbonate (186 mg, 1.35 mmol) N,N-dimethylalanine (6.3 mg, 0.54 mmol) and palladium (II) acetate (1.5 mg, 0.006 mmol) in NMP (3 ml) was stirred at 120°C overnight. It was let to cool down and the poured onto water and extracted with ethyl acetate. The organic layer was washed with water. Solvent was removed to yield 200 mg (80%) of the title compound.
LRMS: m/z 360 (M+1)⁺
Retention time: 7.30min (method B)

### PREPARATION 13

### Tert-butyl 3-(4-(5-(butylamino)-1,3,4-thiadiazol-2-yl)phenyl)propanoate

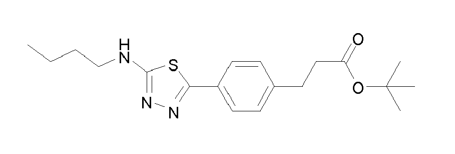

A mixture of the title compound of Preparation 12 (200 mg, 0.56 mmol) and Pd/C with a 50% of water (20 mg) in methanol (200ml) was stirred under hydrogen at 20 psi overnight. 20 mg more of the palladium catalyst were added and the mixture was stirred under hydrogen at the same pressure for 3 days. The catalyst was filtered off and solvent was removed to yield a crude product that was purified following purification method A. 85 mg of the title compound (42%) were obtained.
LRMS: m/z 362 (M+1)⁺
Retention time: 7.06min (method B)
¹H NMR (200 MHz, DMSO-D6) δ ppm 0.9 (t, *J*=7.2 Hz, 3 H) 1.3 (m, 2 H) 1.4 (s,
9 H) 1.6 (m, 2 H) 2.6 (m, 2 H) 2.8 (t, *J*=7.2 Hz, 2 H) 3.2 (m, 2 H) 7.3 (d, *J*=7.8
Hz, 2 H) 7.7 (d, *J*=7.8 Hz, 2 H) 7.9 (t, *J*=5.5 Hz, 1 H)

### PREPARATION 14

### tert-Butyl 3-(4-(5-(N-butyl-3-methoxybenzamido)-1,3,4-thiadiazol-2-yl)phenyl)propanoate

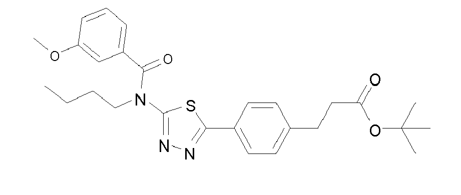

Obtained (63%) from the title compound of Preparation 13 and 3-methoxybenzoyl chloride following the experimental procedure of Preparation 11.
LRMS: m/z 496 (M+1)⁺
Retention time: 3.89min (method A)

### PREPARATION 15

### 5-(4-Methoxy-3,5-dimethylphenyl)-N-methyl-1,3,4-thiadiazol-2-amine

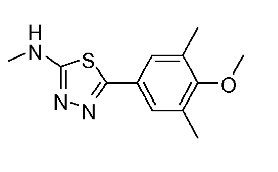

Obtained (65%) from 4-methoxy-3,5-dimethylbenzoic acid and N-methylhydrazinecarbothioamide following the experimental procedure of Preparation 2.
LRMS: m/z 250 (M+1)⁺
Retention time: 5.73min (method B)
¹H NMR (200 MHz, DMSO-D6) δ ppm 2.3 (s, 6 H) 2.9 (s, 3 H) 3.7 (s, 3 H) 7.4
(s, 2 H) 7.8 (s, 1 H)

### PREPARATION 16

### N-Butyl-5-(3,4-dimethoxyphenyl)-1,3,4-thiadiazol-2-amine

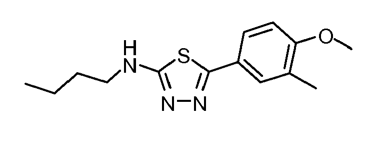

Obtained (89%) from 4-methoxy-3-methylbenzoic acid and N-butylhydrazinecarbothioamide following the experimental procedure of Preparation 2.
LRMS: m/z 278 (M+1)⁺
Retention time: 3.48min (method A)

### PREPARATION 17

### N-Butyl-5-(3-chloro-4-methoxyphenyl)-1,3,4-thiadiazol-2-amine

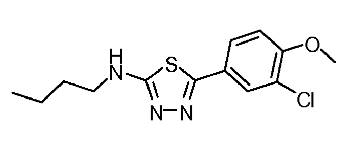

Obtained (68%) from 3-chloro-5-methoxybenzoic acid and N-butylhydrazinecarbothioamide following the experimental procedure of Preparation 2.
LRMS: m/z 298 (M+1)⁺
Retention time: 3.44min (method A)
¹H NMR (200 MHz, DMSO-D6) δ ppm 0.9 (t, *J*=7.2 Hz, 3 H) 1.4 (m, 2 H) 1.6 (m,
2 H) 3.3 (m, 2 H) 3.9 (s, 3 H) 7.2 (d, *J*=9.0 Hz, 1 H) 7.7 (dd, *J*=8.6, 2.3 Hz, 1 H)
7.8 (d, *J*=2.3 Hz, 1 H) 7.9 (t, *J*=5.5 Hz, 1 H)

### PREPARATION 18

### 4-(5-(Butylamino)-1,3,4-thiadiazol-2-yl)benzoic acid methyl esther

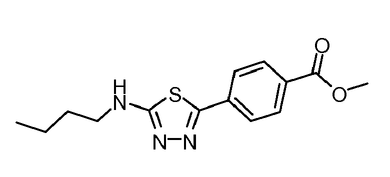

Obtained (38%) from 4-(methoxycarbonyl)benzoic acid and N-butylhydrazinecarbothioamide following the experimental procedure of Preparation 2.
LRMS: m/z 292 (M+1)⁺
Retention time: 3.33min (method A)
¹H NMR (200 MHz, DMSO-D6) δ ppm 0.9 (t, *J*=7.2 Hz, 3 H) 1.4 (m, 2 H) 1.6 (m,
2 H) 3.3 (m, 2 H) 3.9 (s, 3 H) 7.9 (d, *J*=8.6 Hz, 2 H) 8.0 (m, 2 H) 8.1 (t, *J*=5.3 Hz, 1 H)

### PREPARATION 19

### 4-(5-(Butylamino)-1,3,4-thiadiazol-2-yl)-2,6-dimethylphenol

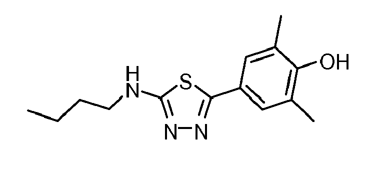

To a stirred suspension of the title product of Preparation 9 ( 2.0 g, 6.86 mmol) in dichloromethane (20 ml) at -78°C, a 1 M BBr3 solution in dichloromethane (10.3 ml, 10.3 mmol) was added and the mixture was stirred at that temperature for 30 min and then a rt overnight. The reaction mixture was poured onto ice water, diluted with dichloromethane and neutralized with saturated solution of potassium bicarbonate. The organic layer was collected and solvent was removed to yield a solid that was washed with ethyl ether. 1.64 g (87% yield) of the title product were obtained.
LRMS: m/z 278 (M+1)⁺
Retention time: 6.09min (method B)
¹H NMR (200 MHz, DMSO-D6) δ ppm 0.9 (t, *J*=7.2 Hz, 3 H) 1.4 (m, 2 H) 1.6 (m,
2 H) 2.2 (s, 6 H) 3.3 (m, 2 H) 7.3 (s, 2 H) 8.0 (s, 1 H) 8.8 (s, 1 H)

### PREPARATION 20

### N-Butyl-5-(4-((2,2-dimethyl-1,3-dioxolan-4-yl)methoxy)-3,5-dimethylphenyl)-1,3,4-thiadiazol-2-amine

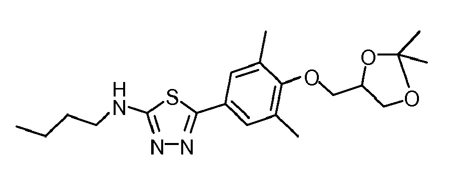

To a stirred solution of the title compound of Preparation 19 (1.0 g, 3.61 mmol) in DMF (15 ml), 60% sodium hydride (0.29 g, 7.25 mmol) was added portionwise and the mixture was stirred at rt for 30 min. Then, 4-(chloromehtyl)-2,2-dimethyl-1,3-dioxolane (0.60 ml, 4.22 mmol) was added and the mixture was stirred at 120°C overnight. Solvent was removed and the residue was partitioned between water and ethyl acetate. The organic layer was washed with water and brine and solvent was finally removed toyield a crude product that was purified according to purification method A. 0.40 g (28% yield) of the title product were obtained.
LRMS: m/z 392 (M+1)⁺
Retention time: 7.12min (method B)
¹H NMR (200 MHz, DMSO-D6) δ ppm 0.9 (t, *J*=7.2 Hz, 3 H) 1.3 (m, 8 H) 1.6 (m,
2 H) 2.3 (s, 6 H) 3.3 (m, 2 H) 3.8 (m, 3 H) 4.1 (t, *J*=7.4 Hz, 1 H) 4.4 (m, 1 H) 7.4
(s, 2 H) 7.9 (t, *J*=5.7 Hz, 1 H)

### PREPARATION 21

### N-Butyl-2-chloro-N-(5-{4-[(2,2-dimethyl-1,3-dioxolan-4-yl)methoxy]-3,5-dimethylphenyl}-1,3,4-thiadiazol-2-yl)benzamide

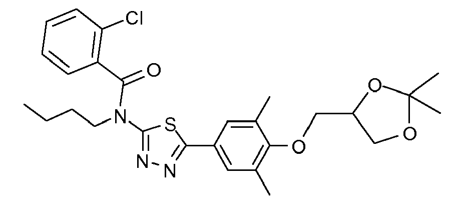

To a stirred suspension of the title compound of Preparation 20 (170 mg, 0.43 mmol) and diisopropylethylamine (0.23 ml, 1.3 mmol) in 1,2-dichloroethane (10 ml), 2-chlorobenzoyl chloride (0.11 ml, 0.87 mmol) and 4-DMAP (27 mg) were added and the mixture was stirred at 60°C overnight. It was diluted with dichloromethane and washed with potassium bicarbonate, saturated solution of citric acid and brine. Solvent was removed and the crude thus obtained was purified accoriding to purification method A. 111 mg (26% yield) of the title product were obtained.
LRMS: m/z 531 (M+1)⁺
Retention time: 20.80min (method C)
¹H NMR (200 MHz, DMSO-*d*₆) ppm 0.7 (t, *J*=7.4 Hz, 3 H) 1.2 (m, 2 H) 1.3 (d,
*J*=10.7 Hz, 6 H) 1.7 (m, 2 H) 2.3 (s, 6 H) 3.8 (m, 4 H) 4.1 (m, 2 H) 4.4 (m, 1 H) 7.7 (m, 6 H)

### PREPARATION 22

### N-Butyl-5-(2-chloro-6-methoxypyridin-4-yl)-1,3,4-thiadiazol-2-amine

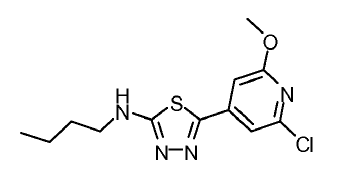

Obtained (70%) from 2-chloro-6-methoxyisonicitinic acid and N-butylhydrazinecarbothioamide following the experimental procedure of Preparation 2.
LRMS: m/z 299 (M+1)⁺
Retention time: 3.61min (method A)
¹H NMR (200 MHz, DMSO-D6) δ ppm 0.9 (t, *J*=7.2 Hz, 3 H) 1.4 (m, 2 H) 1.6 (m,
2 H) 3.3 (m, 2 H) 3.9 (s, 3 H) 7.1 (d, *J*=1.2 Hz, 1 H) 7.4 (d, *J*=1.2 Hz, 1 H) 8.4 (t,
*J*=6.1 Hz, 1 H)

### PREPARATION 23

### N-(2-methoxyethyl)hydrazinecarbothioamide

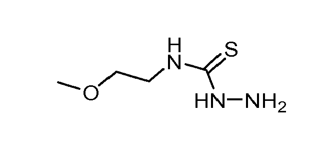

Obtained (89%) from 1-isothiocyanato-2-methoxyethane following the experimental procedure of Preparation 1.
¹H NMR (200 MHz, DMSO-D6) δ ppm 3.2 (s, 3 H) 3.4 (m, 2 H) 3.6 (m, 2 H) 4.5 (s, 2 H) 7.8 (s, 1 H) 8.7 (s, 1 H)

### PREPARATION 24

### 5-(4-Methoxy-3,5-dimethylphenyl)-N-(2-methoxyethyl)-1,3,4-thiadiazol-2-amine

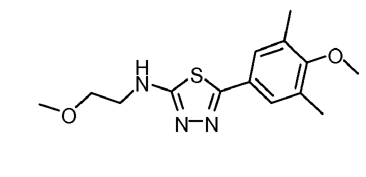

Obtained (23%) from 4-methoxy-3,5-dimethylbenzoic acid and the title compound of Preparation 23 following the experimental procedure of Preparation 2.
LRMS: m/z 294 (M+1)⁺
Retention time: 5.83min (method B)
¹H NMR (200 MHz, CDCl₃) δ ppm 2.3 (s, 6 H) 3.4 (s, 3 H) 3.6 (b.s., 4 H) 3.8 (s,
3 H) 7.5 (s, 2 H)

### PREPARATION 25

### N-ethyl-5-(4-methoxy-3,5-dimethylphenyl)-1,3,4-thiadiazol-2-amine

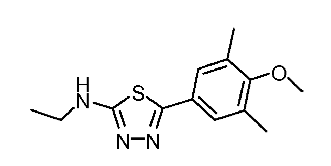

Obtained (83%) from 4-methoxy-3,5-dimethylbenzoic acid and N-ethylhydrazinecarbothioamide following the experimental procedure of Preparation 2.
LRMS: m/z 264 (M+1)⁺
Retention time: 6.13min (method B)
¹H NMR (200 MHz, CDCl₃) δ ppm 1.3 (t, *J*=7.1 Hz, 3 H) 2.3 (s, 6 H) 3.4 (q,
*J*=7.1 Hz, 2 H) 3.7 (s, 3 H) 5.3 (m, 1 H) 7.5 (s, 2 H)

### PREPARATION 26

### N-Butyl-5-(4-(2-methoxyethoxy)-3,5-dimethylphenyl)-1,3,4-thiadiazol-2-amine

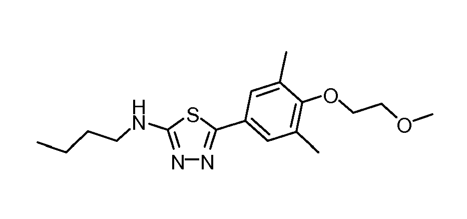

To a stirred solution of the title compound of Preparation 19 (300 mg, 1.08 mmol) in DMF (5 ml), 60% sodium hydride (90 mg, 2.25 mmol) was added portionwise and the mixture was stirred at rt for 30 min. Then, 1-bromo-2-methoxyethane (310 mg, 2.20 mmol) in DMF (2 ml) was added and the mixture was stirred at rt overnight. The reaction mixture was poured onto ice water and is extracted with ethyl acetate twice. The organic layer was washed with water and brine and solvent was finally removed to yield a crude product that was purified according to purification method A. 0.210 g (56% yield) of the title product were obtained.
LRMS: m/z 336 (M+1)⁺
Retention time: 6.75min (method B)
¹H NMR (200 MHz, DMSO-D6) δ ppm 0.9 (t, *J*=7.2 Hz, 3 H) 1.4 (m, 2 H) 1.6 (m,
2 H) 2.3 (s, 6 H) 3.3 (m, 5 H) 3.6 (dd, *J*=5.6, 3.2 Hz, 2 H) 3.9 (dd, *J*=5.6, 3.2 Hz,
2 H) 7.4 (s, 2 H) 7.9 (t, *J*=5.5 Hz, 1 H)

### PREPARATION 27

### N-isopentylhydrazinecarbothioamide

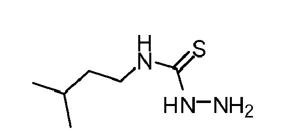

Obtained (86%) from 1-isothiocyanato-3-methylbutane following the experimental procedure of Preparation 1
LRMS: m/z 162 (M+1)⁺
Retention time: 4.95min (method B)
¹H NMR (200 MHz, DMSO-D6) δ ppm 0.9 (d, *J*=6.6 Hz, 6 H) 1.4 (m, 2 H) 1.6
(m, 1 H) 3.5 (m, 2 H) 4.4 (s, 2 H) 7.7 (s, 1 H) 8.5 (s, 1 H)

### PREPARATION 28

### N-Isopentyl-5-(4-methoxy-3,5-dimethylphenyl)-1,3,4-thiadiazol-2-amine

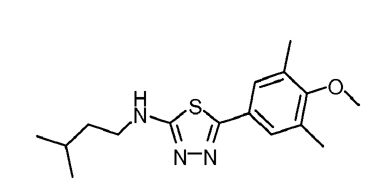

Obtained (72%) from 4-methoxy-3,5-dimethylbenzoic acid and the title compound of Preparation 27 following the experimental procedure of Preparation 2.
LRMS: m/z 306 (M+1)⁺
Retention time: 7.16min (method B)

### PREPARATION 29

### N-(3-(Diethylamino)propyl)hydrazinecarbothioamide

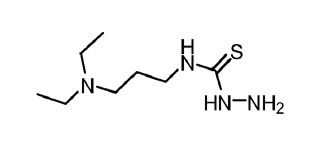

Obtained (96%) from N,N-diethyl-3-isothiocyanatopropan-1-amine following the experimental procedure of Preparation 1.
LRMS: m/z 205 (M+1)⁺
Retention time: 1.07min (method B)

### PREPARATION 30

### N1,N1-diethyl-N3-(5-(4-methoxy-3,5-dimethylphenyl)-1,3,4-thiadiazol-2-yl) propane-1,3-diamine

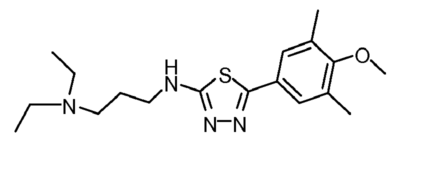

Obtained (20 %) from 4-methoxy-3,5-dimethylbenzoic acid and the title compound of Preparation 29 following the experimental procedure of Preparation 2.
LRMS: m/z 349 (M+1)⁺
Retention time: 4.43min (method B)

### PREPARATION 31

### N-Butyl-5-(2-chloro-6-methylpyridin-4-yl)-1,3,4-thiadiazol-2-amine

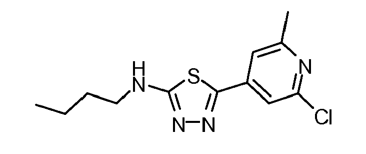

Obtained (30%) from 2-chloro-6-methylisonicitinic acid and N-butylhydrazinecarbothioamide following the experimental procedure of Preparation 2.
LRMS: m/z 283 (M+1)⁺
Retention time: 3.31min (method A)

### PREPARATION 32

### N-propylhydrazinecarbothioamide

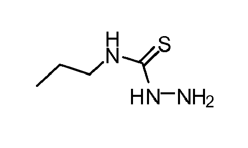

Obtained (88%) from 1-isothiocyanatopropane following the experimental procedure of Preparation 1.
LRMS: m/z 134 (M+1)⁺
Retention time: 3.21min (method B)

### PREPARATION 33

### 5-(4-Methoxy-3,5-dimethylphenyl)-N-propyl-1,3,4-thiadiazol-2-amine

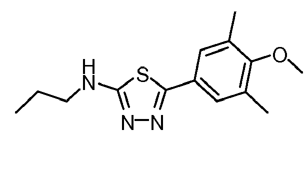

Obtained (76%) from 4-methoxy-3,5-dimethylbenzoic acid and the title compound of Preparation 32 following the experimental procedure of Preparation 2.
LRMS: m/z 278 (M+1)⁺
Retention time: 6.29min (method B)

### PREPARATION 34

### tert-Butyl 2-(4-(5-(butylamino)-1,3,4-thiadiazol-2-yl)-2,6-dimethylphenoxy)acetate

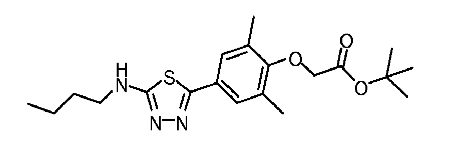

To a 0°C stirred solution of the title compound of Preparation 19 (300 mg, 1.08 mmol) in DMF (5 ml), 60% sodium hydride (43 mg, 1.79 mmol) was added portionwise and the mixture was stirred at rt for 30 min. Then, *tert*-butyl 2-bromoacetate (210 mg, 2.20 mmol) in DMF (1.5 ml) was added and the mixture was stirred at that temperature for 30 min. The reaction mixture was poured onto ice water and was extracted with ethyl acetate twice. The organic layer was washed with water and brine and solvent was finally removed to yield a crude product that was purified according to purification method A. 0.160 g (38% yield) of the title product were obtained.
LRMS: m/z 392 (M+1)⁺
Retention time: 7.28min (method B)
¹H NMR (200 MHz, DMSO-D6) δ ppm 0.9 (m, 3 H) 1.4 (m, 2 H) 1.5 (s, 9 H) 1.6
(m, 2 H) 2.3 (s, 6 H) 3.3 (m, 2 H) 4.4 (s, 2 H) 7.4 (s, 2 H) 7.9 (t, *J*=5.1 Hz, 1 H)

### PREPARATION 35

### N-Butyl-5-(2-chloropyridin-4-yl)-1,3,4-thiadiazol-2-amine

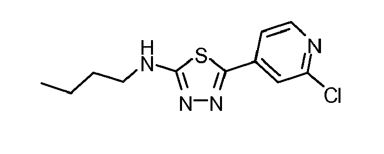

Obtained (18%) from 2-chloroisonicotinic acid and N-butylhydrazinecarbothioamide following the experimental procedure of Preparation 2.
LRMS: m/z 269 (M+1)⁺
Retention time: 3.15min (method A)

### PREPARATION 36

### N-Butyl-5-(2-methoxypyridin-4-yl)-1,3,4-thiadiazol-2-amine

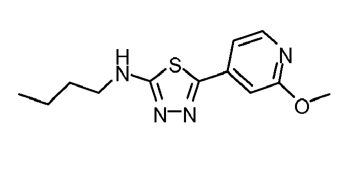

To a stirred solution of the title compound of Preparation 35 in methanol (20ml), sodium (34 mg, 1.48 mmol) was added portionwise and the mixture was stirred at rt overnight. 10 ml of a solution of 100mg of sodium in methanol were further added and the mixture was stirred at 60°C for 4 days. The reaction mixture was poured onto water and extracted with dichloromethane. Solvent was removed to yield 196 mg (73% yield) of the title compound.
LRMS: m/z 265 (M+1)⁺
Retention time: 3.10min (method A)

### PREPARATION 37

### N-(Cyclopropylmethyl)hydrazinecarbothioamide

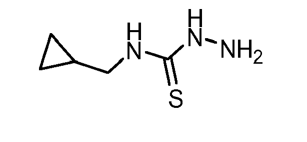

Obtained (88%) from (isothiocyanatomethyl)cyclopropane following the experimental procedure of Preparation 1.
LRMS: m/z 146 (M+1)⁺
Retention time: 3.58min (method B)

### PREPARATION 38

### N-(Cyclopropylmethyl)-5-(4-methoxy-3,5-dimethylphenyl)-1,3,4-thiadiazol-2-amine

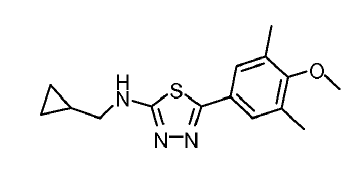

Obtained (100%) from 4-methoxy-3,5-dimethylbenzoic acid and the title compound of Preparation 37 following the experimental procedure of Preparation 2.
LRMS: m/z 290 (M+1)⁺
Retention time: 6.35min (method B)

### PREPARATION 39

### 6-methoxynicotinic acid

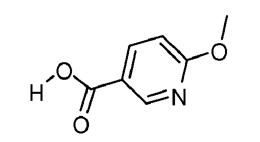

A reaction mixture of 6-metoxynicotinonitrile (4g, 29.8mmol) in 60ml of MeOH and 5M NaOH (60ml, 300mmol) was stirred overnight at 100°C. The mixture was concentrated and redissolved in water (50ml). A 2N solution of HCl was added until pH acid and a solid was formed, filtered and washed with water twice and with hexane twice. 3.04g of the title compound were obtained as a white solid (yield=66%).
LRMS: m/z 154 (M+1)⁺
Retention time: 2.22min (method A)

### PREPARATION 40

### N-butyl-5-(6-methoxypyridin-3-yl)-1,3,4-thiadiazol-2-amine

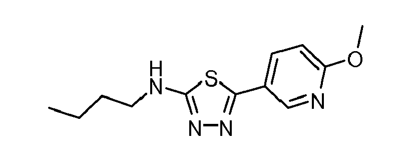

Obtained (31%) from the title compound of Preparation 39 and the title compound of Preparation 1 following the experimental procedure of Preparation 2.
LRMS: m/z 265 (M+1)⁺
Retention time: 3.06min (method A)
¹H NMR (200 MHz, DMSO-d₆) ppm 0.91 (t, J=8 Hz, 3H), 1.39(m, 2H), 1.58(m,
2H), 3.31 (q, J=6Hz, 2H), 3.90(s,3H), 6.93(d,J=8Hz, 1 H), 7.94 (m,1 H), 8.09
(d,J=8Hz, 1 H), 8.52 (s, 1 H)

### PREPARATION 41

### N-butyl-5-(imidazo[1,2-a]pyridin-6-yl)-1,3,4-thiadiazol-2-amine

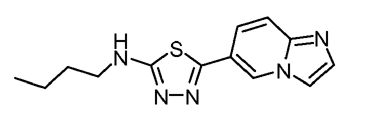

Obtained (47%) from imidazo[1,2-a]pyridine-6-carboxylic acid and the title compound of Preparation 1 following the experimental procedure of Preparation 2.
LRMS: m/z 274 (M+1)⁺
Retention time: 2.08min (method A)
¹H NMR (200 MHz, DMSO-d₆) ppm 0.98(t, J=8 Hz, 3H), 1.46(m, 2H), 1.69(m,
2H), 3.41 (t, J=8Hz, 2H), 5.48 (brs, 1 H), 7.65(m, 4H), 8.64(s, 1 H)

### PREPARATION 42

### Methyl imidazo[1,2-a]pyridine-7-carboxylate

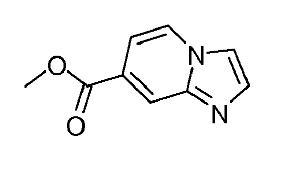

A mixture of 2-bromo-1,1-diethoxyethane (6.20ml, 41.2mmol) and HCl 35% (0.82ml, 26.8mmol) in water (68ml) was stirred for 2.5h, then heated at 80°C and stirred at this temperature for 1.5h. The mixture was cooled down to 20°C and NaHCO₃ (4.49g, 53.45mmol) was added in four portions. Finally, methyl 2-aminoisonicotinate (5g, 32.86mmol) was added and the reaction mixture stirred at room temperature overnight. The solid formed was filtered, washed with water and dried in the vacuum oven to give 5.15g of the title compound as a brown solid (yield=89%).
LRMS: m/z 177 (M+1)⁺
Retention time: 1.46min (method A)
¹H NMR (200 MHz, CHCl₃) ppm 3.97(s, 3H), 7.27(s,1H), 7.42(d, J=6Hz, 1 H),
7.70(s, 1 H), 7.80(s,1H), 8.19(d, J=6Hz, 1 H), 8.37(s,1H)
Yield 89%

### PREPARATION 43

### Imidazo[1,2-a]pyridine-7-carboxylic acid

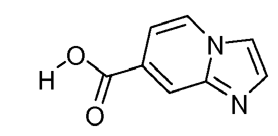

The title compound of Preparation 42 was dissolved in 100ml of HCl 37%. The reaction mixture was heated at 50°C for 64h. After this time the mixture was concentrated to dryness and dried in the vacuum oven to give 1.95g of the title compound as a solid (yield=100%).
LRMS: m/z 163 (M+1)⁺
Retention time: 0.41 min (method A)
¹H NMR (200 MHz, DMSO-d₆) ppm 7.79 (d, J=8Hz, 1 H), 8.31 (s, 1 H),
8.38(s,1H), 8.48(s,1H), 8.97(d,J=8Hz, 1 H)
Yield 99.9%

### PREPARATION 44

### N-butyl-5-(imidazo[1,2-a]pyridin-7-yl)-1,3,4-thiadiazol-2-amine

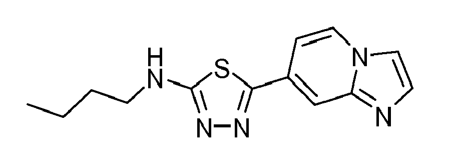

Obtained (76%) from the title compound of Preparation 43 and the title compound of Preparation 1 following the experimental procedure of Preparation 2.
LRMS: m/z 274 (M+1)⁺
Retention time: 1.97min (method A)
¹H NMR (200 MHz, DMSO-D6) δ ppm 0.9 (t, *J*=6.2 Hz, 3 H) 1.4 (m, 2 H) 1.6 (m,
2 H) 3.3 (m, 2 H) 7.4 (d, *J*=6.6 Hz, 1 H) 7.7 (s, 1 H) 7.8 (s, 1 H) 8.1 (m, 2 H) 8.6
(d, *J*=5.1 Hz, 1 H)

### PREPARATION 45

### (4-(5-(butylamino)-1,3,4-thiadiazol-2-yl)phenyl)metanol

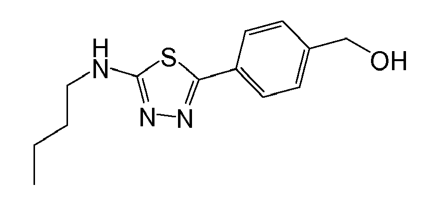

The title compound of preparation 18 (1.5g, 5.15mmol) was dissolved in THF (20ml) and LiAlH₄ (0.2g, 5.27mmol) was added portionwise and the mixture stirred overnight at room temperature. Then to the mixture was added water (2ml), NaOH 32% (4ml), water (4ml) and finally ethyl acetate. The organic layer was collected, dried and concentrated to yield the title compound as a yellow solid (75%).
LRMS: m/z 264 (M+1)⁺
Retention time: 2.75min (method A)
1H NMR (200 MHz, CDCl₃) δ ppm 0.97 (t, *J*=7.22 Hz, 3 H) 1.45 (td, *J*=14.74,
7.22 Hz, 2 H) 1.60 - 1.83 (m, 2 H) 3.37 (t, *J*=7.03 Hz, 2 H) 4.74 (s, 2 H) 5.72 (br.
s., 1 H) 7.42 (d, *J*=8.20 Hz, 2 H) 7.78 (d, *J*=8.59 Hz, 2 H)

### PREPARATION 46

### 4-(5-(butylamino)-1,3,4-thiadiazol-2-yl)benzaldehyde

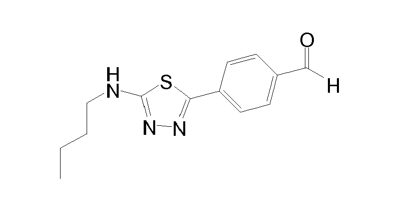

To a solution of oxalyl chloride (0.73ml, 8.35mmol) in DCM (20ml) under argon at - 60°C , DMSO (0.89g, 11.39mmol) was slowly added keeping the temperature at -60°C and the mixture stirred at this temperature for 15min. A suspension of the title compound of preparation 45 (1 g, 3.8mmol) in 10ml of DCM was slowly added to this mixture. Finally, diisopropylethylamine was added (4.40ml, 25.3mmol) and the mixture stirred at -60°C for 1h and at room temperature overnight. Solvent was removed and the residue was solved in ethyl acetate and washed with a 4% solution of NaHCO₃. The organic layer was dried, solvent was removed in vaccuo and the crude was purified according to purification method A to yield the title compound as a solid (yield=18%).
LRMS: m/z 262 (M+1)⁺
Retention time: 5.87min (method B)
¹H NMR (200 MHz, CDCl₃) δ ppm 0.98 (t, *J*=7.22 Hz, 3 H) 1.46 (dd, *J*=15.03,
7.22 Hz, 2 H) 1.72 (t, *J*=3.51 Hz, 2 H) 3.41 (t, *J*=7.03 Hz, 2 H) 7.85 - 8.05 (m, 4
H) 10.04 (s, 1 H)

### PREPARATION 47

### N-butyl-2-fluoro-N-(5-(4-formylphenyl)-1,3,4-thiadiazol-2-yl)benzamide

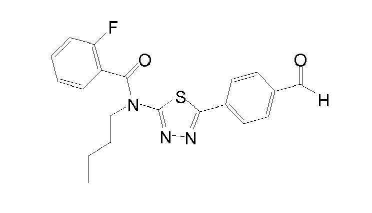

Obtained (18%) from 2-fluorobenzoyl chloride and the title compound of Preparation 46 following the experimental procedure of Preparation 11.
LRMS: m/z 384(M+1)⁺
Retention time: 7.09min (method B)

### PREPARATION 48

### (E)-4-(3-methoxy-3-oxoprop-1-enyl)-2-methylbenzoic acid

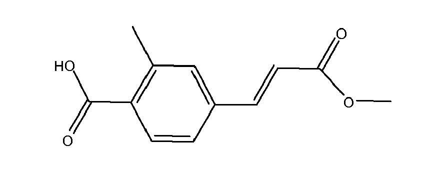

To a mixture of 4-bromo-2-methylbenzoic acid (2g, 9.3mmol), methyl acrylate (0.9g, 10.5mmol), N,N-dimethylalanine (0.1g, 0.85mmol), potassium carbonate (2.6g, 18.8mmol) in NMP (50ml) was added Pd(OAc)₂ (0.1g, 0.45mmol) under nitrogen atmosphere. The mixture was stirred overnight at 120°C. The reaction mixture was then concentrated, redissolved in ethyl ether and washed with water. The organic layer was dried and evaporated to give the title compound as an oil (yield= 77%).
LRMS: m/z 221 (M+1)⁺
Retention time: 5.49min (method B)
¹H NMR (200 MHz, CDCl₃) δ ppm 2.66 (s, 3 H) 3.83 (s, 3 H) 6.52 (d, *J*=16.01
Hz, 1 H) 7.36 - 7.46 (m, 2 H) 7.68 (d, *J*=16.01 Hz, 1 H) 8.05 (d, *J*=7.81 Hz, 1 H)

### PREPARATION 49

### 4-(3-methoxy-3-oxopropyl)-2-methylbenzoic acid

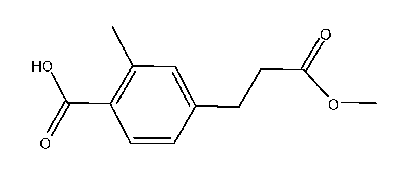

The title compound of preparation 48 (2g, 9.08mmol) was dissolved in methanol (50ml) and after the addition of 10% Pd/C (0.1 g, 0.9mmol) the mixture was hydrogenated at 20psi for 1 h. The catalyst was filtered off and the filtrate concentrated to give the title compound as a brown oil (yield= 85%) which was used without further purification.
LRMS: m/z 223(M+1)⁺
Retention time: 5.51 min (method B)

### PREPARATION 50

### Methyl 3-(4-(5-(butylamino)-1,3,4-thiadiazol-2-yl)-3-methylphenyl)propanoate

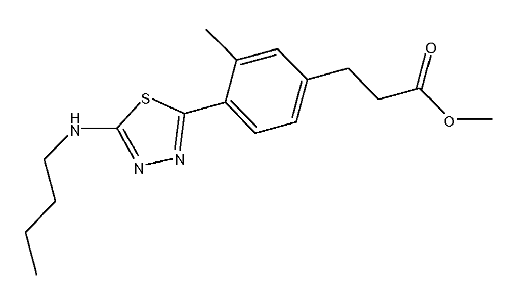

Obtained (15% yield) from the title compound of Preparation 1 and the title compound of Preparation 49 following the experimental procedure of Preparation 2. The crude was purified according to purification method A.
LRMS: m/z 334(M+1)⁺
Retention time: 3.44min (method A)

### PREPARATION 51

### Methyl 3-(4-(5-(N-butyl-2-fluorobenzamido)-1,3,4-thiadiazol-2-yl)-3-methylphenyl)-propanoate

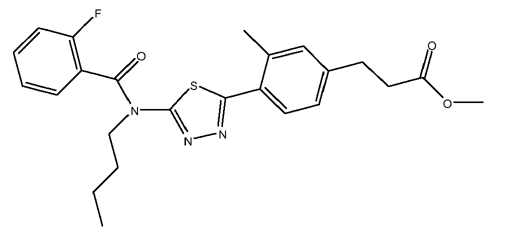

Obtained (60%) from 2-fluorobenzoyl chloride and the title compound of Preparation 50 following the experimental procedure of Preparation 11.
LRMS: m/z 456(M+1)⁺
Retention time: 7.38min (method B)

### PREPARATION 52

### 5-(4-methoxy-3,5-dimethylphenyl)-N-phenethyl-1,3,4-thiadiazol-2-amine

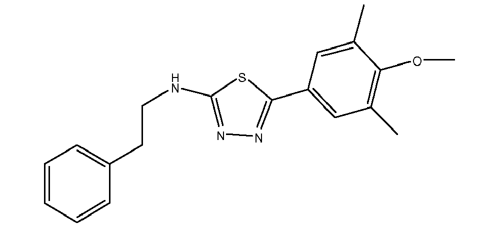

Obtained (97%) from 4-methoxy-3,5-dimethylbenzoic acid and N-phenethylhydrazine-carbothioamide following the experimental procedure of Preparation 2.
LRMS: m/z 340(M+1)⁺
Retention time: 6.94min (method B)
¹H NMR (200 MHz, DMSO-*d*₆) δ ppm 2.27 (s, 6 H) 2.81 - 2.99 (m, 2 H) 3.56 (d,
*J*=1.56 Hz, 2 H) 3.68 (s, 3 H) 7.12 - 7.49 (m, 7 H) 7.98 (s, 1 H).

### PREPARATION 53

### 4-cyano-2,6-dimethylphenyl trifluoromethanesulfonate

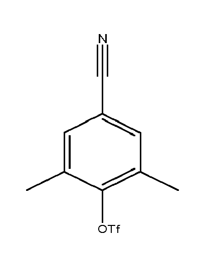

To a mixture of 4-hydroxy-3,5-dimethylbenzonitrile (7g, 47.5mmol), pyridine (70ml) and DCM (12ml) was added dropwise trifluoromethanesulfonic anhydride (14g, 49.6mmol). The reaction mixture was stirred overnight at room temperature and then concentrated to give the title compound as an oil (84% yield).
LRMS: m/z 280(M+1)⁺
Retention time: 7.04min (method B)

### PREPARATION 54

### (E)-tert-butyl 3-(4-cyano-2,6-dimethylphenyl)acrylate

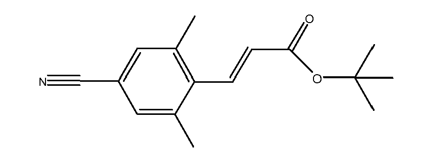

Obtained (38%) from *tert*-butyl acrylate and the title compound of Preparation 53 following the experimental procedure of Preparation 48.
LRMS: m/z 258(M+1)⁺
Retention time: 7.31 min (method B)
¹H NMR (200 MHz, CDCl₃) δ ppm 1.55 (s, 9 H) 2.35 (s, 3 H) 6.01 (d, *J*=16.40
Hz, 1 H) 7.34 (s, 2 H) 7.52 - 7.73 (m, 1 H)

### PREPARATION 55

### (E)-4-(2-carboxyvinyl)-3,5-dimethylbenzoic acid

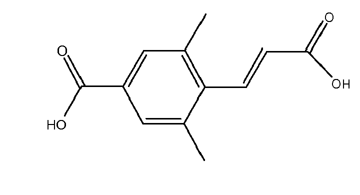

The title compound of Preparation 54 (1g,3.89mmol) was dissolved in ethanol (20ml) and NaOH 32% (20ml) was added. The reaction mixture was stirred overnight at 110°C. 5N HCl was added until a solid was formed. This solid was filtered off and dried in the vacuum oven to give a solid as the title compound (yield= 89%).
LRMS: m/z 221 (M+1)⁺
Retention time: 4.91 min (method B)

### PREPARATION 56

### 4-(2-carboxyethyl)-3,5-dimethylbenzoic acid

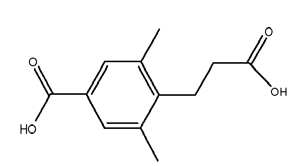

Obtained (84%) from the title compound of Preparation 55 following the experimental procedure of Preparation 49.
LRMS: m/z 223(M+1)⁺
Retention time: 4.95min (method B)

### PREPARATION 57

### 3-(4-(5-(butylamino)-1,3,4-thiadiazol-2-yl)-2,6-dimethylphenyl)propanoic acid

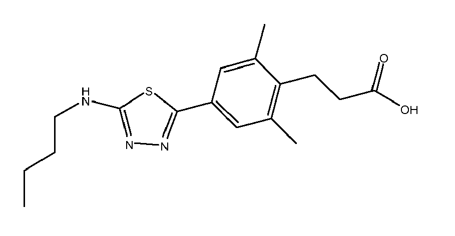

Obtained (40% yield) from the title compound of Preparation 1 and the title compound of Preparation 56 following the experimental procedure of Preparation 2.
LRMS: m/z 334(M+1)⁺
Retention time: 5.86min (method B)
¹H NMR (200 MHz, DMSO-*d*₆) δ ppm 0.83 - 0.98 (m, 3 H) 1.22 - 1.44 (m, 2 H)
1.52 (d, *J*=7.81 Hz, 2 H) 2.33 (s, 6 H) 2.97 (s, 4 H) 3.22 (d, *J*=5.47 Hz, 2 H) 7.60
(s, 2 H)

### PREPARATION 58

### tert-butyl 3-(4-(5-(butylamino)-1,3,4-thiadiazol-2-yl)-2,6-dimethylphenyl)-propanoate

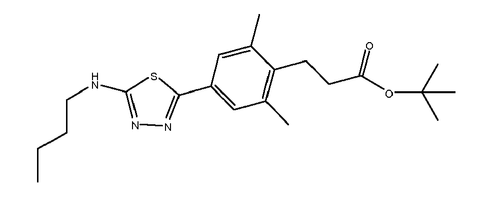

A suspension of the title compound of Preparation 57 (0.2g, 0.6mol) in toluene (10ml) was heated to 100°C and 1,1-di-tert-butoxy-N,N-dimethylmethanamine was added. The mixture was stirred at this temperature for 2h and then concentrated to dryness. The crude mixture was dissolved in ethyl acetate and washed with a solution of potassium carbonate. The organic layer was dried and concentrated to give the title compound as a solid (yield=62%).
LRMS: m/z 390(M+1)⁺
Retention time: 7.34min (method B)
¹H NMR (200 MHz, CDCl₃) δ ppm 0.96 (t, *J*=7.03 Hz, 3 H) 1.45 (d, *J*=6.25 Hz, 2
H) 1.59 (s, 9 H) 1.66 (d, *J*=7.42 Hz, 2 H) 2.37 (s, 6 H) 2.94 - 3.14 (m, 4 H) 3.31
(br. s., 2 H) 7.64 (s, 2 H)

### PREPARATION 59

### tert-butyl 3-(4-(5-(N-butyl-2-fluorobenzamido)-1,3,4-thiadiazol-2-yl)-2,6 dimethylphenyl)propanoate

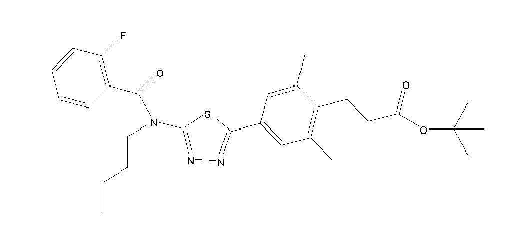

Obtained (20%) from 2-fluorobenzoyl chloride and the title compound of Preparation 58 following the experimental procedure of Preparation 11.
LRMS: m/z 512 (M+1)⁺
Retention time: 7.87min (method B)

### PREPARATION 60

### (R)-N-butyl-5-(4-(2,2-dimethyl-1,3-dioxolan-4-yloxy)phenyl)-1,3,4-thiadiazol-2-amine

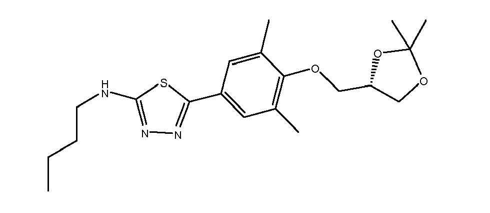

Obtained (12% yield) from the title compound of Preparation 19 (0.35 g, 1.26 mmol) and (R)-4-(cloromethyl)-2,2-dimethyl-1,3-dioxolane following the experimental procedure of Preparation 20. The crude was purified according to purification method A.
LRMS: m/z 392 (M+1)⁺
Retention time: 7.12min (method B)

### PREPARATION 61

### (R)-N-butyl-N-(5-(4-(2,2-dimethyl-1,3-dioxolan-4-yloxy)phenyl)-1,3,4-thiadiazol-2-yl)-2-fluorobenzamide

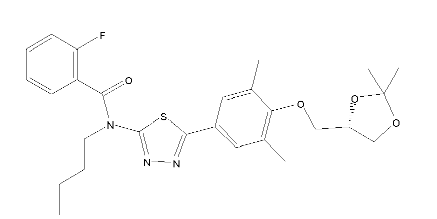

Obtained (20%) from 2-fluorobenzoyl chloride and the title compound of Preparation 60 following the experimental procedure of Preparation 11.
LRMS: m/z 514 (M+1)⁺
Retention time: 7.70min (method B)

### PREPARATION 62

### Methyl 4-(5-(N-butyl-2-fluorobenzamido)-1,3,4-thiadiazol-2-yl)benzoate

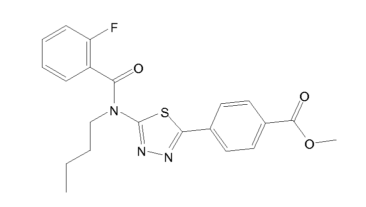

Obtained (41 %) from 2-fluorobenzoyl chloride and the title compound of Preparation 18 following the experimental procedure of Preparation 11.
LRMS: m/z 414 (M+1)⁺
Retention time: 7.37min (method B)

### PREPARATION 63

### 4-(5-(N-butyl-2-fluorobenzamido)-1,3,4-thiadiazol-2-yl)benzoic acid

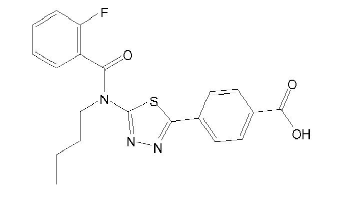

Obtained (20%) from the title compound of Preparation 62 following the experimental procedure of Preparation 43.
LRMS: m/z 400 (M+1)⁺
Retention time: 6.91min (method B)
¹H NMR (200 MHz, DMSO-D6) δ ppm 0.7 (t, *J*=7.2 Hz, 3 H) 1.2 (m, 2 H) 1.7 (m,
2 H) 4.1 (m, 2 H) 7.5 (m, 2 H) 7.7 (m, 2 H) 8.1 (m, 4 H).

### EXAMPLES

### EXAMPLE 1

### N-{5-[4-(aminosulfonyl)phenyl]-1,3,4-thiadiazol-2-yl}-N-butyl-3-methoxybenzamide

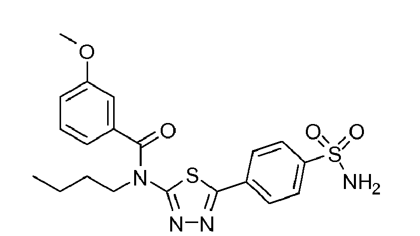

To a stirred solution of the title compound of preparation 4 (100 mg, 0.18 mmol) in dioxane (5 mL), 5 mL of HCl 4M in dioxane were added and the mixture was stirred at rt for 24 h. Solvent was removed *in vaccuo* and resulting crude was purified following the purification method A to yield 40 mg (50% yield) of the title product.
LRMS: m/z 447 (M+1)⁺
Retention time: 16.34 (method C)
1H NMR (200 MHz, DMSO-*d*₆) δ ppm 0.71 (t, *J*=7.22 Hz, 3 H) 0.99 - 1.27 (m, 2
H) 1.67 (m, 2 H) 3.80 (s, 3 H) 4.09 (m, 2 H) 7.19 (m, 4 H) 7.48 (t, *J*=7.81 Hz, 2
H) 7.96 (d, *J*=8.20 Hz, 2 H) 8.19 (d, *J*=8.20 Hz, 2 H)

### EXAMPLE 2

### N-{5-[4-(aminosulfonyl)phenyl]-1,3,4-thiadiazol-2-yl}-N-butyl-2-naphthamide

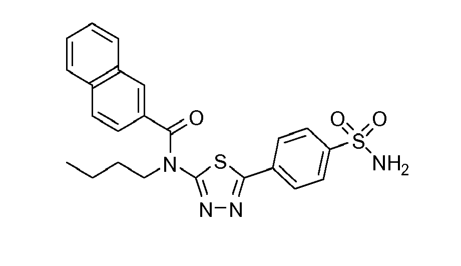

Obtained (43% yield) from the title compound of Preparation 5 following the procedure described in example 1.
LRMS: m/z 467 (M+1)⁺
Retention time: 17.90min (method C)
¹H NMR (200 MHz, DMSO-*d*₆) δ ppm 0.67 (t, *J*=7.22 Hz, 3 H) 1.14 (m, 2 H) 1.73
(m, 2 H) 4.22 (t, *J*=7.03 Hz, 2 H) 7.28 - 7.86 (m, 5 H) 7.86 - 8.53 (m, 8 H).

### EXAMPLE 3

### N-{5-[4-(aminosulfonyl)phenyl]-1,3,4-thiadiazol-2-yl}-N-butylbiphenyl-4-carboxamide

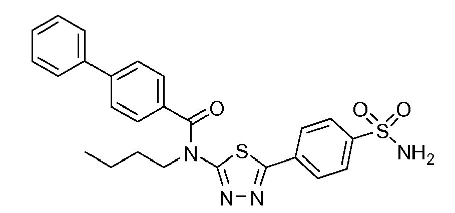

Obtained (41% yield) from the title compound of Preparation 6 following the procedure described in example 1.
LRMS: m/z 493 (M+1)⁺
Retention time: 18.78min (method C)
¹H NMR (200 MHz, DMSO-*d*₆) δ ppm 0.75 (t, *J*=7.22 Hz, 3 H) 0.95 - 1.39 (m, 2
H) 1.53 - 1.91 (m, 2 H) 4.21 (t, *J*=7.03 Hz, 2 H) 7.26 - 7.63 (m, 5 H) 7.64 - 8.09
(m, 8 H) 8.22 (d, *J*=8.20 Hz, 2 H).

### EXAMPLE 4

### N-{5-[4-(aminosulfonyl)phenyl]-1,3,4-thiadiazol-2-yl}-4-butoxy-N-butylbenzamide

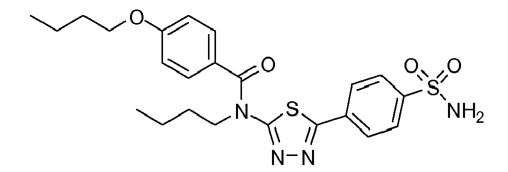

Obtained (14% yield) from the title compound of Preparation 7 following the procedure described in example 1.
LRMS: m/z 489 (M+1)⁺
Retention time: 19.24min (method C)
¹H NMR (200 MHz, DMSO-*d*₆) δ ppm 0.75 (t, *J*=7.22 Hz, 3 H) 0.95 (t, *J*=7.22
Hz, 3 H) 1.25 (m, 2H) 1.45 (m, 2 H) 1.65 (m, 4H) 4.07 (t, *J*=6.25 Hz, 2 H) 4.20 (t, *J*=6.83 Hz, 2 H) 7.10 (d, *J*=8.20 Hz, 2 H) 7.51 (s, 2 H) 7.62 (d, *J*=8.20 Hz, 2
H) 7.97 (d, *J*=8.20 Hz, 2 H) 8.19 (d, *J*=8.20 Hz, 2 H).

### EXAMPLE 5

### N-{5-[4-(aminosulfonyl)phenyl]-1,3,4-thiadiazol-2-yl}-N-butylbenzamide

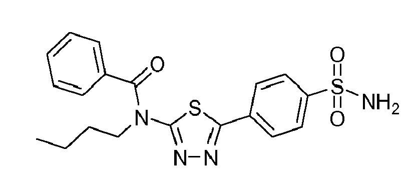

Obtained (90% yield) from the title compound of Preparation 8 following the procedure described in example 1.
LRMS: m/z 417 (M+1)⁺
Retention time: 16.02min (method C)
¹H NMR (200 MHz, DMSO-*d*₆) δ ppm 0.71 (t, *J*=7.03 Hz, 3 H) 1.16 (m, 2 H) 1.69 (m, 2 H) 4.13 (m, 2 H) 7.63 (m, 5 H) 7.98 (d, *J*=8.20 Hz, 2 H) 8.21 (d, *J*=8.20
Hz, 2 H) 8.44 (br. s., 2 H)

### EXAMPLE 6

### 3-(4-(5-(N-butyl-3-methoxybenzamido)-1,3,4-thiadiazol-2-yl)phenyl)propanoic acid

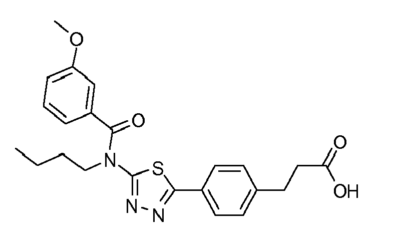

To a stirred solution of the title product of Preparation 14 (74 mg, 0.15 mmol) in dichloromethane (2 mL) at 0°C, 0.5 mL of trifluoroacetic acid were added and the mixture was stirred at rt overnight. Solvent was removed in vacuo and resulting crude was treated with diethyl ether. The solid thus obtained was filtered and washed with cold diethyl ether to yield 14 mg (21 % yield) of the title product.
LRMS: m/z 440 (M+1)⁺
Retention time: 17.76min (method C)
¹H NMR (200 MHz, DMSO-D6) δ ppm 0.7 (t, *J*=7.4 Hz, 3 H) 1.1 (m, 2 H) 1.7 (m,
2 H) 2.6 (t, *J*=7.2 Hz, 2 H) 2.9 (t, *J*=7.4 Hz, 2 H) 3.8 (s, 3 H) 4.1 (m, 2 H) 7.2 (m,
3 H) 7.5 (m, 3 H) 7.9 (d, *J*=8.2 Hz, 2 H) 12.2 (s, 1 H)

### EXAMPLE 7

### N-butyl-2-chloro-N-[5-(4-methoxy-3,5-dimethylphenyl)-1,3,4-thiadiazol-2-yl] benzamide

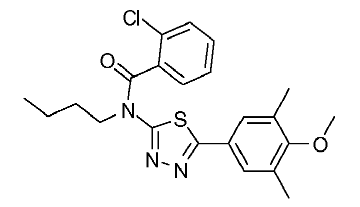

To a suspension of the title product of Preparation 9 (1g, 3.43 mmol) in dichloromethane (40 mL), DIEA (2.4 mL, 13.72 mmol) was added and the mixture was stirred for 5 min. Then a solution of 2-chlorobenzoyl chloride (0.87 mL, 6.85 mmol) in dichloromethane (5 mL) was added dropwise and the final mixture was stirred overnight. The reaction mixture was diluted with dichloromethane and washed with HCl 2M, sat Na₂CO₃ solution and brine. The organic phase was dried on Na₂SO₄, filtered and the solvent was removed *in vaccuo* to yield a solid that was purified according to purification method A. 620 mg of the title product were obtained (42% yield).
LRMS: m/z 430 (M+1)⁺
Retention time: 20.52min (method C)
¹H NMR (200 MHz, DMSO-*d*₆) ppm 0.63 (t, *J*=7.20 Hz, 3 H) 1.12 (m, 2 H)
1.70 (m, 2 H) 2.30 (s, 6 H) 3.72 (s, 3 H) 4.10 (m, 2 H) 7.60-7.90 (m, 6 H)

### EXAMPLE 8

### N-butyl-2-chloro-N-[5-(4-hydroxy-3,5-dimethylphenyl)-1,3,4-thiadiazol-2-yl]benzamide

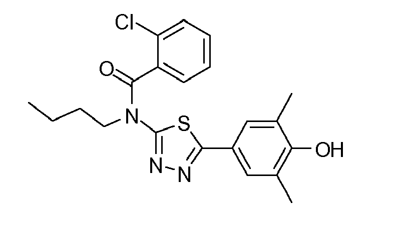

To a stirred suspension of the title product of example 7 (300 mg, 0.70 mmol) at -78°C a solution of boron tribromide in dichlorometane 1 M (1.40 mL, 1.40 mmol) was added and the mixture was stirred at that temperature for 15 min and the it was let to slowly reach rt during 2 h. Ice water was added to the reaction mixture and it was stirred at rt for 30 min. The organic layer was washed with brine, dried on Na₂SO₄ and solvent was removed *in vaccuo.* The solid thus obtained was recrystalized from diisopropyl ether. 203 mg (70% yield) of the title product were obtained.
LRMS: m/z 416 (M+1)⁺
Retention time: 18.80min (method C)
¹H NMR (200 MHz, DMSO-*d*₆) δ ppm 0.69 (t, *J*=7.22 Hz, 3 H) 1.10 (m, 2 H) 1.66
(m, 2 H) 2.25 (s, 6 H) 4.05 (m, 2H) 7.48 - 7.83 (m, 6 H) 8.94 (br. s., 1 H).

### EXAMPLE 9

### N-butyl-2-fluoro-N-[5-(4-methoxy-3,5-dimethylphenyl)-1,3,4-thiadiazol-2-yl]benzamide

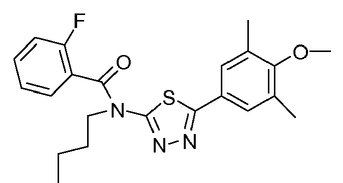

Obtained (22% yield) from 2-fluorobenzoyl chloride and the title compound of Preparation 9 following the procedure described in example 7.
LRMS: m/z 414 (M+1)⁺
Retention time: 19.94min (method C)
¹H NMR (200 MHz, CDCl₃) δ ppm 0.8 (t, *J*=7.6 Hz, 3 H) 1.2 (m, 2 H) 1.7 (m, 2 H) 2.4 (s,
6 H) 3.8 (s, 3 H) 4.1 (m, 2 H) 7.3 (m, 2 H) 7.5 (m, 2 H) 7.7 (s, 2 H).

### EXAMPLE 10

### N-Butyl-2-fluoro-N-[5-(4-hydroxy-3,5-dimethylphenyl)-1,3,4-thiadiazol-2-yl]benzamide

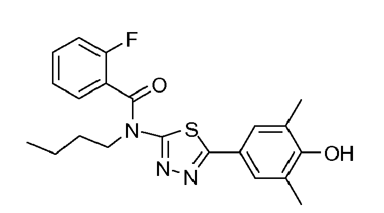

Obtained (14% yield) from the title compound of Example 9 following the procedure described in example 8.
LRMS: m/z 400 (M+1)⁺
Retention time: 18.00min (method C)
1H NMR (400 MHz, CDCl₃) δ ppm 0.77 (t, J=7.43 Hz, 3 H) 1.09 - 1.32 (m, 2 H)
1.74 (m, 2 H) 2.32 (s, 6 H) 4.14 (t, J=7.24 Hz, 2 H) 7.03 - 7.60 (m, 4 H) 7.70 (s,
2 H).

### EXAMPLE 11

### N-butyl-N-[5-(4-methoxy-3,5-dimethylphenyl)-1,3,4-thiadiazol-2-yl]benzamide

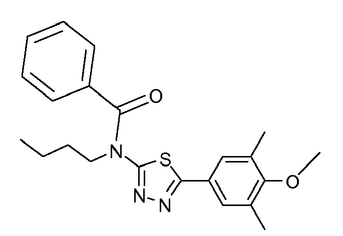

Obtained (27% yield) from benzoyl chloride and the title compound of Preparation 9 following the procedure described in example 7.
LRMS: m/z 396 (M+1)⁺
Retention time: 20.10min (method C)
¹H NMR (200 MHz, DMSO-*d*₆) δ ppm 0.72 (t, *J*=7.26 Hz, 3 H ) 1.13 (sxt, *J*=7.26
Hz, 2 H) 1.68 (quin, *J*=7.26 Hz, 2 H) 2.31 (s, 6 H) 3.78 (s, 3 H) 4.09 (t, *J*=7.42
Hz, 2 H) 7.58 - 7.93 (m, 7 H).

### EXAMPLE 12

### N-butyl-3-methoxy-N-[5-(4-methoxy-3,5-dimethylphenyl)-1,3,4-thiadiazol-2-yl]benzamide

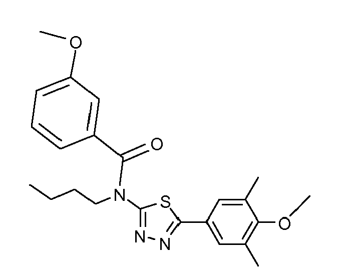

Obtained (31% yield) from 3-methoxybenzoyl chloride and the title compound of Preparation 9 following the procedure described in example 7.
LRMS: m/z 426 (M+1)⁺
Retention time: 20.15min (method C)
¹H NMR (200 MHz, DMSO-D6) δ ppm 0.7 (t, *J*=7.4 Hz, 3 H) 1.1 (m, 2 H) 1.7 (m,
2 H) 2.3 (s, 6 H) 3.7 (s, 3 H) 3.8 (s, 3 H) 4.1 (m, 2 H) 7.2 (m, 3 H) 7.5 (t, *J*=8.0
Hz, 1 H) 7.7 (s, 2 H).

### EXAMPLE 13

### N-butyl-N-[5-(4-methoxy-3,5-dimethylphenyl)-1,3,4-thiadiazol-2-yl]-1-naphthamide

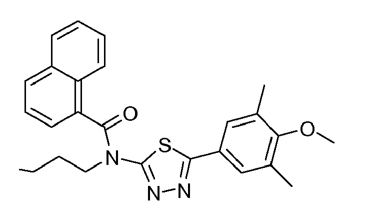

To a 0°C stirred solution of the title compound of Preparation 9 (350 mg, 1.2 mmol) in THF (4 mL), triethylamine (0.52 mL, 3.6 eq), 1-naphthoyl chloride (360 mg, 1.44 mmol) and 4-DMAP (4 mg) were added. The final mixture was stirred at rt for 1 h and then at 60°C overnight. It was let to cool down to rt and HCl 2M was added to pH 7. Solvent was removed *in vaccuo* and the final crude was purified following method A. 348 mg (63% yield) of the title product were obtained.
LRMS: m/z 446 (M+1)⁺
Retention time: 21.00min (method C)
¹H NMR (200 MHz, CDCl₃) δ ppm 0.62 (t, *J*=7.26 Hz, 3 H) 1.05 (m, 2 H) 2.37 (s,
6 H) 3.79 (s, 3 H) 7.42 - 7.84 (m, 7 H) 7.98 (m, 2 H)

### EXAMPLE 14

### N-butyl-2,6-dichloro-N-(5-(4-methoxy-3,5-dimethylphenyl)-1,3,4-thiadiazol-2-yl)benzamide

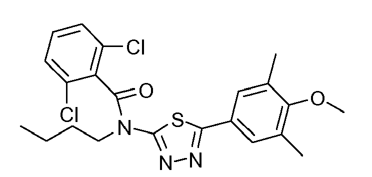

Obtained (31% yield) from 2,6-dichlorobenzoyl chloride and the title compound of Preparation 9 following the procedure described in example 13.
LRMS: m/z 464 (M+1)⁺
Retention time: 21.11min (method C)
¹H NMR (200 MHz, CDCl₃) δ ppm 0.82 (t, *J*=7.22 Hz, 3 H) 1.30 (m, 2 H) 1.81
(m, 2 H) 2.36 (s, 6 H) 3.78 (s, 3 H) 4.01 (m, 2 H) 7.41 (m, 3 H) 7.80 (s, 2 H)

### EXAMPLE 15

### N-butyl-N-[5-(4-methoxy-3,5-dimethylphenyl)-1,3,4-thiadiazol-2-yl]-2-(trifluoromethyl)benzamide

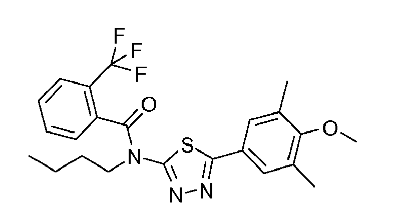

Obtained (45% yield) from 2-(trifluoromethyl)benzoyl chloride and the title compound of Preparation 9 following the procedure described in example 13.
LRMS: m/z 464 (M+1)⁺
Retention time: 20.40min (method C)
¹H NMR (200 MHz, CDCl₃) δ ppm 0.81 (t, *J*=7.22 Hz, 3 H) 1.24 (m, 2 H) 1.65 -
1.95 (m, 2 H) 2.34 (s, 6 H) 3.76 (s, 3 H) 4.13 (m, 2 H) 7.51 - 7.96 (m, 6 H)

### EXAMPLE 16

### N-butyl-N-[5-(4-methoxy-3,5-dimethylphenyl)-1,3,4-thiadiazol-2-yl]-2-phenylacetamide

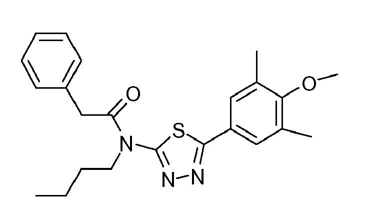

A microwave reactor containing a mixture of the title compound of preparation 9 (200mg, 0.69mmol), 2-penylacetyl chloride (118mg, 0.76mmol), triethylamine (290 µl, 2.08mol) and DMAP (10mg, 0.08mmol) in THF (2ml) mixture was heated at a Biotage Initiator device at 90°C and medium absorvance for 15 min in the microwave. Then 2N HCl was added and the solvent removed under reduced pressure. The crude obtained was purified according to purification method A to yield the desired product as a yellow solid (yield=29%)
LRMS: m/z 410 (M+1)⁺
Retention time: 20.14min (method C)
¹H NMR (200 MHz, CDCl₃) δ ppm 0.98 (t, *J*=7.22 Hz, 3 H) 1.45 (m, 2 H) 1.80 (m, 2 H) 2.30 (s, 6 H) 3.76 (s, 3 H) 4.05 (s, 2 H) 4.28 (m, 2 H) 7.30 (m, 5 H) 7.60
(s, 2 H).

### EXAMPLE 17

### N-butyl-N-[5-(4-methoxy-3,5-dimethylphenyl)-1,3,4-thiadiazol-2-yl]-2-naphthamide

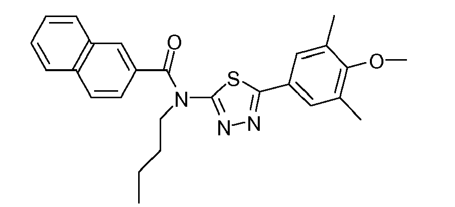

Obtained (3% yield) from 2-naphthoyl chloride and the title compound of Preparation 9 following the procedure described in example 16.
LRMS: m/z 446 (M+1)⁺
Retention time: 21.10min (method C)
1 H NMR (200 MHz, CDCl₃) δ ppm 0.8 (t, *J*=7.2 Hz, 3 H) 1.2 (m, 2 H) 1.8 (m, 2
H) 2.4 (s, 6 H) 3.8 (s, 3 H) 4.3 (m, 2 H) 7.6 (m, 3 H) 7.7 (s, 2 H) 8.0 (m, 4 H).

### EXAMPLE 18

### N-butyl-N-[5-(4-methoxy-3,5-dimethylphenyl)-1,3,4-thiadiazol-2-yl]-2,3-dihydro-1,4-benzodioxine-6-carboxamide

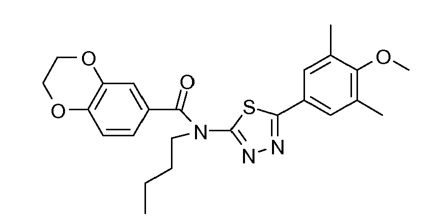

In a microwave oven vessel the title compound of Preparation 9 (250 mg, 0.86 mmol), 2,3-dihydrobenzo[b][1,4]dioxine-6-carboxylic acid (170 mg, 0.94 mmol), HBTU (325 mg, 0.86 mmol) and diisopropylethylamine (0.300 ml, 1.71 mmol) were placed and DMF (3 ml) was added. The mixture was heated at a Biotage Initiator device at 150°C and high absorvance for 15 min. Then HCl 2M was added and the solvent removed *in vaccuo.* The reaction crude was purified according to purification method A to yield 108 mg (27%) of the title compound.
LRMS: m/z 454 (M+1)⁺
Retention time: 19.90min (method C)
¹H NMR (200 MHz, CDCl₃) δ ppm 0.80 (m, *J*=7.42 Hz, 3 H) 1.28 (m, 2 H) 1.79 (m, 2 H) 2.35 (s, 6 H) 3.77 (s, 3 H) 4.32 (m, 6 H) 6.88 - 7.17 (m, 2 H) 7.23 (s, 2
H) 7.66 (s, 1 H).

### EXAMPLE 19

### N-butyl-2-methoxy-N-[5-(4-methoxy-3,5-dimethylphenyl)-1,3,4-thiadiazol-2-yl]benzamide

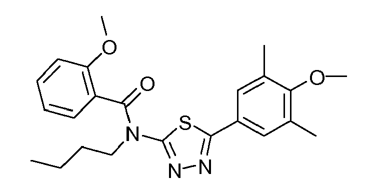

Obtained (27% yield) from 2-methoxybenzoyl chloride and the title compound of Preparation 9 following the procedure described in example 16.
LRMS: m/z 426 (M+1)⁺
Retention time: 19.60min (method C)
¹H NMR (200 MHz, CDCl₃) δ ppm 0.8 (m, 3 H) 1.2 (m, 2 H) 1.7 (m, 2 H) 2.3 (m,
6 H) 3.8 (s, 3 H) 3.9 (s, 3 H) 4.2 (m, *J*=14.3, 6.4 Hz, 2 H) 7.0 (d, *J*=8.6 Hz, 1 H)
7.1 (d, *J*=7.8 Hz, 1 H) 7.3 (m, 1 H) 7.5 (m, 1 H) 7.7 (s, 2 H).

### EXAMPLE 20

### N-Butyl-3-fluoro-N-[5-(4-methoxy-3,5-dimethylphenyl)-1,3,4-thiadiazol-2-yl]benzamide

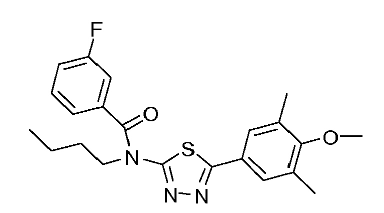

Obtained (36% yield) from the title compound of Preparation 9 and 3-fluorobenzoylchloride following the procedure described in example 16. Pyridine was used as solvent.
LRMS: m/z 414 (M+1)⁺
Retention time: 19.80min (method C)
1 H NMR (400 MHz, CDCl₃) δ ppm 0.82 (t, *J*=7.24 Hz, 3 H) 1.24 (m, 2 H) 1.79 (m, 2 H) 2.36 (s, 6 H) 3.77 (s, 3 H) 4.19 (m, 2 H) 7.38 (m, 3 H) 7.49 (m, 1 H)
7.66 (s, 2 H).

### EXAMPLE 21

### N-Butyl-N-[5-(4-methoxy-3,5-dimethylphenyl)-1,3,4-thiadiazol-2-yl]nicotinamide

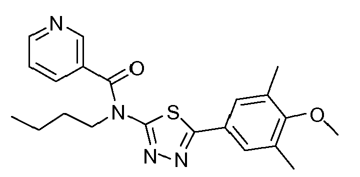

Obtained (34% yield) from the title compound of Preparation 9 and nicotinoyl chloride following the procedure described in example 16. Pyridine was used as solvent.
LRMS: m/z 397 (M+1)⁺
Retention time: 17.60min (method C)
¹H NMR (200 MHz, CDCl₃) δ ppm 0.8 (t, *J*=7.4 Hz, 3 H) 1.3 (m, 2 H) 1.8 (m, 2
H) 2.4 (s, 6 H) 3.8 (s, 3 H) 4.2 (m, 2 H) 7.5 (dd, *J*=7.8, 5.9 Hz, 1 H) 7.7 (s, 2 H)
7.9 (m, 1 H) 8.8 (m, 2 H).

### EXAMPLE 22

### N-Butyl-N-[5-(4-methoxy-3,5-dimethylphenyl)-1,3,4-thiadiazol-2-yl]pyridine-2-carboxamide

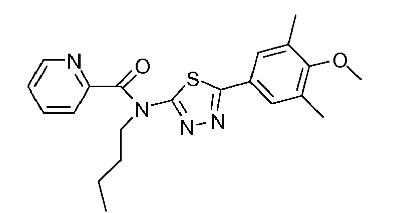

Obtained (21% yield) from the title compound of Preparation 9 and picolinoyl chloride following the procedure described in example 16. Pyridine was used as solvent.
LRMS: m/z 397 (M+1)⁺
Retention time: 19.00min (method C)
¹H NMR (200 MHz, CDCl₃) δ ppm 0.83 (t, J=7.22 Hz, 13 H) 1.26 (m, 2 H) 1.87 (m, 2 H) 2.36 (s, 6 H) 3.77 (s, 3 H) 4.41 (m, 2 H) 7.49 (d, J=5.47 Hz, 1 H) 7.67
(s, 2 H) 7.74 - 8.00 (m, 2 H) 8.69 (d, J=4.69 Hz, 1 H)

### EXAMPLE 23

### N-Butyl-6-fluoro-N-[5-(4-methoxy-3,5-dimethylphenyl)-1,3,4-thiadiazol-2-yl]pyridine-2-carboxamide

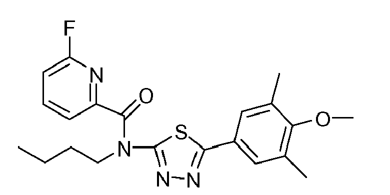

Obtained (5% yield) from the title compound of Preparation 9 and 6-fluoropicolinic acid following the procedure described in example 18.
LRMS: m/z 415 (M+1)⁺
Retention time: 19.70min (method C)
¹H NMR (200 MHz, CDCl₃) δ ppm 0.87 (t, J=7.22 Hz, 3 H) 1.31 (m, 2 H) 1.93 (m, 2 H) 2.35 (s, 6 H) 3.77 (s, 3 H) 4.37 (m, 2 H) 7.00 - 7.40 (m, 2 H) 7.45 - 8.25
(m, 3 H)

### EXAMPLE 24

### N-butyl-N-[5-(4-methoxy-3,5-dimethylphenyl)-1,3,4-thiadiazol-2-yl]-2-methylbenzamide

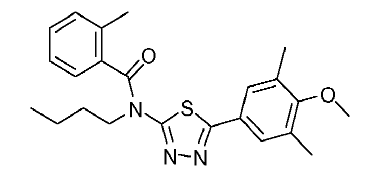

Obtained (30% yield) from the title compound of Preparation 9 and 2-methylbenzoyl chloride following the procedure described in example 16. Pyridine was used as solvent.
LRMS: m/z 410 (M+1)⁺
Retention time: 20.50min (method C)
1H NMR (200 MHz, CDCl₃) δ ppm 0.8 (t, *J*=7.2 Hz, 3 H) 1.2 (m, 2 H) 1.7 (m, 2
H) 2.3 (s, 6 H) 2.4 (s, 3H) 3.8 (s, 3 H) 4.1 (s, 2 H) 7.3 (m, 4 H) 7.7 (s, 2 H).

### EXAMPLE 25

### 2-chloro-N-[5-(4-methoxy-3,5-dimethylphenyl)-1,3,4-thiadiazol-2-yl]-Nmethylbenzamide

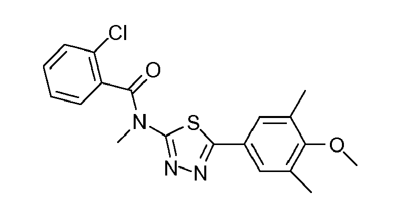

Obtained (60% yield) from the title compound of Preparation 15 and 2-chlorobenzoylchloride following the procedure described in example 13.
LRMS: m/z 388 (M+1)⁺
Retention time: 18.60min (method C)
¹H NMR (200 MHz, CDCl₃) δ ppm 2.36 (s, 6 H) 3.52 (s, 3H) 3.91 (s, 3 H) 7.34 -
7.80 (m, 6 H)

### EXAMPLE 26

### N-butyl-2-chloro-N-[5-(4-methoxy-3-methylphenyl)-1,3,4-thiadiazol-2-yl]benzamide

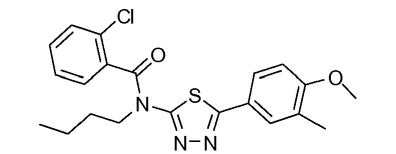

Obtained (44% yield) from the title compound of Preparation 16 and 2-chlorobenzoylchloride following the procedure described in example 13.
LRMS: m/z 416 (M+1)⁺
Retention time: 20.20min (method C)
¹H NMR (200 MHz, DMSO-*d*₆) δ ppm 0.69 (t, *J*=7.22 Hz, 3 H) 1.21 (m, 2 H) 1.66 (m, 2 H) 2.24 (s, 3 H) 3.88 (s, 3 H) 4.20 (m, 2H) 7.11 (d, *J*=8.20 Hz, 1 H) 7.35 -
8.01 (m, 6 H)

### EXAMPLE 27

### N-butyl-2-chloro-N-[5-(3-chloro-4-methoxyphenyl)-1,3,4-thiadiazol-2-yl]benzamide

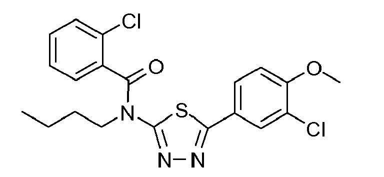

Obtained (49% yield) from the title compound of Preparation 17 and 2-chlorobenzoylchloride following the procedure described in example 13.
LRMS: m/z 437 (M+1)⁺
Retention time: 20.10min (method C)
¹H NMR (200 MHz, DMSO-*d*₆) δ ppm 0.69 (t, *J*=7.42 Hz, 3 H) 1.14 (m, 2 H) 1.65 (m, 2 H) 3.96 (s, 3 H) 4.10 (m, 2H) 7.33 (d, *J*=8.98 Hz, 1 H) 7.46 - 7.84 (m, 4 H)
7.95 (dd, *J*=8.59, 1.95 Hz, 1 H) 8.03 (s, 1 H)

### EXAMPLE 28

### Methyl 4-{5-[butyl(2-chlorobenzoyl)amino]-1,3,4-thiadiazol-2-yl}benzoate

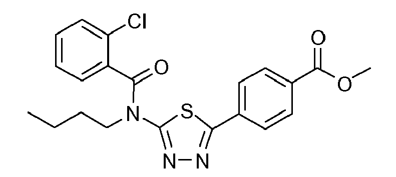

Obtained (58% yield) from the title compound of Preparation 18 and 2-chlorobenzoylchloride following the procedure described in example 13.
LRMS: m/z 430 (M+1)⁺
Retention time: 18.66min (method C)
¹H NMR (200 MHz, DMSO-*d*₆) δ ppm 0.69 (t, *J*=7.22 Hz, 3 H) 1.2 (m, 2 H) 1.6
(m, 2 H) 3.9 (s, 3 H) 4.1 (m, 2 H) 7.6 (m, 4 H) 8.2 (m, 4 H)

### EXAMPLE 29

### 4-{5-[Butyl(2-chlorobenzoyl)amino]-1,3,4-thiadiazol-2-yl}benzoic acid

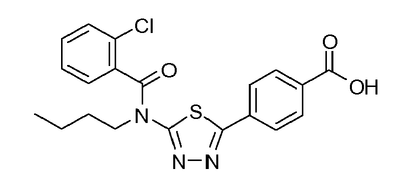

A suspension of the title compound of example 28 (200 mg, 0.47 mmol) in HCl conc (10 ml) was stirred at 50°C overnight. The reaction mixture was cooled down and filtered. The solid thus obtained was washed with water and hexane and dried. 137mg (71 % yield) of the title product were obtained as a white solid.
LRMS: m/z 416 (M+1)⁺
Retention time: 18.00min (method C)
¹H NMR (200 MHz, DMSO-*d*₆) δ ppm 0.70 (t, *J*=7.22 Hz, 3 H) 1.16 (m, 2 H) 1.68
(m, 2 H) 3.93 (m, 2 H) 7.47 - 7.86 (m, 4 H) 8.01 - 8.27 (m, 4 H)

### EXAMPLE 30

### N-Butyl-2-chloro-N-{5-[4-(2,3-dihydroxypropoxy)-3,5-dimethylphenyl]-1,3,4-thiadiazol-2-yl}benzamide

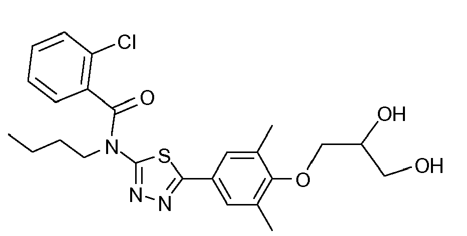

To a stirred solution of the title compound of Preparation 21 (56 mg, 0.11 mmol) in acetonitrile (2 ml), HCl 2M (0.1 ml, 0.20 mmol) was added and the mixture was stirred at 40°C for 2 h. Solvent was removed and the resulting crude product was purified according to purification method A. 25 mg (47% yield) of the title compound were obtained.
LRMS: m/z 491 (M+1)⁺
Retention time: 17.40min (method C)
¹H NMR (200 MHz, DMSO-*d*₆) δ ppm 0.69 (t, *J*=7.22 Hz, 3 H) 1.12 (m, 2 H) 1.65 (m, 2 H) 2.33 (s, 6 H) 3.45 - 3.96 (m, 5 H) 4.66 (t, *J*=5.47 Hz, 1 H) 4.98 (d,
*J*=4.69 Hz, 1 H) 7.40 - 7.94 (m, 6 H).

### EXAMPLE 31

### N-Butyl-2-chloro-N-[5-(2-chloro-6-methoxypyridin-4-yl)-1,3,4-thiadiazol-2-yl]benzamide

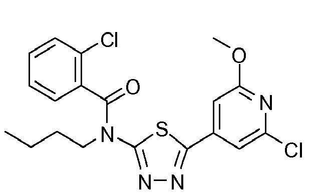

Obtained (58% yield) from the title compound of Preparation 22 and 2-chlorobenzoylchloride following the procedure described in example 13.
LRMS: m/z 438 (M+1)⁺
Retention time: 20.80min (method C)
¹H NMR (200 MHz, DMSO-*d*₆) δ ppm 0.69 (t, *J*=7.42 Hz, 3 H) 1.12 (m, 2 H) 1.67
(m, 2 H) 3.79 (m, 2 H) 4.13 (s, 3 H) 7.41 (s, 2 H) 7.48 - 7.91 (m, 4 H)

### EXAMPLE 32

### N-butyl-2-chloro-N-{5-[4-(2-methoxyethoxy)-3,5-dimethylphenyl]-1,3,4-thiadiazol-2-yl}benzamide

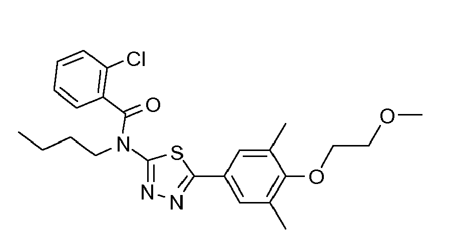

Obtained (21% yield) from the title compound of Preparation 26 and 2-chlorobenzoylchloride following the procedure described in example 13.
LRMS: m/z 475 (M+1)⁺
Retention time: 19.90min (method C)
¹H NMR (200 MHz, CDCl₃) δ ppm 0.8 (t, *J*=7.4 Hz, 3 H) 1.2 (m, 2 H) 1.8 (m, 2
H) 2.4 (s, 6 H) 3.5 (s, 3 H) 3.8 (dd, *J*=6.1, 3.3 Hz, 2 H) 4.0 (m, 2 H) 4.3 (m, 2 H)
7.5 (m, 4 H) 7.7 (s, 2 H).

### EXAMPLE 33

### 2-Chloro-N-[5-(4-methoxy-3,5-dimethylphenyl)-1,3,4-thiadiazol-2-yl]-N-(2-methoxyethyl)benzamide

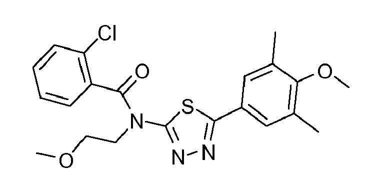

Obtained (16% yield) from the title compound of Preparation 24 and 2-chlorobenzoylchloride following the procedure described in example 16.
LRMS: m/z 432 (M+1)⁺
Retention time: 18.85min (method C)
¹H NMR (200 MHz, CDCl₃) δ ppm 2.4 (s, 6 H) 3.2 (s, 3 H) 3.6 (m, 1 H) 3.8 (s, 3
H) 3.9 (m, 1 H) 4.1 (m, 1 H) 4.5 (m, 1 H) 7.4 (m, 4 H) 7.7 (s, 2 H).

### EXAMPLE 34

### 2-Chloro-N-ethyl-N-[5-(4-methoxy-3,5-dimethylphenyl)-1,3,4-thiadiazol-2-yl]benzamide

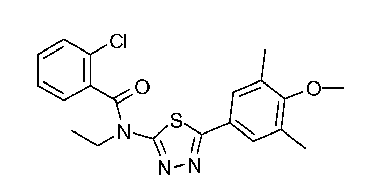

Obtained (20% yield) from the title compound of Preparation 25 and 2-chlorobenzoylchloride following the procedure described in example 16.
LRMS: m/z 402 (M+1)⁺
Retention time: 19.00min (method C)
1 H NMR (200 MHz, CDCl₃) δ ppm 1.32 (t, *J*=7.03 Hz, 3 H) 2.36 (s, 6 H) 3.78 (s,
3 H) 7.45 (m, 4 H) 7.67 (s, 2 H)

### EXAMPLE 35

### tert-Butyl (4-{5-[butyl(2-chlorobenzoyl)amino]-1,3,4-thiadiazol-2-yl}-2,6-dimethyl-phenoxy)acetate

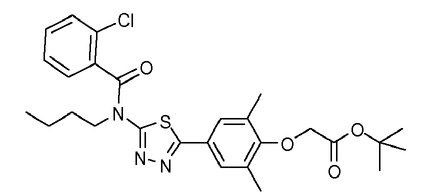

Obtained (70% yield) from the title compound of Preparation 34 and 2-chlorobenzoylchloride following the procedure described in example 13.
LRMS: m/z 530 (M+1)⁺
Retention time: 21.01min (method C)
¹H NMR (200 MHz, DMSO-D6) δ ppm 0.7 (t, *J*=7.4 Hz, 3 H) 1.2 (m, 2 H) 1.5 (s,
9 H) 1.7 (m, 2 H) 2.3 (s, 6 H) 3.8 (m, 1 H) 4.1 (m, 1 H) 4.5 (s, 2 H) 7.7 (m, 6 H).

### EXAMPLE 36

### (4-{5-[Butyl(2-chlorobenzoyl)amino]-1,3,4-thiadiazol-2-yl}-2,6-dimethylphenoxy)acetic acid

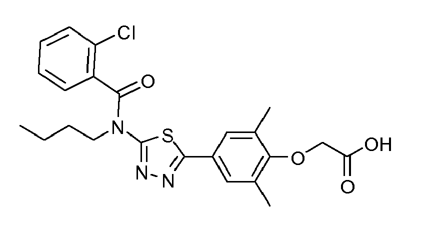

Obtained (45% yield) from the title compound of Example 35 following the procedure described in example 6.
LRMS: m/z 474 (M+1)⁺
Retention time: 18.31min (method C)
¹H NMR (200 MHz, DMSO-D6) δ ppm 0.7 (t, *J*=7.22 Hz, 3 H) 1.2 (m, 2 H) 1.7
(m, 2 H) 2.33 (s, 6 H) 3.60 (t, J=6.25 Hz, 2 H) 4.47 (s, 2 H) 7.7 (m, 6 H).

### EXAMPLE 37

### 2-Chloro-N-[5-(4-methoxy-3,5-dimethylphenyl)-1,3,4-thiadiazol-2-yl]-N-(3-methylbutyl)benzamide

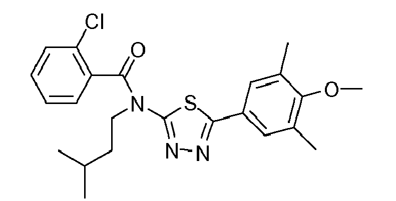

Obtained (30% yield) from the title compound of Preparation 28 and 2-chlorobenzoylchloride following the procedure described in example 16.
LRMS: m/z 444 (M+1)⁺
Retention time: 20.90min (method C)
¹H NMR (200 MHz, CDCl₃) δ ppm 0.74 (s, 6 H) 1.50 (m, 2 H) 2.36 (s, 6 H) 3.78
(s, 3 H) 4.12 (m, 2 H) 7.72 (m, 6 H)

### EXAMPLE 38

### 2-Chloro-N-[3-(diethylamino)propyl]-N-[5-(4-methoxy-3,5-dimethylphenyl)-1,3,4-thiadiazol-2-yl]benzamide

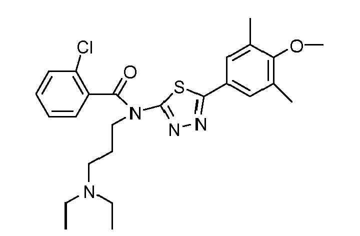

Obtained (30% yield) from the title compound of Preparation 30 and 2-chlorobenzoylchloride following the procedure described in example 16.
LRMS: m/z 488 (M+1)⁺
Retention time: 13.70min (method C)
¹H NMR (200 MHz, CDCl₃) δ ppm 0.9 (t, *J*=7.2 Hz, 6 H) 2.4 (m, 12 H) 3.8 (s, 3
H) 3.9 (m, 1 H) 4.3 (m, 1 H) 7.5 (m, 4 H) 7.7 (s, 2 H).

### EXAMPLE 39

### N-Butyl-2-chloro-N-[5-(2-chloro-6-methylpyridin-4-yl)-1,3,4-thiadiazol-2-yl]benzamide

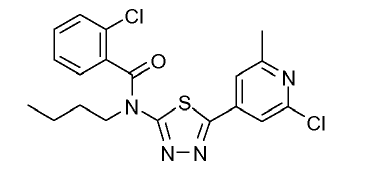

Obtained (25% yield) from the title compound of Preparation 31 and 2-chlorobenzoylchloride following the procedure described in example 13.
LRMS: m/z 422 (M+1)⁺
Retention time: 19.80min (method C)
¹H NMR (200 MHz, DMSO-d₆) δ ppm 0.69 (t, J=7.22 Hz, 3 H) 1.15 (m, 2 H) 1.65
(m, 2 H) 2.51 (s, 3 H) 3.95 - 4.24 (m, 2 H) 7.42 - 7.80 (m, 4 H) 7.95 (s, 2 H)

### EXAMPLE 40

### N-Butyl-2-chloro-N-[5-(6-chloro-2-oxo-1,2-dihydropyridin-4-yl)-1,3,4-thiadiazol-2-yl]benzamide

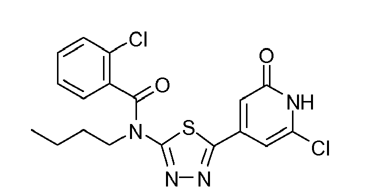

To a stirred solution of the title compound of Example 31 (100 mg, 0.23 mmol) in acetonitrile (5 ml) under argon, sodium iodide (172 mg, 1.15 mmol) and trimethylsilyl chloride (145 µl, 1.15 mmol) were added. The mixture was stirred at R.T. for 1h h and then water (50 ml) was added. The aqueous solution was extracted with dichloromethane, then basified and extracted with dichloromethane again. The combined organic layers were dried and solvent was removed. The crude product thus obtained was purified according to purification method A to yield 82 mg (85%) of the title compound.
LRMS: m/z 424 (M+1)⁺
Retention time: 18.40min (method C)
¹H NMR (200 MHz, DMSO-d₆) δ ppm 0.69 (t, *J*=7.26 Hz, 3 H) 1.14 (sxt, *J*=7.26
Hz, 2 H) 1.67 (m, 2 H) 3.85 - 4.18 (m, 2 H) 7.18 (s, 2 H) 7.45-7.88 (m, 4 H).

### EXAMPLE 41

### N-Butyl-N-[5-(2-chloro-6-methoxypyridin-4-yl)-1,3,4-thiadiazol-2-yl]-2-fluorobenzamide

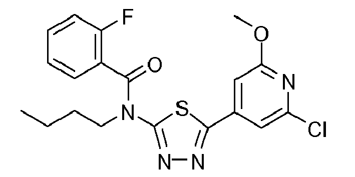

Obtained (68% yield) from the title compound of Preparation 22 and 2-fluorobenzoylchloride following the procedure described in example 13.
LRMS: m/z 421 (M+1)⁺
Retention time: 20.40min (method C)
¹H NMR (200 MHz, CDCl₃) δ ppm 0.87 (t, J=7.22 Hz, 3 H) 1.31 (m, 2 H) 1.93 (m, 2 H) 3.77 (s, 3 H) 4.37 (m, 2 H) 7.42 (m, 3 H) 7.75 (m, 3 H).

### EXAMPLE 42

### N-Butyl-N-[5-(2-chloro-6-methylpyridin-4-yl)-1,3,4-thiadiazol-2-yl]-2-fluorobenzamide

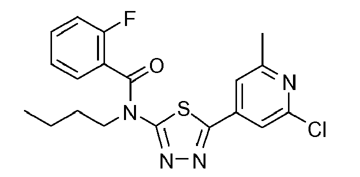

Obtained (40% yield) from the title compound of Preparation 31 and 2-fluorobenzoylchloride following the procedure described in example 13.
LRMS: m/z 405 (M+1)⁺
Retention time: 19.20min (method C)
¹H NMR (200 MHz, DMSO-d₆) δ ppm 0.70 (t, J=7.22 Hz, 3 H) 1.10 (m, 2 H) 1.65 (m, 2 H) 2.53 (s, 3 H) 4.09 (t, J=7.22 Hz, 2 H) 7.42 (m, 2H), 7.68 (m, 2H) 7.92
(s, 2 H)

### EXAMPLE 43

### 2-fluoro-N-[5-(4-methoxy-3,5-dimethylphenyl)-1,3,4-thiadiazol-2-yl]-N-propylbenzamide

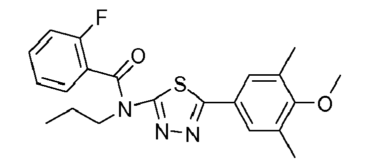

Obtained (40% yield) from the title compound of Preparation 33 and 2-fluorobenzoylchloride following the procedure described in example 16.
LRMS: m/z 400 (M+1)⁺
Retention time: 19.30min (method C)
¹H NMR (200 MHz, CDCl₃) δ ppm 0.80 (t, J=7.42 Hz, 3 H) 1.78 (m, 2H) 2.36 (s,
6 H) 3.78 (s, 3H) 4.08 (m, 2 H) 7.23 (m, 2H) 7.45 (m, 2 H) 7.66 (s, 2 H).

### EXAMPLE 44

### N-Butyl-2-fluoro-N-[5-(2-methylpyridin-4-yl)-1,3,4-thiadiazol-2-yl]benzamide

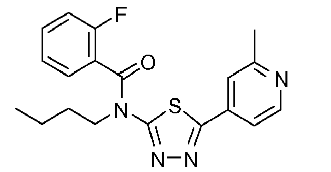

To a stirred solution of title compound of Example 42 (60 mg, 0.15 mmol) in ethanol (10 ml), 10% Pd/C (12 mg) and HCl in dioxane (0.1 ml) were added and the mixture was stirred under hydrogen (30 psi) for 4 days. The reaction mixture was filtered and solvent removed in vaccuo to yield a crude product that was purified according to purification method A to yield 36 mg (65%) of the title compound.
LRMS: m/z 371 (M+1)⁺
Retention time: 15.90min (method C)
¹H NMR (200 MHz, DMSO-d₆) δ ppm 0.70 (t, J=7.42 Hz, 3 H) 1.05 (m, 2H) 1.64 (m, 2 H) 2.54 (s, 3 H) 4.09 (t, J=7.42 Hz, 2 H) 7.40 (m, 2H) 7.64 (m, 2 H) 7.82
(s, 2H) 8.63 (d, *J*=5.08 Hz, 1 H)

### EXAMPLE 45

### N-butyl-2-fluoro-N-[5-(2-methoxypyridin-4-yl)-1,3,4-thiadiazol-2-yl]benzamide

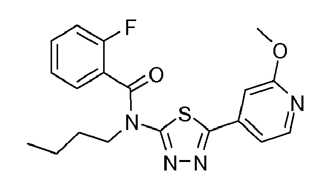

Obtained (16% yield) from the title compound of Preparation 36 and 2-fluorobenzoylchloride following the procedure described in example 13.
LRMS: m/z 387 (M+1)⁺
Retention time: 18.70min (method C)
¹H NMR (200 MHz, DMSO-d₆) δ ppm 0.70 (t, J=7.22 Hz, 3 H) 1.16 (m, 2 H) 1.66 (m, 2 H) 3.85 (s, 3 H) 4.09 (m, 2 H) 7.37 - 7.80 (m, 6 H) 8.35 (d, *J*=5.08 Hz, 1 H).

### EXAMPLE 46

### N-(cyclopropylmethyl)-2-fluoro-N-[5-(4-methoxy-3,5-dimethylphenyl)-1,3,4-thiadiazol-2-yl]benzamide

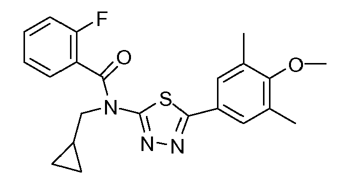

Obtained (10% yield) from the title compound of Preparation 38 and 2-fluorobenzoylchloride following the procedure described in example 16.
LRMS: m/z 419 (M+1)⁺
Retention time: 19.44min (method C)
¹H NMR (200 MHz, CDCl₃) δ ppm 0.1 (q, *J*=5.3 Hz, 2 H) 0.4 (m, 2 H) 1.3 (m, 1
H) 2.4 (s, 6 H) 3.8 (s, 3 H) 4.1 (d, *J*=7.0 Hz, 2 H) 7.3 (m, 2 H) 7.5 (m, 2 H) 7.7 (s, 2 H).

### EXAMPLE 47

### 3-(4-{5-[butyl(2-fluorobenzoyl)amino]-1,3,4-thiadiazol-2-yl}-2,6-dimethylphenyl) propanoic acid

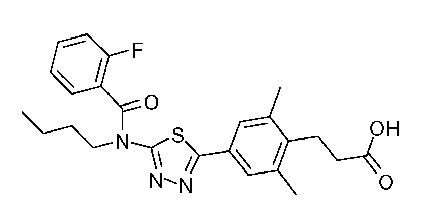

Obtained (6% yield) from the title compound of Preparation 59 following the procedure described in example 1.
LRMS: m/z 456 (M+1)⁺
Retention time: 18.01min (method C)

### EXAMPLE 48

### N-butyl-2-fluoro-N-(5-(6-methoxypyridin-3-yl)-1,3,4-thiadiazol-2-yl)benzamide

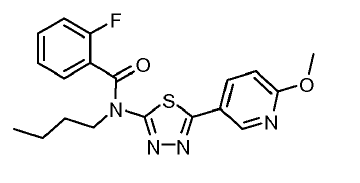

Obtained (20% yield) from the title compound of Preparation 40 and 2-fluorobenzoylchloride following the procedure described in example 13.
LRMS: m/z 387 (M+1)⁺
Retention time: 18.26min (method C)
¹H NMR (200 MHz, CDCl₃) δ ppm 0.77 (t, *J*=8 Hz, 3 H) 1.23 (m, 2 H) 1.74 (m, 2
H) 4.01 (s, 3 H) 4.16 (m, 2 H) 6.87 (d, *J*=10 Hz, 1 H) 7.35 (m, 2 H) 7.52 (m, 2
H) 8.23 (m, 1 H) 8.73 (s, 1 H).

### EXAMPLE 49

### N-butyl-2-fluoro-N-(5-(imidazo[1,2-a]pyridin-6-yl)-1,3,4-thiadiazol-2-yl)benzamide

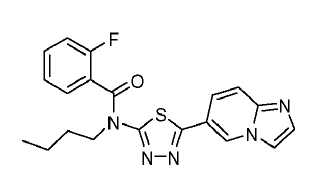

Obtained (36% yield) from the title compound of Preparation 41 and 2-fluorobenzoylchloride following the procedure described in example 13.
LRMS: m/z 396 (M+1)⁺
Retention time: 11.96min (method C)
¹H NMR (200 MHz, DMSO-d₆) δ ppm 0.71 (t, *J*=8 Hz, 3 H) 1.17 (m, 2 H) 1.67 (m, 2 H) 4.08 (t, *J*=8 Hz, 2 H) 7.46 (m, 2 H) 7.75 (m, 4 H) 8.07 (s, 1 H) 8.36 (s,
1 H) 9.41 (s, 1 H).

### EXAMPLE 50

### N-butyl-2-fluoro-N-(5-(imidazo[1,2-a]pyridin-7-yl)-1,3,4-thiadiazol-2-yl)benzamide

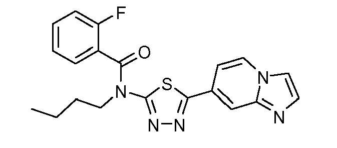

Obtained (34% yield) from the title compound of Preparation 44 and 2-fluorobenzoylchloride following the procedure described in example 13.
LRMS: m/z 396 (M+1)⁺
Retention time: 11.91min (method C)
¹H NMR (200 MHz, DMSO-d₆) δ ppm 0.71 (t, *J*=8 Hz, 3 H) 1.17 (m, 2 H) 1.67(m,
2 H) 4.08(t, *J*=6 Hz, 2 H) 7.46 (m, 2 H) 7.57 (m, 2 H) 7.74 (m, 2 H) 8.15 (d, *J*=16
Hz, 2 H) 8.70 (d, *J*=6 Hz, 1 H).

### EXAMPLE 51

### 3-(4-(5-(N-butyl-2-chlorobenzamido)-1,3,4-thiadiazol-2-yl)benzamido)propanoic acid

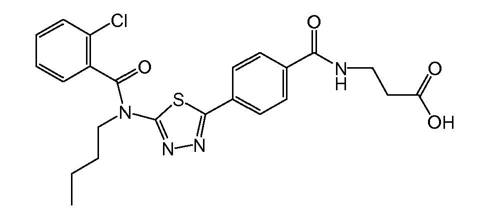

A mixture of the title compound of Example 29 (50mg, 0.12mmol), EDC (25mg, 0.13mmol) and HOBt (18mg, 0.13mmol) in THF (2ml) was stirred at room temperature for 2h. Then ethyl 3-aminopropanoate (16mg, 0.14 mmol) was added and the reaction mixture stirred at rt overnight. Solvent was removed and the crude redissolved in ethyl acetate. The organic layer was washed with water, dried and solvent was removed. TFA (1ml) and DCM (1ml) were added and the final mixture was stirred at rt overnight.

Solvent was removed *in vaccuo* and the oil obtained purified according to purification method A. 15mg of the title compound were obtained (yield=49%) as a white solid.
LRMS: m/z 487 (M+1)⁺
Retention time: 16.67min (method C)
¹H NMR (200 MHz, CDCl₃) δ ppm 0.76 (t, *J*=7.03 Hz, 3 H) 1.20 (m, 2 H) 1.48 -
2.01 (m, 2 H) 2.57 - 2.89 (m, 2 H) 3.54 - 3.96 (m, 2 H) 4.27 (d, J=8.20 Hz, 2 H)
7.47 (m, 4 H) 7.76 - 8.23 (m, 4 H).

### EXAMPLE 52

### Ethyl 3-(4-(5-(N-butyl-2-chlorobenzamido)-1,3,4-thiadiazol-2-yl)benzamido) propanoate

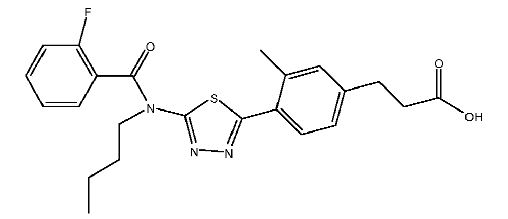

Obtained (2% yield) from the title compound of Preparation 51 following the procedure described in example 29.
LRMS: m/z 442 (M+1)⁺
Retention time: 18.14min (method C)
¹H NMR (400 MHz, CHLOROFORM-*d*) δ ppm 0.77 (s, 3 H) 1.10 - 1.33 (m, 2 H)
1.64 - 1.83 (m, 2 H) 2.70 - 2.80 (m, 2 H) 2.93 - 3.08 (m, 2 H) 4.09 - 4.22 (m, 2
H) 7.13 - 7.24 (m, 2 H) 7.24 - 7.25 (m, 1 H) 7.29 - 7.34 (m, 1 H) 7.43 - 7.49 (m,
1 H) 7.50 - 7.58 (m, 1 H) 7.64 - 7.68 (m, 1 H).

### EXAMPLE 53

### N-butyl-N-(5-(4-carbamoylphenyl)-1,3,4-thiadiazol-2-yl)-2-chlorobenzamide

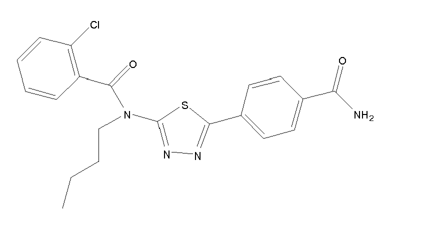

Obtained (23% yield) from the title compound of Example 29 and ammonia following the procedure described in example 51.
LRMS: m/z 415 (M+1)⁺
Retention time: 16.51min (method C)
¹H NMR (200 MHz, DMSO-*d*₆) δ ppm 0.70 (t, *J*=7.03 Hz, 3 H) 1.01 - 1.25 (m, 2
H) 1.65 (d, *J*=8.59 Hz, 2 H) 3.86 (br. s., 2 H) 7.43 - 7.84 (m, 4 H) 7.93 - 8.22 (m,
4 H) 8.39 (s, 2 H).

### EXAMPLE 54

### 1-(4-(5-(N-butyl-2-fluorobenzamido)-1,3,4-thiadiazol-2-yl)benzyl)azetidine-3-carboxylic acid

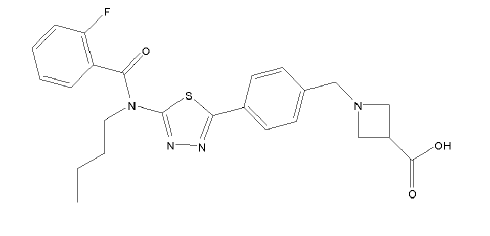

Obtained (23% yield) from the title compound of Preparation 47 and azetidine-3-carboxylic acid following the procedure described in Preparation 10.
LRMS: m/z 469 (M+1)⁺
Retention time: 13.02min (method C)
¹H NMR (200 MHz, DMSO-*d*₆) δ ppm 0.72 (t, 3 H) 1.16 (m, 2 H) 1.66 (m, 2 H)
3.24 (m., 1 H) 3.34 - 3.53 (m, 2 H) 3.64 (s, 6 H) 4.06 (t, 2 H) 7.46 (m, 4 H) 7.61 -
7.86 (m, 2 H) 7.95 (m, 2 H).

### EXAMPLE 55

### (R)-N-butyl-N-(5-(4-(2,3-dihydroxypropoxy)-3,5-dimethylphenyl)-1,3,4-thiadiazol-2-yl)-2-fluorobenzamide

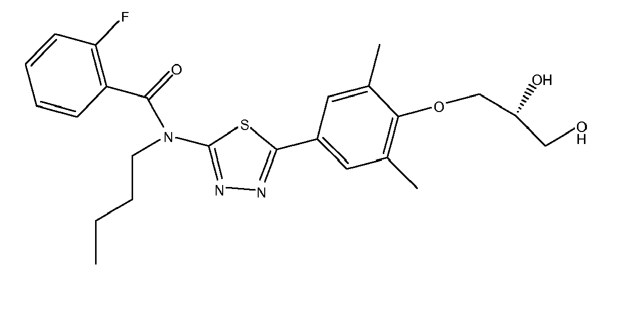

Obtained (16% yield) from the title compound of Preparation 61 following the procedure described in Example 30.
LRMS: m/z 475 (M+1)⁺
Retention time: 16.96min (method C)
¹H NMR (200 MHz, CDCl₃) δ ppm 0.73 (t, *J*=7.22 Hz, 3 H) 1.18 (m, 2 H) 2.33 (s,
6 H) 3.86 (m, 4 H) 4.66 (m, 3 H) 7.40 - 7.84 (m, 6 H).

### EXAMPLE 56

### 2-fluoro-N-(5-(4-methoxy-3,5-dimethylphenyl)-1,3,4-thiadiazol-2-yl)-N-phenethylbenzamide

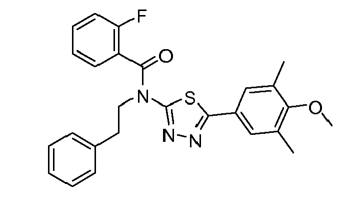

Obtained (27% yield) from the title compound of Preparation 52 and 2-fluorobenzoylchloride following the procedure described in example 13.
LRMS: m/z 462 (M+1)⁺
Retention time: 20.26min (method C)
¹H NMR (200 MHz, CDCl₃) δ ppm 2.4 (s, 6 H) 3.1 (t, *J*=7.0 Hz, 2 H) 3.8 (s, 3 H)
4.4 (m, 2 H) 7.0 (m, 3 H) 7.2 (m, 5 H) 7.5 (m, 1 H) 7.7 (s, 2 H).

### EXAMPLE 57

### 2-(4-(5-(N-butyl-2-fluorobenzamido)-1,3,4-thiadiazol-2-yl)benzamido)acetic acid

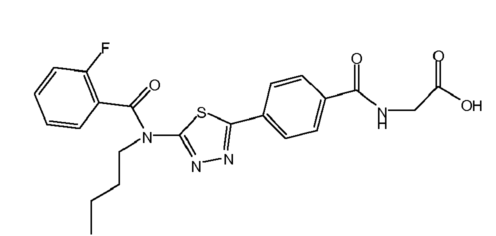

Obtained (5% yield) from the title compound of Preparation 63 and *tert*-butyl 2-aminoacetate following the procedure described in example 51
LRMS: m/z 457 (M+1)⁺
Retention time: 15.85min (method C)
¹H NMR (200 MHz, CDCl₃) δ ppm 0.6 (t, *J*=7.0 Hz, 3 H) 1.1 (m, 2 H) 1.6 (m, 2
H) 4.0 (m, 4 H) 7.2 (m, 2 H) 7.4 (m, 2 H) 7.8 (m, 4 H) 8.2 (s, 1 H).

### PHARMACOLOGICAL ACTIVITY

### 35S-GTP-g binding assay:

The effect of the compounds was measured using a 35S-GTPyS binding assay. Briefly, membranes were incubated in a buffer containing 20 mM HEPES pH 7.4, 100 mM NaCl, 10 mM MgCl2, 10µM GDP, 50µg/ml saponin and 0.2% fatty acid-free BSA at various concentrations (0.1nM-10µM) and 0.1 nM 35S-GTPyS. 10µM S1P was used as 100% maximum efficacy. The assay was incubated for 90 min at room temperature with gentle mixing, and terminated by filtrating the reaction mixture through GF/C filter plates using the Manifold Filtration System. The filters were immediately washed with sodium phosphate pH 7.4 buffer. After drying the filter plates scintillant liquid were added to each well and 35S-GTPyS binding was measured on a Trilux Scintillation Counter.

The results are shown in Table 1.

**Table 1**

| **EXAMPLES** | **EC₅₀ (nM)** |
|---|---|
| 8 | 6.2 |
| 9 | 4.4 |
| 19 | 8.3 |
| 31 | 3.4 |
| 32 | 64.2 |
| 39 | 3.8 |
| 46 | 36.0 |
| 48 | 115.5 |
| 49 | 88.7 |
| 53 | 46.3 |

The 2-amidothiadiazole derivatives of the invention may also be combined with other active compounds in the treatment of diseases known to be susceptible to improvement by treatment with a sphingosine-1-phosphate receptor agonist (S1P1).

The combinations of the invention can optionally comprise one or more additional active substances which are known to be useful in the treatment of autoimmune diseases, chronic immune and inflammatory diseases, transplant rejection, malignant neoplastic diseases, angiogenic-related disorders, pain, neurological diseases,viral and infectious diseases, such as (a) beta interferons such as Betaseron, Avonex or Rebif, (b), immunomodulators such as glatiramer acetate, (c) inhibitors of DNA synthesis and repair, such as Mitoxantrone, (d) anti-alpha 4 integrin antibodies, such as Natalizumab (Tysabri), (e) alpha 4 integrin antagonists such as R-1295 , TBC-4746, CDP-323, ELND-002, Firategrast and TMC-2003, (f), dyhydrofolate reductase inhibitors, such as Methotrexate or CH-1504, (g) glucocorticoids such as prednisone or methylprednisolone, (h), DHODH inhibitors such as Teriflunomide, (i) fumaric acid esters, such as *BG-12*, (j) immunomodulators such as Laquinimod, (k) anti-CD20 monoclonal antibodies such as Rituximab, Ocrelizumab Ofatumumab or TRU-015, (l) anti-CD52 such as alemtuzumab, (m) anti-CD25 such as daclizumab, (n) anti-CD88, such as eculizumab or pexilizumab, (o) calcineurin inhibitors such as cyclosporine A or tacrolimus, (p) IMPDH inhibitors, such as mycophenolate mophetyl, (q) cannabinoid receptor agonists such as Sativex, (r) chemokine CCR1 antagonists such as MLN-3897 or PS-031291, (s) chemokine CCR2 antagonists such as INCB-8696, (t) interferon alpha such as Sumiferon MP, (u) NF-kappaB activation inhibitors such as FAE and MLN-0415, (v) JAK inhibitors such as CP-690550 or INCB018424, (W) Syk inhibitors, such as R-112, (x) PKC inhibitors, such as NVP-AEB071, (y) phosphosdiesterase IV inhibitors such as GRC-4039, (z) P38 Inhibitors such as ARRY-797, and (aa) MEK inhibitors, such as ARRY-142886 or ARRY-438162

The combinations of the invention may be used in the treatment of disorders which are susceptible to amelioration by sphingosine-1-phosphate receptors agonists (S1P1),. Thus, the present application encompasses methods of treatment of these disorders, as well as the use of the combinations of the invention in the manufacture of a medicament for the treatment of these disorders.

Preferred examples of such disorders are multiple sclerosis, transplant rejection, systemic lupus erythematosus, asthma, psoriasis, rheumatoid arthritis, psoriatic arthritis and Crohn's disease, more preferably multiple sclerosis, transplant rejection, asthma and rheumatoid arthritis, and most preferably multiple sclerosis.

The active compounds in the combinations of the invention may be administered by any suitable route, depending on the nature of the disorder to be treated, e.g. orally (as syrups, tablets, capsules, lozenges, controlled-release preparations, fast-dissolving preparations, etc); topically (as creams, ointments, lotions, nasal sprays or aerosols, etc); by injection (subcutaneous, intradermic, intramuscular, intravenous, etc.) or by inhalation (as a dry powder, a solution, a dispersion, etc).

The active compounds in the combination, i.e. the sphingosine-1-phosphate agonist of the invention, and the other optional active compounds may be administered together in the same pharmaceutical composition or in different compositions intended for separate, simultaneous, concomitant or sequential administration by the same or a different route.

One execution of the present invention consists of a kit of parts comprising a sphingosine-1-phosphate agonist of the invention together with instructions for simultaneous, concurrent, separate or sequential use in combination with another active compound useful in the treatment of multiple sclerosis, transplant rejection, systemic lupus erythematosus, asthma, psoriasis, rheumatoid arthritis, psoriatic arthritis and Crohn's disease,

Another execution of the present invention consists of a package comprising a sphingosine-1-phosphate agonist of formula (I) and another active compound useful in the treatment of multiple sclerosis, transplant rejection, systemic lupus erythematosus, asthma, psoriasis, rheumatoid arthritis, psoriatic arthritis and Crohn's disease,

The pharmaceutical formulations may conveniently be presented in unit dosage form and may be prepared by any of the methods well known in the art of pharmacy.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil- in-water liquid emulsion or a water-in-oil liquid emulsion. The active ingredient may also be presented as a bolus, electuary or paste.

A syrup formulation will generally consist of a suspension or solution of the compound or salt in a liquid carrier for example, ethanol, peanut oil, olive oil, glycerine or water with flavouring or colouring agent.

Where the composition is in the form of a tablet, any pharmaceutical carrier routinely used for preparing solid formulations may be used. Examples of such carriers include magnesium stearate, talc, gelatine, acacia, stearic acid, starch, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

Dry powder compositions for topical delivery to the lung by inhalation may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred. Each capsule or cartridge may generally contain between 2µg and 150 µg of each therapeutically active ingredient. Alternatively, the active ingredient (s) may be presented without excipients.

Packaging of the formulation for inhalation may be carried out by using suitable inhaler devices such as the Novolizer SD2FL which is described in the following patent applications: WO 97/000703, WO 03/000325 and WO 03/061742.

Typical compositions for nasal delivery include those mentioned above for inhalation and further include non-pressurized compositions in the form of a solution or suspension in an inert vehicle such as water optionally in combination with conventional excipients such as buffers, anti-microbials, tonicity modifying agents and viscosity modifying agents which may be administered by nasal pump.

Typical dermal and transdermal formulations comprise a conventional aqueous or non-aqueous vehicle, for example a cream, ointment, lotion or paste or are in the form of a medicated plaster, patch or membrane.

Preferably the composition is in unit dosage form, for example a tablet, capsule or metered aerosol dose, so that the patient may administer a single dose.

The amount of each active which is required to achieve a therapeutic effect will, of course, vary with the particular active, the route of administration, the subject under treatment, and the particular disorder or disease being treated.

Effective doses are normally in the range of 2-2000 mg of active ingredient per day. Daily dosage may be administered in one or more treatments, preferably from 1 to 4 treatments, per day. Preferably, the active ingredients are administered once or twice a day.

When combinations of actives are used, it is contemplated that all active agents would be administered at the same time, or very close in time. Alternatively, one or two actives could be taken in the morning and the other (s) later in the day. Or in another scenario, one or two actives could be taken twice daily and the other (s) once daily, either at the same time as one of the twice-α-day dosing occurred, or separately. Preferably at least two, and more preferably all, of the actives would be taken together at the same time. Preferably, at least two, and more preferably all actives would be administered as an admixture.

The following preparations forms are cited as formulation examples:

### COMPOSITION EXAMPLE 1

50,000 capsules, each containing 100 mg of N-butyl-N-[5-(4-methoxy-3,5-dimethylphenyl)-1,3,4-thiadiazol-2-yl]-1-naphthamide (active ingredient), were prepared according to the following formulation:

| | |
|---|---|
| Active ingredient | 5 Kg |
| Lactose monohydrate | 10 Kg |
| Colloidal silicon dioxide | 0.1 Kg |
| Corn starch | 1 Kg |
| Magnesium stearate | 0.2 Kg |

### Procedure

The above ingredients were sieved through a 60 mesh sieve, and were loaded into a suitable mixer and filled into 50,000 gelatine capsules.

### COMPOSITION EXAMPLE 2

50,000 tablets, each containing 50 mg of N-butyl-N-[5-(4-methoxy-3,5-dimethylphenyl)-1,3,4-thiadiazol-2-yl]-1-naphthamide (active ingredient), were prepared from the following formulation:

| | |
|---|---|
| Active ingredient | 2.5 Kg |
| Microcrystalline cellulose | 1.95 Kg |
| Spray dried lactose | 9.95 Kg |
| Carboxymethyl starch | 0.4 Kg |
| Sodium stearyl fumarate | 0.1 Kg |
| Colloidal silicon dioxide | 0.1 Kg |

### Procedure

All the powders were passed through a screen with an aperture of 0.6 mm, then mixed in a suitable mixer for 20 minutes and compressed into 300 mg tablets using 9 mm disc and flat bevelled punches. The disintegration time of the tablets was about 3 minutes.

## Claims

1. A compound of formula (I), or a pharmaceutically acceptable salt or N-oxide thereof 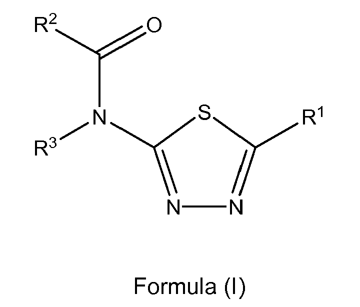 wherein
R¹ represents:
➢ an 8 to 10 membered bicyclic N-containing heteroaryl group optionally substituted by one or more substitutents selected from halogen atoms, hydroxycarbonyl groups, C₁₋₄ alkyl groups, C₁₋₄ haloaklyl groups, C₁₋₄ alkoxy groups and C₃₋₄ cycloalkyl groups;
➢ a pyridyl group substituted with one or more substituents selected from halogen atoms, hydroxy groups, hydroxycarbonyl groups, C₁₋₄ alkyl groups, C₁₋₄ haloaklyl groups, C₁₋₄ alkoxy groups, C₃₋₄ cycloalkyl groups and, -NR'R" groups, wherein R' represents a hydrogen atom or a C₁₋₄ alkyl group and R" represents a hydrogen atom or a C₁₋₄ alkyl group optionally substituted by a hydroxy group;
➢ a pyridinone group substituted with one or more substituents selected from halogen atoms, C₁₋₄ alkyl groups and C₁₋₄ haloaklyl groups; or
➢ a group of formula: 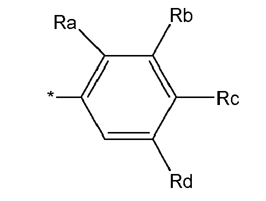
wherein:
• Ra represents a hydrogen atom or a C₁₋₄ alkyl group,
• Rb represents a hydrogen atom, halogen atom or C₁₋₄ alkyl group,
• Rd represents a hydrogen atom, a C₁₋₄ alkyl group or a C₃₋₄ cycloalkyl group,
• Rc represents a hydroxy group; a C₁₋₄ alkoxy group which is optionally substituted with one or more substituents selected from hydroxy groups, C₁₋₃ alkoxy groups, hydroxycarbonyl groups, C₁₋₄ alkoxycarbonyl groups and NHR⁴ groups, wherein R⁴ represents a hydrogen atom; or Rc is a C₂₋₄ acyl group or a C₁₋₄ alkyl group optionally substituted by a hydroxycarbonyl group, or Rc represents -(CH₂)₍₀₋₄₎-L-R⁵ wherein L represents -C(O)O-, -C(O)NH-, -S(O)₂NH-, -NH-, -CONHS(O)₂- or a group of formula: 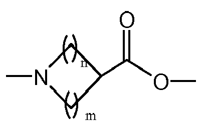
wherein n and m independently are integer from 1 to 2, and R⁵ represents a hydrogen atom or a C₁₋₄ alkyl group optionally substituted by a hydroxycarbonyl group;
R² represents:
• a monocyclic or bicyclic C₅₋₁₀ aryl group optionally substituted with one or more substituents selected from halogen atoms, C₁₋₄ alkyl groups, C₁₋₄ alkoxy groups and phenyl groups, wherein the alkyl group is optionally substituted by one or more halogen atoms,
• a monocyclic or bicyclic 5-10 membered heteroaryl group comprising one or more heteroatoms selected from N, S and O optionally substituted with one or more substituents selected from halogen atoms, C₁₋₄ alkyl and C₁₋₄ alkoxy groups, wherein the C₁₋₄ alkyl group is optionally substituted by one or more halogen atoms,
• a dihydrobenzodioxine group or a benzyl group which is optionally substituted
with one or more substituents selected from halogen atoms,
and
R³ represents:
• a linear or branched C₁₋₆ alkyl, C₃₋₆ cycloalkyl-C₁₋₄ alkyl, C₁₋₄ alkoxy-C₁₋₄ alkyl, dialkylamino-C₁₋₄alkyl group, or phenyl-C₁₋₄alkyl group;
with the proviso that when Rc represents a methoxy or ethoxy group, then one of Rb or Rd can not be a hydrogen atom;
with the additional proviso that the compound of formula (I) is not N-methyl-N-(5-(6-methylpyridin-3-yl)-1,3,4-thiadiazol-2-yl)nicotinamide, nor N-methyl-N-(5-(6-methylpyridin-3-yl)-1,3,4-thiadiazol-2-yl)isonicotinamide.

2. A compound according to claim 1, wherein R¹ represents an imidazo[1,2-a]pyridyl group; a pyridyl group which is substituted with one or more substituents selected from halogen atoms, C₁₋₄ alkyl groups and C₁₋₄ alkoxy groups; a pyridinone group substituted with a chlorine atom; or a group of formula: 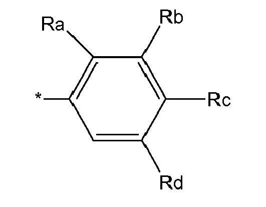 wherein:
• Ra represents a hydrogen atom or a methyl group,
• Rb represents a hydrogen atom, halogen atom or C₁₋₄ alkyl group,
• Rd represents a hydrogen atom or a C₁₋₄ alkyl group,
• Rc represents a hydroxy group, a C₁₋₄ alkoxy group which is optionally substituted with one or more substituents selected from hydroxy groups or C₁₋₃ alkoxy groups, or Rc represents -(CH₂)₍₀₋₂₎-L-R⁵, wherein L represents -C(O)NH-, -NH-, or a group of formula: 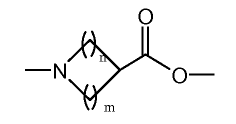
wherein n and m independently are integers from 1 to 2, and R⁵ represents a hydrogen atom or a C₁₋₄ alkyl group optionally substituted by a hydroxycarbonyl group.

3. A compound according to claim 1 or 2, wherein R¹ represents a pyridyl group substituted with one or two substituents selected from halogen atoms, C₁₋₄ alkyl groups and C₁₋₄ alkoxy groups; or a group of formula: 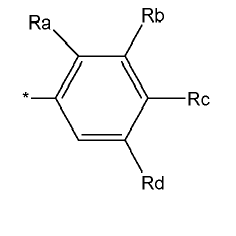 wherein:
• Ra represents a hydrogen atom,
• Rb represents a hydrogen atom or a C₁₋₄ alkyl group,
• Rd represents a hydrogen atom or C₁₋₄ alkyl group,
• Rc represents a hydroxy group, a C₁₋₄ alkoxy group which is optionally substituted by one or more substituents selected from hydroxy groups, or Rc represents -(CH₂)₍₀₋₂₎-L-R⁵, wherein L represents -C(O)NH-, -NH-, or a group of formula: 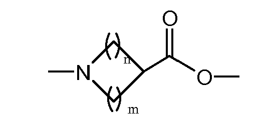
wherein n and m independently are integers from 1 to 2, and R⁵ represents a hydrogen atom or a C₁₋₄ alkyl group optionally substituted by a hydroxycarbonyl group.

4. A compound according to any one of the preceding claims, wherein R1 represents a pyridyl group substituted with one or two substituents selected from chlorine atoms, methyl and methoxy groups; or a group of formula: 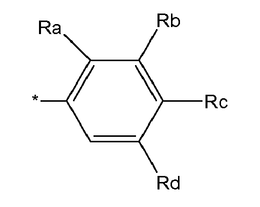 wherein:
• Ra represents a hydrogen atom,
• Rd represents a hydrogen atom or methyl group,
• Rb represents a hydrogen atom or methyl group,
• Rc represents a hydroxy group, a C₁₋₄ alkoxy group which is optionally substituted by one or more substituents selected from hydroxy groups; or Rc represents -(CH₂)₍₀₋₁₎-L-R⁵, wherein L represents -C(O)NH-, -NH-, or a group of formula: 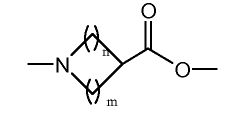
wherein both n and m have a value of 1, and R⁵ represents a hydrogen atom or a C₁₋₂ alkyl group optionally substituted by a hydroxycarbonyl group.

5. A compound according to any one of claims 1 to 4, wherein R² represents a phenyl group substituted by one substituent selected from a halogen atom, a C₁₋₄ alkyl group and a C₁₋₄ alkoxy group, wherein the alkyl group is optionally substituted by one or more halogen atoms; or R² represents a monocyclic N-containing 5-10 membered heteroaryl group which is substituted by a halogen atom.

6. A compound according to any one of the preceding claims, wherein R² represents a phenyl group which is substituted with one substituent selected from a fluorine atom, a chlorine atom, a methyl, a trifluoromethyl or a methoxy group; or R² represents a pyridyl group which is substituted with a fluorine atom.

7. A compound according to any one of the preceding claims, wherein R² represents a phenyl group substituted with one substituent selected from a fluorine atom, a chlorine atom or a methoxy group.

8. A compound according to any one of claims 1 to 7, wherein R³ represents a linear C₃₋₆ alkyl group or a C₃₋₄ cycloalkyl-C₁₋₂ alkyl group.

9. A compound according to any one of the preceding claims, wherein R³ represents a propyl, butyl or cyclopropylmethyl group.

10. A compound according to any one of the preceding claims, wherein R³ represents a butyl or a cyclopropylmethyl group.

11. A compound according to any one of claims 1 to 10, wherein R¹ represents a pyridyl group substituted with one or two substituents selected from chlorine atoms, methyl and methoxy groups; or a group of formula: 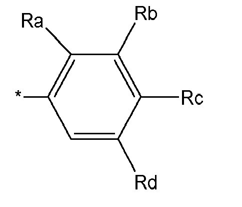 wherein:
• Ra represents a hydrogen atom,
• Rb represents a hydrogen atom or methyl group,
• Rd represents a hydrogen atom or methyl group,
• Rc represents a hydroxy group, a C₁₋₄ alkoxy group which is optionally substituted with one or more substituents selected from hydroxy groups; or Rc represents -(CH₂)₍₀₋₁₎-L-R⁵, wherein L represents -C(O)NH-, -NH-, or a group of formula: 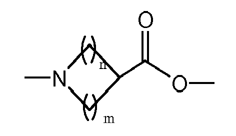
wherein both n and m have a value of 1, and R⁵ represents a hydrogen atom or a C₁₋₄ alkyl group optionally substituted by a hydroxycarbonyl group;
R² represents a phenyl group substituted with one substituent selected from a fluorine atom, a chlorine atom and a methoxy group;
and R³ represents a butyl or a cyclopropylmethyl group.

12. A compound according to claim 1, wherein R¹ represents:
- an imidazo [1, 2-a] pyridyl group;
- a pyridyl group substituted with one or two substituents selected from chlorine atoms, methyl groups and methoxy groups;
- a pyridone group substituted with a chlorine atom; or
- a group of formula: 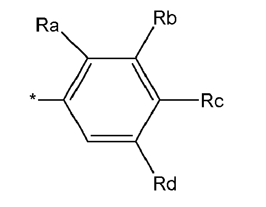
wherein
• Ra represents a hydrogen atom or a methyl group,
• Rb represents a hydrogen atom, a chlorine atom or a methyl group,
• Rd represents a hydrogen atom or a methyl group,
• Rc represents a hydroxy group; a C₁₋₄ alkoxy group which is optionally substituted with one or more substituents selected from hydroxy groups, methoxy groups, hydroxycarbonyl groups, C₁₋₄ alkoxycarbonyl groups; or Rc represents -(CH₂)₍₀₋₂₎-L-R⁵, wherein L represents -CO(O)-, -C(O)NH-, -
S(O)₂NH- or a group of formula: 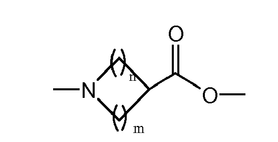
wherein n and m are both 1, and R⁵ represents a hydrogen atom or a C₁₋₂ alkyl group optionally substituted by a hydroxycarbonyl group;
R² represents:
- a phenyl or naphthyl group optionally substituted with one or two substituents selected from chlorine atoms, fluorine atoms, methyl groups, trifluoromethyl groups, C₁₋₄ alkoxy groups and phenyl groups;
- a pyridine group optionally substituted with a fluorine atom;
- a dihydrobenzodioxine group or a benzyl group; and
R³ represents a linear or branched C₁₋₅ alkyl, cyclopropylmethyl, methoxypropyl, diethylaminopropyl or phenylethyl group.

13. A compound according to claim 1 which is one of:
N-{5-[4-(aminosulfonyl)phenyl]-1,3,4-thiadiazol-2-yl}-N-butyl-3-methoxy-benzamide
N-{5-[4-(aminosulfonyl)phenyl]-1,3,4-thiadiazol-2-yl}-N-butyl-2-naphthamide
N-{5-[4-(aminosulfonyl)phenyl]-1,3,4-thiadiazol-2-yl}-N-butylbiphenyl-4-carboxamide
N-{5-[4-(aminosulfonyl)phenyl]-1,3,4-thiadiazol-2-yl}-4-butoxy-N-butylbenzamide
N-{5-[4-(aminosulfonyl)phenyl]-1,3,4-thiadiazol-2-yl}-N-butylbenzamide
3-(4-(5-(N-butyl-3-methoxybenzamido)-1,3,4-thiadiazol-2-yl)phenyl)propanoic acid
N-butyl-2-chloro-N-[5-(4-methoxy-3,5-dimethylphenyl)-1,3,4-thiadiazol-2-yl] benzamide
N-butyl-2-chloro-N-[5-(4-hydroxy-3,5-dimethylphenyl)-1,3,4-thiadiazol-2-yl]benzamide
N-butyl-2-fluoro-N-[5-(4-methoxy-3,5-dimethylphenyl)-1,3,4-thiadiazol-2-yl]benzamide
N-Butyl-2-fluoro-N-[5-(4-hydroxy-3,5-dimethylphenyl)-1,3,4-thiadiazol-2-yl]benzamide
N-butyl-N-[5-(4-methoxy-3,5-dimethylphenyl)-1,3,4-thiadiazol-2-yl]benzamide
N-butyl-3-methoxy-N-[5-(4-methoxy-3,5-dimethylphenyl)-1,3,4-thiadiazol-2-yl]benzamide
N-butyl-N-[5-(4-methoxy-3,5-dimethylphenyl)-1,3,4-thiadiazol-2-yl]-1-naphthamide
N-butyl-2,6-dichloro-N-(5-(4-methoxy-3,5-dimethylphenyl)-1,3,4-thiadiazol-2-yl)benzamide
N-butyl-N-[5-(4-methoxy-3,5-dimethylphenyl)-1,3,4-thiadiazol-2-yl]-2-(trifluoromethyl)benzamide
N-butyl-N-[5-(4-methoxy-3,5-dimethylphenyl)-1,3,4-thiadiazol-2-yl]-2-phenylacetamide
N-butyl-N-[5-(4-methoxy-3,5-dimethylphenyl)-1,3,4-thiadiazol-2-yl]-2-naphthamide
N-butyl-N-[5-(4-methoxy-3,5-dimethylphenyl)-1,3,4-thiadiazol-2-yl]-2,3-dihydro-1,4-benzodioxine-6-carboxamide
N-butyl-2-methoxy-N-[5-(4-methoxy-3,5-dimethylphenyl)-1,3,4-thiadiazol-2-yl]benzamide
N-Butyl-3-fluoro-N-[5-(4-methoxy-3,5-dimethylphenyl)-1,3,4-thiadiazol-2-yl]benzamide
N-Butyl-N-[5-(4-methoxy-3,5-dimethylphenyl)-1,3,4-thiadiazol-2-yl]nicotinamide
N-Butyl-N-[5-(4-methoxy-3,5-dimethylphenyl)-1,3,4-thiadiazol-2-yl]pyridine-2-carboxamide
N-Butyl-6-fluoro-N-[5-(4-methoxy-3,5-dimethylphenyl)-1,3,4-thiadiazol-2-yl]pyridine-2-carboxamide
N-butyl-N-[5-(4-methoxy-3,5-dimethylphenyl)-1,3,4-thiadiazol-2-yl]-2-methylbenzamide
2-chloro-N-[5-(4-methoxy-3,5-dimethylphenyl)-1,3,4-thiadiazol-2-yl]-Nmethylbenzamide
N-butyl-2-chloro-N-[5-(4-methoxy-3-methylphenyl)-1,3,4-thiadiazol-2-yl]benzamide
N-butyl-2-chloro-N-[5-(3-chloro-4-methoxyphenyl)-1,3,4-thiadiazol-2-yl]benzamide
Methyl 4-{5-[butyl(2-chlorobenzoyl)amino]-1,3,4-thiadiazol-2-yl}benzoate
4-{5-[Butyl(2-chlorobenzoyl)amino]-1,3,4-thiadiazol-2-yl}benzoic acid
N-Butyl-2-chloro-N-{5-[4-(2,3-dihydroxypropoxy)-3,5-dimethylphenyl]-1,3,4-thiadiazol-2-yl}benzamide
N-Butyl-2-chloro-N-[5-(2-chloro-6-methoxypyridin-4-yl)-1,3,4-thiadiazol-2-yl]benzamide
N-butyl-2-chloro-N-{5-[4-(2-methoxyethoxy)-3,5-dimethylphenyl]-1,3,4-thiadiazol-2-yl}benzamide
2-Chloro-N-[5-(4-methoxy-3,5-dimethylphenyl)-1,3,4-thiadiazol-2-yl]-N-(2-methoxyethyl)benzamide
2-Chloro-N-ethyl-N-[5-(4-methoxy-3,5-dimethylphenyl)-1,3,4-thiadiazol-2-yl]benzamide
*tert*-Butyl (4-{5-[butyl(2-chlorobenzoyl)amino]-1,3,4-thiadiazol-2-yl}-2,6-dimethyl-phenoxy)acetate
(4-{5-[Butyl(2-chlorobenzoyl)amino]-1,3,4-thiadiazol-2-yl}-2,6-dimethylphenoxy)acetic acid
2-Chloro-N-[5-(4-methoxy-3,5-dimethylphenyl)-1,3,4-thiadiazol-2-yl]-N-(3-methylbutyl)benzamide
2-Chloro-N-[3-(diethylamino)propyl]-N-[5-(4-methoxy-3,5-dimethylphenyl)-1,3,4-thiadiazol-2-yl]benzamide
N-Butyl-2-chloro-N-[5-(2-chloro-6-methylpyridin-4-yl)-1,3,4-thiadiazol-2-yl]benzamide
N-Butyl-2-chloro-N-[5-(6-chloro-2-oxo-1,2-dihydropyridin-4-yl)-1,3,4-thiadiazol-2-yl]benzamide
N-Butyl-N-[5-(2-chloro-6-methoxypyridin-4-yl)-1,3,4-thiadiazol-2-yl]-2-fluorobenzamide
N-Butyl-N-[5-(2-chloro-6-methylpyridin-4-yl)-1,3,4-thiadiazol-2-yl]-2-fluorobenzamide
2-fluoro-N-[5-(4-methoxy-3,5-dimethylphenyl)-1,3,4-thiadiazol-2-yl]-N-propylbenzamide
N-Butyl-2-fluoro-N-[5-(2-methylpyridin-4-yl)-1,3,4-thiadiazol-2-yl] benzamide
N-butyl-2-fluoro-N-[5-(2-methoxypyridin-4-yl)-1,3,4-thiadiazol-2-yl] benzamide
N-(cyclopropylmethyl)-2-fluoro-N-[5-(4-methoxy-3,5-dimethylphenyl)-1,3,4-thiadiazol-2-yl]benzamide
3-(4-{5-[butyl(2-fluorobenzoyl)amino]-1,3,4-thiadiazol-2-yl}-2,6-dimethylphenyl)propanoic acid
N-butyl-2-fluoro-N-(5-(6-methoxypyridin-3-yl)-1,3,4-thiadiazol-2-yl)benzamide
N-butyl-2-fluoro-N-(5-(imidazo[1,2-a]pyridin-6-yl)-1,3,4-thiadiazol-2-yl)benzamide
N-butyl-2-fluoro-N-(5-(imidazo[1,2-a]pyridin-7-yl)-1,3,4-thiadiazol-2-yl)benzamide
3-(4-(5-(N-butyl-2-chlorobenzamido)-1,3,4-thiadiazol-2-yl)benzamido) propanoic acid
Ethyl 3-(4-(5-(N-butyl-2-chlorobenzamido)-1,3,4-thiadiazol-2-yl)benzamido) propanoate
N-butyl-N-(5-(4-carbamoylphenyl)-1,3,4-thiadiazol-2-yl)-2-chlorobenzamide
1-(4-(5-(N-butyl-2-fluorobenzamido)-1,3,4-thiadiazol-2-yl)benzyl)azetidine-3-carboxylic acid
(R)-N-butyl-N-(5-(4-(2,3-dihydroxypropoxy)-3,5-dimethylphenyl)-1,3,4-thiadiazol-2-yl)-2-fluorobenzamide
2-fluoro-N-(5-(4-methoxy-3,5-dimethylphenyl)-1,3,4-thiadiazol-2-yl)-N-phenethylbenzamide
2-(4-(5-(N-butyl-2-fluorobenzamido)-1,3,4-thiadiazol-2-yl)benzamido)acetic acid
and the pharmaceutically acceptable salts and N-oxides thereof.

14. A compound according to any one of claims 1 to 13 for use in the treatment of the human or animal body.

15. A compound according to any one of claims 1 to 13 for use in the treatment of a pathological condition or disease susceptible to amelioration by sphingosine-1-phosphate receptors (S1P1) agonists.

16. A compound accroding to claim 15, wherein the pathological condition or disease is selected from autoimmune diseases, chronic immune and inflammatory diseases, transplant rejection, malignant neoplastic diseases, angiogenic-related disorders, pain, neurological diseases,viral and infectious diseases.

17. A compound according to claim 16 wherein the pathological condition or disease is selected from multiple sclerosis, transplant rejection, systemic lupus erythematosus, asthma, psoriasis, rheumatoid arthritis, psoriatic arthritis and Crohn's disease,

18. A pharmaceutical composition comprising a compound as defined in any one of claims 1 to 13 in association with a pharmaceutically acceptable diluent or carrier.

19. Use of a compound as defined in any one of claims 1 to 13 in the manufacture of a medicament for the treatment of a pathological condition or disease as defined in claims 15 to 17.

20. A method for treating a subject afflicted with a pathological condition or disease as defined in claims 15 to 17, which comprises administering to said subject an effective amount of a compound as defined in any one of claims 1 to 13.

21. A combination product comprising (i) a compound according to any one of claims 1 to 13; and (ii) another compound selected from:
a) Beta interferons such as Betaseron, Avonex or Rebif
b) Immunomodulators such as glatiramer acetate
c) Inhibitors of DNA synthesis and repair, such as Mitoxantrone
d) Anti-alpha 4 integrin antibodies, such as Natalizumab (Tysabri)
e) Alpha 4 integrin antagonists such as R-1295 , TBC-4746, CDP-323, ELND-002, Firategrast and TMC-2003
f) Dyhydrofolate reductase inhibitors, such as Methotrexate or CH-1504
g) Glucocorticoids such as prednisone or methylprednisolone
h) DHODH inhibitors such as teriflunomide
*i)* Fumaric acid esters, such as *BG-12*
*j)* Immunomodulators such as Laquinimod
k) Anti-CD20 monoclonal antibodies such as Rituximab, Ocrelizumab Ofatumumab or TRU-015
l) Anti-CD52 such as alemtuzumab
m) Anti-CD25 such as daclizumab
n) Anti-CD88, such as eculizumab or pexilizumab
o) Calcineurin inhibitors such as cyclosporine A or tacrolimus
p) IMPDH inhibitors, such as mycophenolate mophetyl
q) Cannabinoid receptor agonists such as Sativex
r) Chemokine CCR1 antagonists such as MLN-3897 or PS-031291
s) Chemokine CCR2 antagonists such as INCB-8696
t) Interferon alpha such as Sumiferon MP
u) NF-kappaB activation inhibitors such as FAE and MLN-0415
v) JAK inhibitors such as CP-690550 or INCB018424
w) Syk inhibitors, such as R-112
x) PKC inhibitors, such as NVP-AEB071
y) Phosphosdiesterase IV inhibitors such as GRC-4039
z) P38 Inhibitors such as ARRY-797
aa) MEK inhibitors, such as ARRY-142886 or ARRY-438162
